# EUROPEAN PATENT APPLICATION

(11) **EP 0 752 465 A1**
(43) Date of publication of application: **08.01.1997**
(21) Application number: 95308269.0
(22) Date of filing: 17.11.1995
(51) Int. Cl.: C11D 1/62, C11D 3/50, C11D 1/90, C11D 1/88, C07C 229/12, C07C 229/14, C07C 229/18, C07C 229/16

(54) **Betaine esters for delivery of alcohols**

(30) Priority: 01.07.1995 EP 95303762
(71) Applicant: THE PROCTER & GAMBLE COMPANY, Cincinnati, Ohio 45202 (US)
(72) Inventor: Struillou, Arnaud P., Newcastle-upon-Tyne NE12 OGD (GB); Hardy, Frederick E., Ponteland, Newcastle-upon-Tyne NE20 9ES (GB)
(74) Representative: Engisch, Gautier

(57) **Abstract**

The invention concerns compositions comprising a compound of general formula selected from: 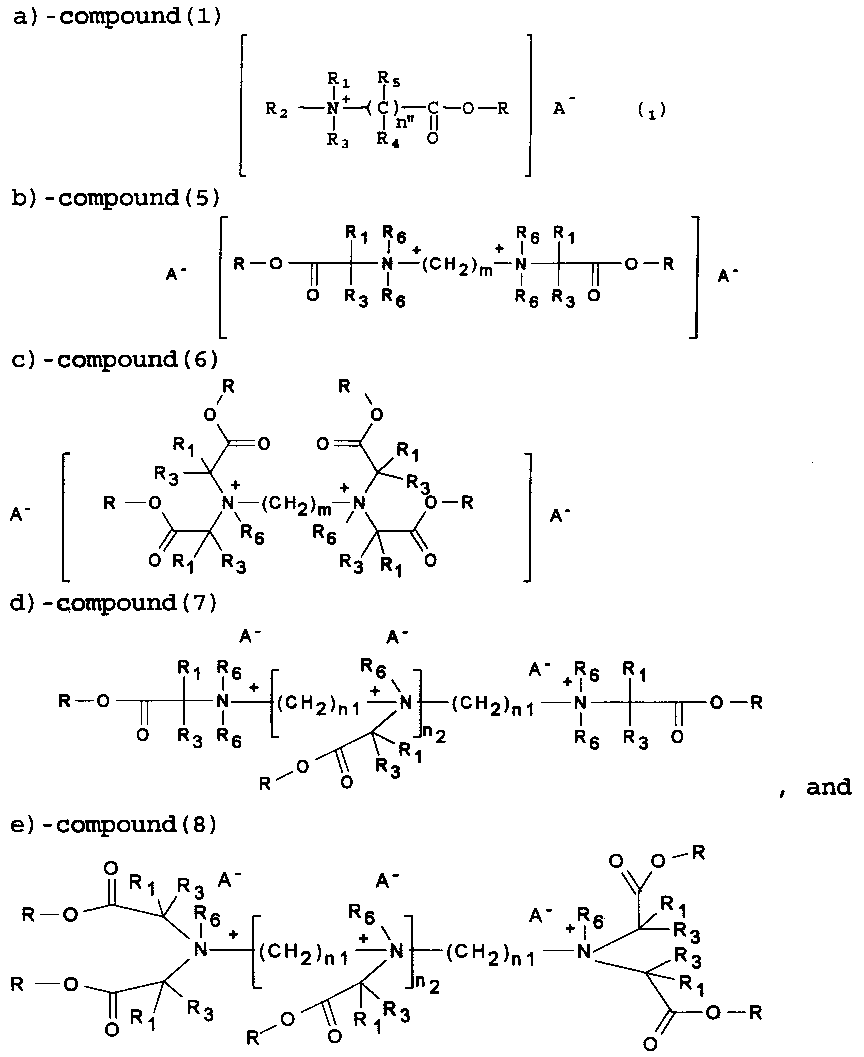 Said compositions show an excellent deposition to a surface followed by delayed release of the R-group.

More in particular, the invention relates to betaine-ester quaternary ammonium derivatives having an odoriferous alcohol as releasable R-group such as geraniol.

## Description

### FIELD OF THE INVENTION

The present invention relates to compositions comprising a betaine ester compound, said compositions showing an excellent deposition to a surface followed by delayed release of the R-group.

More in particular, the invention relates to betaine-ester quaternary ammonium derivatives having an odoriferous alcohol as releasable R-group such as geraniol.

### BACKGROUND OF THE INVENTION

Alcohols, odoriferous and biocide type alcohols in particular, which are volatile can not be delivered during a long period of time on a surface such as fabrics, floors and the like, because of quick evaporation.

It is known that consumer acceptance of cleaning and laundry products is determined not only by the performance achieved with said products, but also the aesthetics associated therewith.
An important aspect of successful formulations of these products are the perfume systems. The choice of a perfume system for a given product is a matter of careful consideration by skilled perfumers. A wide variety of chemicals and ingredients are available to perfumers. However cost and compatibility with other components in the laundry and cleaning products limit the practical options. So there is a continuing need for low-cost, compatible perfume materials useful for cleaning and laundry compositions.

In general, betaine esters and their preparation are known in the art. Examples are for instance disclosed in WO 93/25197, WO 91/17975 and EP 230,698. However, the betaine esters are known for pharmaceutical use, for disinfection purposes or as softening compound if long hydrophobic hydrocarbon groups are coupled to the nitrogen atom in the betaine ester.

In WO 91/17975 is disclosed that tertiary amines and quaternary ammonium compounds containing two long alkyl or alkenyl groups and at least one ester group can be hydrolyzed. These compounds are useful as bactericides and textile softeners. The compounds are employed in conventional manner in the given fields of utilization. As bactericides the compounds can be used for example in the food industry and in hospitals for disinfection of equipment.

It has been discovered that betaine esters of the invention are particularly well suited for laundry and cleaning compositions.
These so-called betaine esters according to the invention do have an efficient deposition to a surface followed by delayed activated release of the odoriferous alcohol.
If the betaine ester is added to a detergent matrix, the ester bond is quickly hydrolysed, subsequently releasing in the wash the water-insoluble alcohol as a fine dispersion. If said betaine ester is added to a fabric softener matrix, it deposits efficiently on the fabric during the rinse cycle and the ester bond is slowly hydrolysed by atmospheric moisture, releasing the alcohol over a long period of time/weeks to months, depending on the levels used. The remaining betaine is a harmless by-product.

If an odoriferous alcohol is used, a noticeable odour is sustained for weeks. Using this technology, if a biocide/bactericide is used, a bacteriostatic effect can be sustained for months.

In addition, slowly hydrolyzable esters of this type of odoriferous alcohols provide release of the perfume over a longer period of time than by the use of the perfume itself in the laundry/cleaning compositions. As a consequence thereof, perfumers are provided with more options for perfume ingredients and are more flexible in terms of formulating the finished cleaning products.

### SUMMARY OF THE INVENTION

The invention relates to a composition comprising a compound with the general formula selected from: 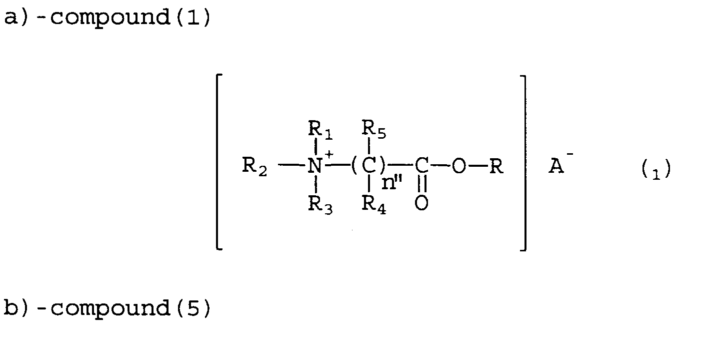 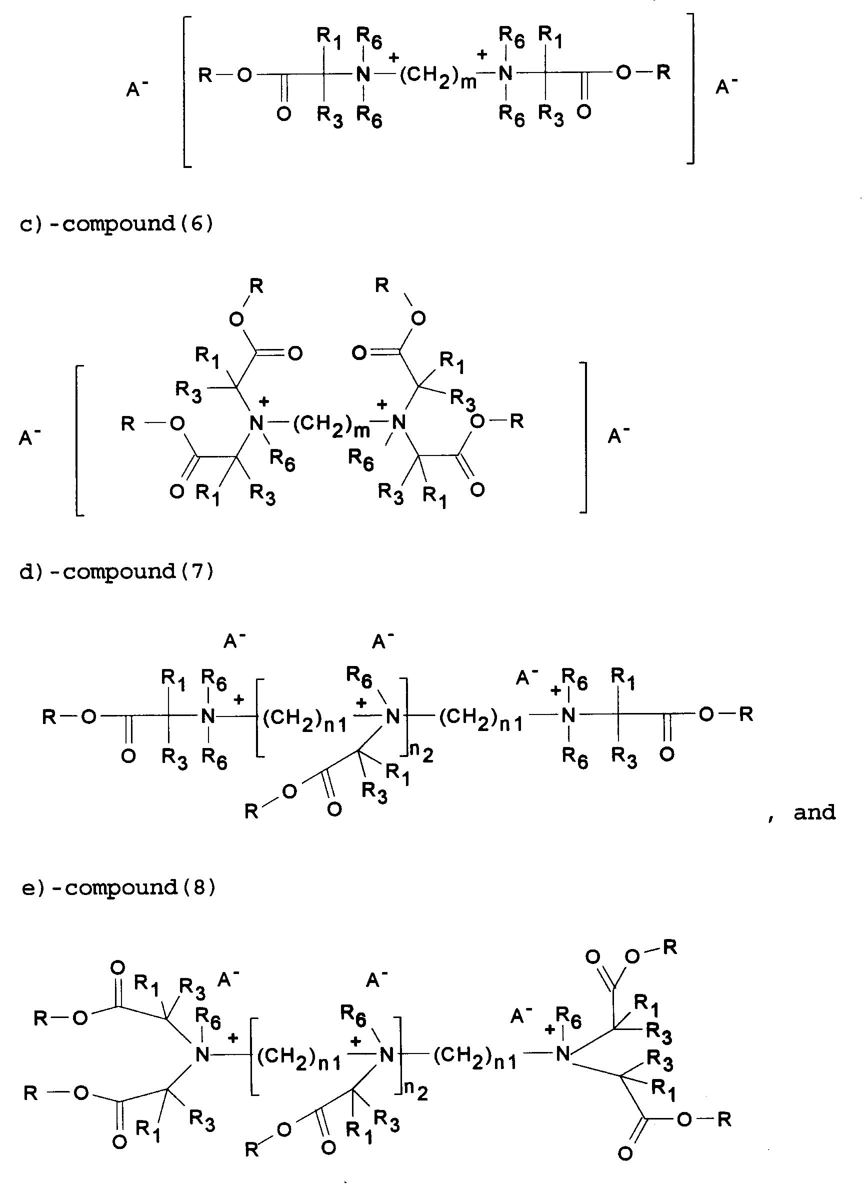 wherein
each R, independently, is derived from an alcohol of more than four (4) carbon atoms of synthetic or natural origin;
each R₁, R₂, R₃, independently is hydrogen, alkyl, hydroxyalkyl, aryl, phenyl, -(CH₂)_{n'}-O-CO-R' with n'≧1,
preferably 2 or 3, and R' is a C₁-C₂₀ (un)saturated alkyl chain, preferably C₇-C₂₀, or a -(CH₂)ₙ-CO-OR'' group wherein R'' is derived from an alcohol of synthetic or natural origin and n is 1, 2 or 3, preferably 1;
each R₄, R₅, independly, is a hydrogen, alkyl, hydroxyalkyl, aryl, phenyl or -(CH₂)ₙ-CO-OR'',
with R'' is derived from an alcohol and
n is an integer preferably 0, 1 or 2;
each A is a compatible anion and
n'' is an integer having the value of 1, 2 or 3, preferably 1, and
wherein in b), c), d) and/or e)
each R₆, independently is hydrogen, alkyl, hydroxyalkyl, aryl or phenyl,
each m is an integer of value equal or greater than 1,
each n1 is an integer lying in the range of 1 to 4,
n2 is an integer lying in the range of 1 to 6,
said compound having an efficient deposition to a surface followed by delayed activated-release of the R-group and/or R'' group.

In a preferred embodiment in the above-mentioned compound(1), R₁ and R₂ is (the same or different) hydrogen, alkyl, hydroxyalkyl, aryl, phenyl, preferably methyl,
R₃ is a -CH₂-CO-OR' group
R₄ and R₅ is hydrogen;
R and R' are derived from alcohols (the same or different) of more than four (4) carbon atoms of synthetic or natural origin and n'' has the integer of 1.

The present invention also encompasses new compounds with the general formula (2) 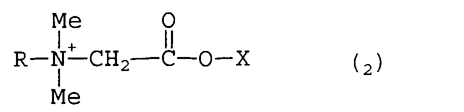
wherein R is alkyl with at least 2 C atoms, preferably butyl, octyl, dodecyl, benzyl, aryl, phenyl, pyridine derivative, -(CH₂)_{n'}-O-CO-R' with n' is preferably 2 or 3 and R' is C₁-C₂₀ atoms;
Me is a methyl group and
X is an alkyl part of an odoriferous alcohol, such as geranyl.

Also part of the invention are new compounds with the general formula (3) 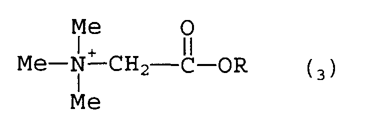
wherein Me is a methyl group and R is defined as an odoriferous alcohol selected from the group of 2-phenoxyethanol, phenylethylalcohol, geraniol, citronellol, 3-methyl-5-phenyl-1-pentanol, 2,4-dimethyl-3-cyclohexene-1-methanol, linalool, tetrahydrolinalool, 1,2-dihydromyrcenol, hydroxycitronellal, farnesol, menthol, eugenol, vanilin, cis-3-hexenol or mixtures thereof.

Furthermore, the invention concerns a new method for preparation of the inventive betaine esters and the preparation of alkaline stable compositions having these betaine esters incorporated.

### DETAILED DESCRIPTION OF THE INVENTION

The present invention compositions comprise a compound with the general formula selected from: 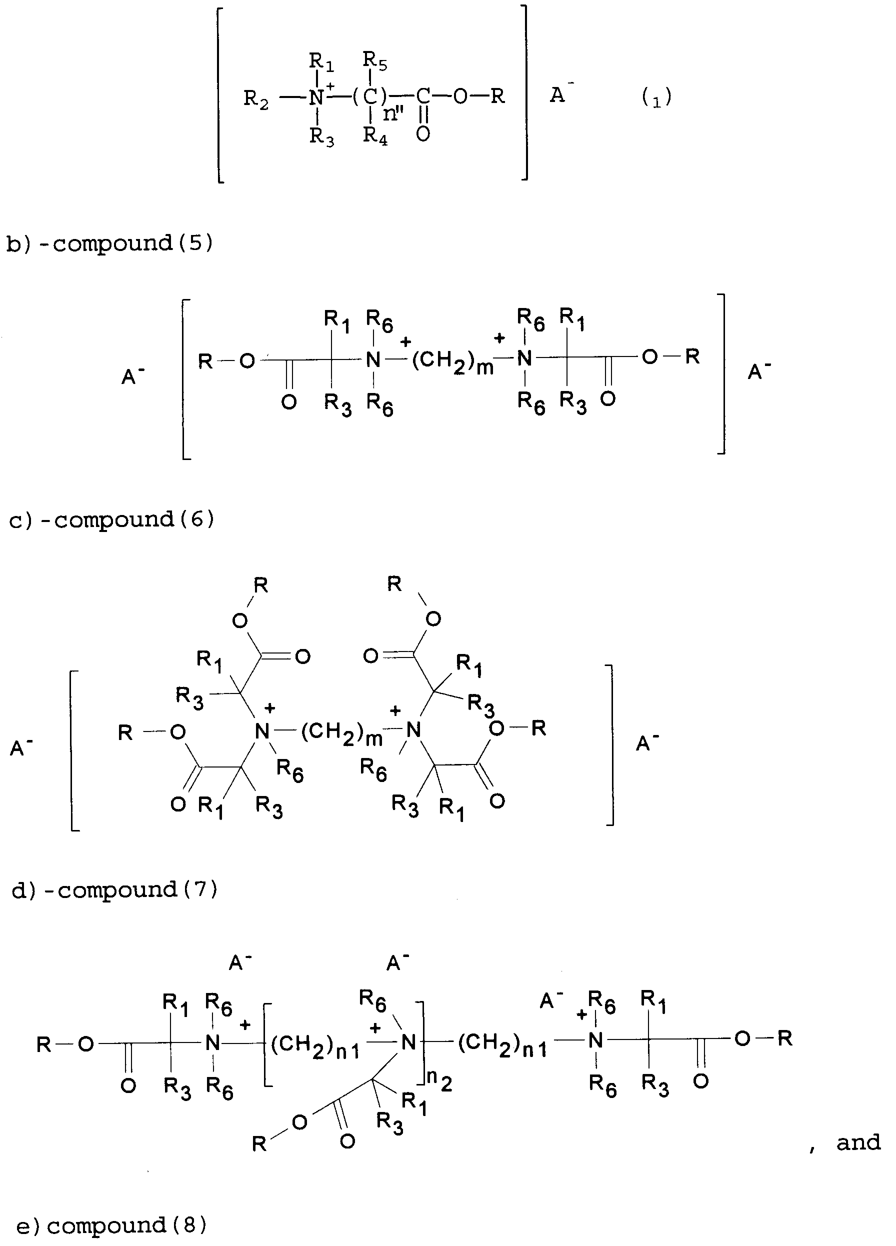 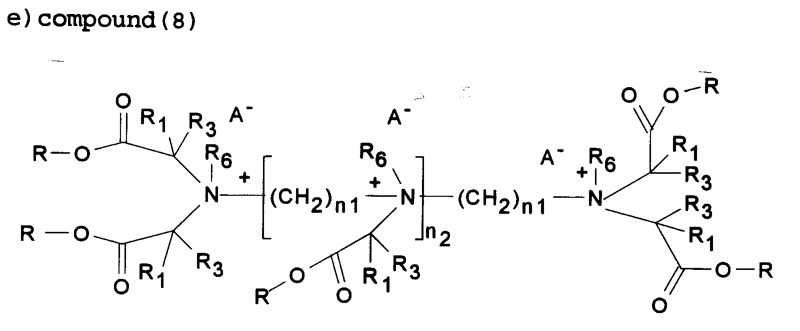 wherein
each R, independently, is derived from an alcohol of more than four (4) carbon atoms of synthetic or natural origin;
each R₁, R₂, R₃, independently is hydrogen, alkyl, hydroxyalkyl, aryl, phenyl, -(CH₂)_{n'}-O-CO-R' with n'≧1,
preferably 2 or 3, and R' is a C₁-C₂₀ (un)saturated alkyl chain, preferably C₇-C₂₀, or a -(CH₂)ₙ-CO-OR'' group wherein R'' is derived from an alcohol of synthetic or natural origin and n is 1, 2 or 3, preferably 1;
each R₄, R₅, independly, is a hydrogen, alkyl, hydroxyalkyl, aryl, phenyl or -(CH₂)ₙ-CO-OR'', with R'' is derived from an alcohol and
n is an integer preferably 0, 1 or 2;
each A is a compatible anion and
n'' is an integer having the value of 1, 2 or 3, preferably 1, and
wherein in b), c), d) and/or e)
each R₆, independently is hydrogen, alkyl, hydroxyalkyl, aryl or phenyl,
each m is an integer of value equal or greater than 1,
each n1 is an integer lying in the range of 1 to 4,
n2 is an integer lying in the range of 1 to 6,
said compound having an efficient deposition to a surface followed by delayed activated-release of the R-group and/or R'' group.

Preferred compounds of formula (1) useful herein are those wherein
R₁, R₂ and R₃ is methyl;
R₄ and R₅ is hydrogen,
R is an odoriferous alcohol of more than four (4) carbon atoms of synthetic or natural origin and
n'' has the integer of 1.

Other preferred compounds of formula (1) are those wherein R₁ and R₂ are methyl, R₃ is an alkyl/alkenyl chain preferably butyl, octyl, dodecyl or benzyl, or an aryl/phenyl chain, or a pyridine derivative, R₄ and R₅ is hydrogen, R is derived from an alcohol of more than four (4) carbon atoms of synthetic or natural origin and n'' has the integer of 1.

Preferred useful compound of formula (1) are also those wherein R₁, R₂, R₃ is ethyl, R₄ and R₅ is hydrogen, R is derived from an alcohol of more than four (4) carbon atoms of synthetic or natural origin and n'' has the integer of 1.

By "odoriferous alcohol" is understood any alcohol commonly used in perfumery, which is capable of assigning, during the washing process and/or during the treatment with a fabric softener, a scent to fabrics.

In addition, the compounds can also be those wherein
R₁ and R₂ is (the same or different) hydrogen, alkyl, hydroxyalkyl, aryl, phenyl, preferably methyl,
R₃ is a -CH₂-CO-OR group
R₄ and R₅ is hydrogen;
R is derived from an alcohol of more than four (4) carbon atoms of synthetic or natural origin and n'' has the integer of 1.

Other options of useful preferred compounds are those wherein
R₁ and R₂ is -(CH₂)n'-O-CO-R' with n'≧1, preferably 2 or 3 and R' is a C₁-C₂₀ (un)saturated alkyl chain, preferably C₇-C₂₀;
R₃ is hydrogen, alkyl, hydroxy alkyl, aryl, phenyl, preferably methyl,
R₄ and R₅ is a hydrogen;
R is derived from an alcohol of more than four (4) carbon atoms of synthetic or natural origin and n'' has the integer of 1.

Last mentioned preferred embodiment is the compound wherein R₁ is hydrogen, alkyl, hydroxyalkyl, aryl, phenyl or -(CH₂)_{n'}-O-CO-R' with
n'≧ 1, preferably 2 or 3 and R' is a C₁-C₂₀ (un)saturated alkyl chain, preferably C₇-C₂₀; and R₂ and R₃ are hydrogen, alkyl, hydroxyalkyl, aryl, phenyl, preferably methyl.

Furthermore, new compounds according to the present invention are those with the general formula (2) and (3) viz. 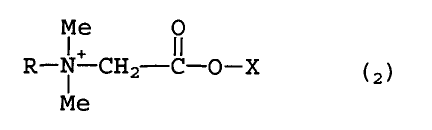
wherein R is alkyl with at least 2 C atoms, preferably butyl, octyl, dodecyl, benzyl, aryl, phenyl, pyridine derivative,
-(CH₂)_{n'}-O-CO-R' with n' is preferably 2 or 3 and R' is C₁-C₂₀ atoms;
Me is a methyl group and
X is an alkyl part of an odoriferous alcohol, such as geranyl; and the compound 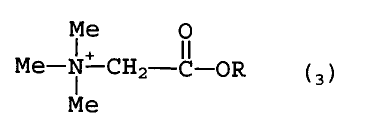
wherein Me is a methyl group and R is defined as an odoriferous alcohol selected from the group of 2-phenoxyethanol, phenylethylalcohol, geraniol, citronellol, 3-methyl-5-phenyl-1-pentanol, 2,4-dimethyl-3-cyclohexene-1-methanol, linalool, tetrahydrolinalool, 1,2-dihydromyrcenol, hydroxycitronellal, farnesol, menthol, eugenol, vanilin, cis-3-hexenol or mixtures thereof.

Still other preferred compounds for the purpose of the invention are compounds of formula selected from: 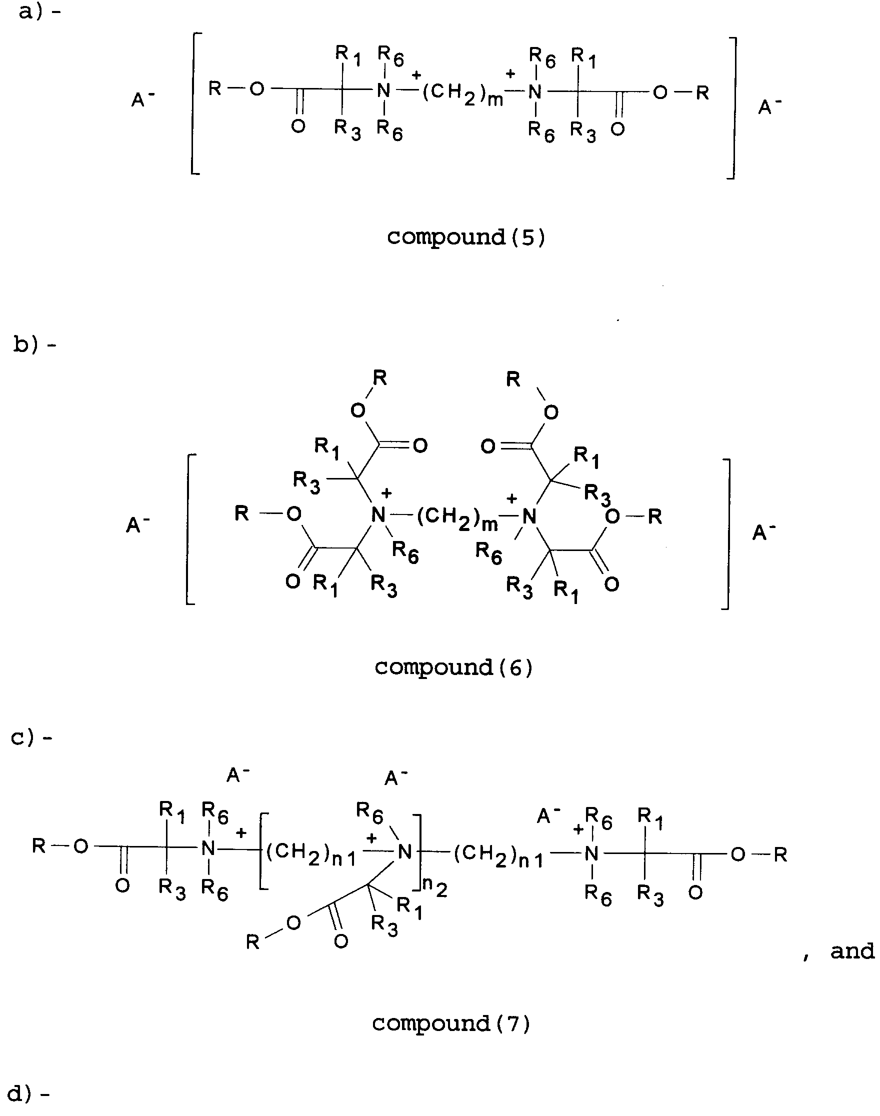 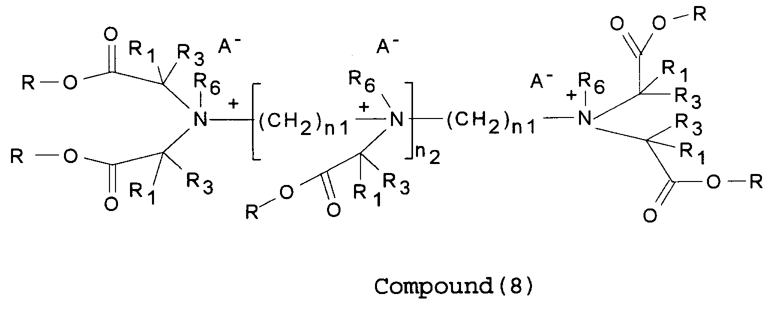 wherein
each R, independently, is derived from an alcohol of more than four (4) carbon atoms of synthetic or natural origin;
each R₁, R₃, independently is hydrogen, alkyl, hydroxyalkyl, aryl, phenyl, -(CH₂)_{n'}-O-CO-R' with n'≧1,
preferably 2 or 3, and R' is a C₁-C₂₀ (un)saturated alkyl chain, preferably C₇-C₂₀, or a -(CH₂)ₙ-CO-OR'' group wherein R'' is derived from an alcohol of synthetic or natural origin and n is 1, 2 or 3, preferably 1;
each R₆, independently is hydrogen, alkyl, hydroxyalkyl, aryl or phenyl,
each A is a compatible anion and
each n, independently, is an integer lying in the range of 0 to 2;
each n'', independently, is an integer lying in the range of 1 to 3, preferably 1, and
each m is an integer of value equal or greater than 1, each n1 is an integer lying in the range of 1 to 4, and
n2 is an integer lying in the range of 1 to 6.

A preferred compound (5) of formula as defined above is one wherein
each R is derived from an alcohol of more than four (4) carbon atoms of synthetic or natural origin;
each R₁, R₃ is hydrogen;
each R6 is methyl,
m is an integer of value 1,2,3,4 or 6.

Another preferred compound (5) of formula as defined above is one wherein each R is derived from an alcohol of more than four (4) carbon atoms of synthetic or natural origin;
each R₁, R₃ is hydrogen;
each R6 is hydrogen, and
m is an integer lying in the range of 2 to 12.

Another preferred compound (5) of formula as defined above is one wherein each R is derived from an alcohol of more than four (4) carbon atoms of synthetic or natural origin;
each R₁ is methyl and each R₃ is hydrogen
each R6 is methyl,
m is an integer of value 1,2,3,4 or 6.

Still another preferred compound (5) of formula as defined above is one wherein each R is derived from an alcohol of more than four (4) carbon atoms of synthetic or natural origin;
each R₁ is methyl and each R₃ is hydrogen
each R6 is hydrogen,
m is an integer lying in the range of 2 to 12.

A preferred compound (6) of formula as defined above is one wherein each R is derived from an alcohol of more than four (4) carbon atoms of synthetic or natural origin;
each R₁, R₃ is hydrogen;
each R6 is hydrogen, and
m is an integer lying in the range of 2 to 12.

Still another preferred compound (6) of formula as defined above is one wherein each R is derived from an alcohol of more than four (4) carbon atoms of synthetic or natural origin;
each R₁ is methyl and each R₃ is hydrogen
each R6 is hydrogen,
m is an integer lying in the range of 2 to 12.

A preferred compound (7) or compound (8) of formula as defined above is one wherein each R is derived from an alcohol of more than four (4) carbon atoms of synthetic or natural origin;
each R₁, R₃ is hydrogen;
each R6 is methyl,
each n1 is an integer of value 2 or 3, and
n2 is an integer lying in the range of 1 to 4.

Another preferred compound (7) or compound (8) of formula as defined above is one wherein each R is derived from an alcohol of more than four (4) carbon atoms of synthetic or natural origin;
each R₁, R₃ is hydrogen;
each R6 is hydrogen,
each n1 is an integer of value 2 or 3, and
n2 is an integer lying in the range of 1 to 4.

Another preferred compound (7) or compound (8) of formula as defined above is one wherein each R is derived from an alcohol of more than four (4) carbon atoms of synthetic or natural origin;
each R₁ is methyl and each R₃ is hydrogen
each R6 is hydrogen,
each n1 is an integer of value 2 or 3, and
n2 is an integer lying in the range of 1 to 4.

Still another preferred compound (7) or compound (8) of formula as defined above is one wherein each R is derived from an alcohol of more than four (4) carbon atoms of synthetic or natural origin;
each R₁ is methyl and each R₃ is hydrogen
each R6 is methyl,
each n1 is an integer of value 2 or 3, and
n2 is an integer lying in the range of 1 to 4.

The present invention compositions include both laundry and cleaning products which are typically used for laundering fabrics and cleaning hard surfaces such as dishware, floors and other surfaces in need of cleaning and/or disinfecting.

Preferred are those laundry compositions which result in contacting the betaine ester of an alcohol perfume such as geraniol as described hereafter with fabric. These are to be understood to include not only detergent compositions which provide fabric cleaning benefits, but also compositions such as rinse added fabric softener compositions and dryer added compositions (e.g. sheets) which provide softening and/or antistatic benefits.

Preferred alcohol perfumes mentioned above are those selected from the group of 2-phenoxyethanol, phenylethylalcohol, geraniol, citronellol, 3-methyl-5-phenyl-1-pentanol, 2,4-dimethyl-3-cyclohexene-1-methanol, linalool, tetrahydrolinalool, 1,2-dihydromyrcenol, hydroxycitronellal, farnesol, menthol, eugenol, vanilin, cis-3-hexenol or mixtures thereof.

The releasable R-group of the compounds according to the invention can also be defined as a bactericide/biocide alcohol such as m-chloroxylenol, 2,4-dichlorophenol, triclosan or 2,4-dichlorobenzylalcohol.

In addition, the inventive technology is applicable in wide applications anywhere a sustained release of active perfume is useful, such as in hard-surface cleaners (long lasting disinfection of kitchen board, toilet bowls), carpet-care (sustained acaracidal effect), health care (deodorant, toothpaste).

In the examples hereinbelow are disclosed new processes for preparing betaine esters according to the invention and the preparation of alkaline stable compositions comprising said betaine esters as well.

Optional ingredients useful for formulating laundry and cleaning compositions according to the present invention may include one or more of the following.

### Cationic or Nonionic Fabric Softening Agents:

The preferred fabric softening agents to be used in the present invention compositions are quaternary ammonium compounds or amine precursors herein having the formula (I) or (II), below : 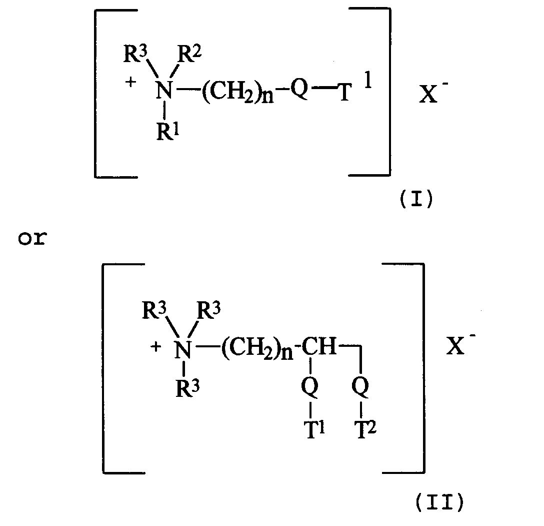
Q is -O-C(O)- or -C(O)-O- or -O-C(O)-O- or -NR⁴-C(O)-or -C(O)-NR⁴-;
R¹ is (CH₂)ₙ-Q-T² or T³;
R² is (CH₂)ₘ-Q-T⁴ or T⁵ or R³;
R³ is C₁-C₄ alkyl or C₁-C₄ hydroxyalkyl or H;
R⁴ is H or C₁-C₄ alkyl or C₁-C₄ hydroxyalkyl;
T¹, T², T³, T⁴, T⁵ are (the same or different) C₁₁-C₂₂ alkyl or alkenyl;
n and m are integers from 1 to 4; and
X⁻ is a softener-compatible anion, such as chloride, methyl sulfate, etc.

The alkyl, or alkenyl, chain T¹, T², T³, T⁴, T⁵ must contain at least 11 carbon atoms, preferably at least 16 carbon atoms. The chain may be straight or branched.

Tallow is a convenient and inexpensive source of long chain alkyl and alkenyl material. The compounds wherein T¹, T², T³, T⁴, T⁵ represents the mixture of long chain materials typical for tallow are particularly preferred. Specific examples of quaternary ammonium compounds suitable for use in the aqueous fabric softening compositions herein include :
1) N,N-di(tallowyl-oxy-ethyl)-N,N-dimethyl ammonium chloride;
2) N,N-di(tallowyl-oxy-ethyl)-N-methyl, N-(2-hydroxyethyl) ammonium chloride;
3) N,N-di(2-tallowyl-oxy-2-oxo-ethyl)-N,N-dimethyl ammonium chloride;
4) N,N-di(2-tallowyl-oxy-ethylcarbonyloxyethyl)-N,N-dimethyl ammonium chloride;
5) N-(2-tallowyl-oxy-2-ethyl)-N-(2-tallowyloxy-2-oxo-ethyl)-N,N-dimethyl ammonium chloride;
6) N,N,N-tri(tallowyl-oxy-ethyl)-N-methyl ammonium chloride;
7) N-(2-tallowyl-oxy-2-oxoethyl)-N-(tallowyl)-N,N-dimethyl ammonium chloride; and
8) 1,2-ditallowyl-oxy-3-trimethylammoniopropane chloride.; and mixtures of any of the above materials.
Of these, compounds 1-7 are examples of compounds of Formula (I); compound 8 is a compound of Formula (II).

Particularly preferred is N,N-di(tallowoyl-oxy-ethyl)-N,N-dimethyl ammonium chloride, where the tallow chains are at least partially unsaturated.

The level of unsaturation of the tallow chain can be measured by the Iodine Value (IV) of the corresponding fatty acid, which in the present case should preferably be in the range of from 5 to 100 with two categories of compounds being distinguished, having a IV below or above 25.

Indeed, for compounds of Formula (I) made from tallow fatty acids having a IV of from 5 to 25, preferably 15 to 20, it has been found that a cis/trans isomer weight ratio greater than about 30/70, preferably greater than about 50/50 and more preferably greater than about 70/30 provides optimal concentrability.

For compounds of Formula (I) made from tallow fatty acids having a IV of above 25, the ratio of cis to trans isomers has been found to be less critical unless very high concentrations are needed.

Other examples of suitable quaternary ammoniums of Formula (I) and (II) are obtained by, e.g.,
- replacing "tallow" in the above compounds with, for example, coco, palm, lauryl, oleyl, ricinoleyl, stearyl, palmityl, or the like, said fatty acyl chains being either fully saturated, or preferably at least partly unsaturated;
- replacing "methyl" in the above compounds with ethyl, ethoxy, propyl, propoxy, isopropyl, butyl, isobutyl or t-butyl;
- replacing "chloride" in the above compounds with bromide, methylsulfate, formate, sulfate, nitrate, and the like.

In fact, the anion is merely present as a counterion of the positively charged quaternary ammonium compounds. The nature of the counterion is not critical at all to the practice of the present invention. The scope of this invention is not considered limited to any particular anion.

By "amine precursors thereof" is meant the secondary or tertiary amines corresponding to the above quaternary ammonium compounds, said amines being substantially protonated in the present compositions due to the claimed pH values.

The quaternary ammonium or amine precursors compounds herein are present at levels of from about 1% to about 80% of compositions herein, depending on the composition execution which can be dilute with a preferred level of active from about 5% to about 15%, or concentrated, with a preferred level of active from about 15% to about 50%, most preferably about 15% to about 35%.

For the preceeding fabric softening agents, the pH of the compositions herein is an essential parameter of the present invention. Indeed, it influences the stability of the quaternary ammonium or amine precursors compounds, especially in prolonged storage conditions.

The pH, as defined in the present context, is measured in the neat compositions at 20°C. For optimum hydrolytic stability of these compositions, the neat pH, measured in the above-mentioned conditions, must be in the range of from about 2.0 to about 4.5, preferably about 2.0 to about 3.5. The pH of these compositions herein can be regulated by the addition of a Bronsted acid.

Examples of suitable acids include the inorganic mineral acids, carboxylic acids, in particular the low molecular weight (C₁-C₅) carboxylic acids, and alkylsulfonic acids. Suitable inorganic acids include HCl, H₂SO₄, HNO₃ and H₃PO₄. Suitable organic acids include formic, acetic, citric, methylsulfonic and ethylsulfonic acid. Preferred acids are citric, hydrochloric, phosphoric, formic, methylsulfonic acid, and benzoic acids.

Softening agents also useful in the present invention compositions are nonionic fabric softener materials, preferably in combination with cationic softening agents. Typically, such nonionic fabric softener materials have a HLB of from about 2 to about 9, more typically from about 3 to about 7. Such nonionic fabric softener materials tend to be readily dispersed either by themselves, or when combined with other materials such as single-long-chain alkyl cationic surfactant described in detail hereinafter. Dispersibility can be improved by using more single-long-chain alkyl cationic surfactant, mixture with other materials as set forth hereinafter, use of hotter water, and/or more agitation. In general, the materials selected should be relatively crystalline, higher melting, (e.g. >40°C) and relatively water-insoluble.

The level of optional nonionic softener in the compositions herein is typically from about 0.1% to about 10%, preferably from about 1% to about 5%.

Preferred nonionic softeners are fatty acid partial esters of polyhydric alcohols, or anhydrides thereof, wherein the alcohol, or anhydride, contains from 2 to 18, preferably from 2 to 8, carbon atoms, and each fatty acid moiety contains from 12 to 30, preferably from 16 to 20, carbon atoms. Typically, such softeners contain from one to 3, preferably 2 fatty acid groups per molecule.

The polyhydric alcohol portion of the ester can be ethylene glycol, glycerol, poly (e.g., di-, tri-, tetra, penta-, and/or hexa-) glycerol, xylitol, sucrose, erythritol, pentaerythritol, sorbitol or sorbitan. Sorbitan esters and polyglycerol monostearate are particularly preferred.

The fatty acid portion of the ester is normally derived from fatty acids having from 12 to 30, preferably from 16 to 20, carbon atoms, typical examples of said fatty acids being lauric acid, myristic acid, palmitic acid, stearic acid, oleic and behenic acid.

Highly preferred optional nonionic softening agents for use in the present invention are the sorbitan esters, which are esterified dehydration products of sorbitol, and the glycerol esters.

Commercial sorbitan monostearate is a suitable material. Mixtures of sorbitan stearate and sorbitan palmitate having stearate/palmitate weight ratios varying between about 10:1 and about 1:10, and 1,5-sorbitan esters are also useful.

Glycerol and polyglycerol esters, especially glycerol, diglycerol, triglycerol, and polyglycerol mono- and/or diesters, preferably mono-, are preferred herein (e.g. polyglycerol monostearate with a trade name of Radiasurf 7248).

Useful glycerol and polyglycerol esters include monoesters with stearic, oleic, palmitic, lauric, isostearic, myristic, and/or behenic acids and the diesters of stearic, oleic, palmitic, lauric, isostearic, behenic, and/or myristic acids. It is understood that the typical monoester contains some di- and tri-ester, etc.

The "glycerol esters" also include the polyglycerol, e.g., diglycerol through octaglycerol esters. The polyglycerol polyols are formed by condensing glycerin or epichlorohydrin together to link the glycerol moieties via ether linkages. The mono- and/or diesters of the polyglycerol polyols are preferred, the fatty acyl groups typically being those described hereinbefore for the sorbitan and glycerol esters.

Additional fabric softening agents useful herein are described in U.S. Pat. No. 4,661,269, issued April 28, 1987, in the names of Toan Trinh, Errol H. Wahl, Donald M. Swartley, and Ronald L. Hemingway; U.S. Pat. No. 4,439,335, Burns, issued March 27, 1984; and in U.S. Pat. NOS.: 3,861,870, Edwards and Diehl; 4,308,151, Cambre; 3,886,075, Bernardino; 4,233,164, Davis; 4,401,578, Verbruggen; 3,974,076, Wiersema and Rieke; 4,237,016, Rudkin, Clint, and Young; and European Patent Application publication No. 472,178, by Yamamura et al.

For example, suitable fabric softener agents useful herein may comprise one, two, or all three of the following fabric softening agents:
(a)the reaction product of higher fatty acids with a polyamine selected from the group consisting of hydroxyalkylalkylenediamines and dialkylenetriamines and mixtures thereof (preferably from about 10% to about 80%); and/or
(b)cationic nitrogenous salts containing only one long chain acyclic aliphatic C₁₅-C₂₂ hydrocarbon group (preferably from about 3% to about 40%); and/or
(c)cationic nitrogenous salts having two or more long chain acyclic aliphatic C₁₅-C₂₂ hydrocarbon groups or one said group and an arylalkyl group (preferably from about 10% to about 80%);
with said (a), (b) and (c) preferred percentages being by weight of the fabric softening agent component of the present invention compositions.

Following are the general descriptions of the preceeding (a), (b), and (c) softener ingredients (including certain specific examples which illustrate, but do not limit the present invention).
Component (a): Softening agents (actives) of the present invention may be the reaction products of higher fatty acids with a polyamine selected from the group consisting of hydroxyalkylalkylenediamines and dialkylenetriamines and mixtures thereof. These reaction products are mixtures of several compounds in view of the multi-functional structure of the polyamines.

The preferred Component (a) is a nitrogenous compound selected from the group consisting of the reaction product mixtures or some selected components of the mixtures. More specifically, the preferred Component (a) is compounds selected from the group consisting of substituted imidazoline compounds having the formula: 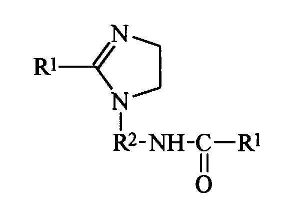
wherein R¹ is an acyclic aliphatic C₁₅-C₂₁ hydrocarbon group and R² is a divalent C₁-C₃ alkylene group.

Component (a) materials are commercially available as: Mazamide® 6, sold by Mazer Chemicals, or Ceranine® HC, sold by Sandoz Colors & Chemicals; stearic hydroxyethyl imidazoline sold under the trade names of Alkazine® ST by Alkaril Chemicals, Inc., or Schercozoline® S by Scher Chemicals, Inc.; N,N''-ditallowalkoyldiethylenetriamine; 1-tallowamidoethyl-2-tallowimidazoline (wherein in the preceeding structure R¹ is an aliphatic C₁₅-C₁₇ hydrocarbon group and R² is a divalent ethylene group).

Certain of the Components (a) can also be first dispersed in a Bronsted acid dispersing aid having a pKa value of not greater than about 4; provided that the pH of the final composition is not greater than about 5. Some preferred dispersing aids are hydrochloric acid, phosphoric acid, or methylsulfonic acid.

Both N,N''-ditallowalkoyldiethylenetriamine and 1-tallow(amidoethyl)-2-tallowimidazoline are reaction products of tallow fatty acids and diethylenetriamine, and are precursors of the cationic fabric softening agent methyl-1-tallowamidoethyl-2-tallowimidazolinium methylsulfate (see "Cationic Surface Active Agents as Fabric Softeners," R. R. Egan, Journal of the American Oil Chemicals' Society, January 1978, pages 118-121). N,N''-ditallow alkoyldiethylenetriamine and 1-tallowamidoethyl-2-tallowimidazoline can be obtained from Witco Chemical Company as experimental chemicals. Methyl-1-tallowamidoethyl-2-tallowimidazolinium methylsulfate is sold by Witco Chemical Company under the tradename Varisoft® 475.

Component (b): The preferred Component (b) is a cationic nitrogenous salt containing one long chain acyclic aliphatic C₁₅-C₂₂ hydrocarbon group, preferrably selected from acyclic quaternary ammonium salts having the formula: 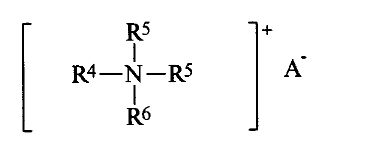
wherein R⁴ is an acyclic aliphatic C₁₅-C₂₂ hydrocarbon group, R⁵ and R⁶ are C₁-C₄ saturated alkyl or hydroxy alkyl groups, and A- is an anion.

Examples of Component (b) are the monoalkyltrimethylammonium salts such as monotallowtrimethylammonium chloride, mono(hydrogenated tallow)trimethylammonium chloride, palmityltrimethyl ammonium chloride and soyatrimethylammonium chloride, sold by Sherex Chemical Company under the trade name Adogen® 471, Adogen® 441, Adogen® 444, and Adogen® 415, respectively. In these salts, R⁴ is an acyclic aliphatic C₁₆-C₁₈ hydrocarbon group, and R⁵ and R⁶ are methyl groups. Mono(hydrogenated tallow)trimethylammonium chloride and monotallowtrimethylammonium chloride are preferred.

Other examples of Component (b) are behenyltrimethylammonium chloride wherein R⁴ is a C₂₂ hydrocarbon group and sold under the trade name Kemamine® Q2803-C by Humko Chemical Division of Witco Chemical Corporation; soyadimethylethylammonium ethylsulfate wherein R⁴ is a C₁₆-C₁₈ hydrocarbon group, R⁵ is a methyl group, R⁶ is an ethyl group, and A- is an ethylsulfate anion, sold under the trade name Jordaquat® 1033 by Jordan Chemical Company; and methyl-bis(2-hydroxyethyl)-octadecylammonium chloride wherein R⁴ is a C₁₈ hydrocarbon group, R⁵ is a 2-hydroxyethyl group and R⁶ is a methyl group and available under the trade name Ethoquad® 18/12 from Armak Company.

Other examples of Component (b) are 1-ethyl-1-(2-hydroxy ethyl)-2-isoheptadecylimidazolinium ethylsulfate, available from Mona Industries, Inc. under the trade name Monaquat® ISIES; mono(tallowoyloxyethyl) hydroxyethyldimethylammonium chloride, i.e., monoester of tallow fatty acid with di(hydroxyethyl)dimethylammonium chloride, a by-product in the process of making diester of tallow fatty acid with di(hydroxyethyl)dimethylammonium chloride, i.e., di(tallowoyloxyethyl)dimethylammonium chloride.

Component (c): Preferred cationic nitrogenous salts having two or more long chain acyclic aliphatic C₁₅-C₂₂ hydrocarbon groups or one said group and an arylalkyl group which can be used either alone or as part of a mixture are selected from the group consisting of:
(i) acyclic quaternary ammonium salts having the formula: 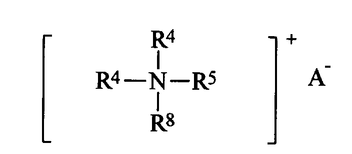
   wherein R⁴ is an acyclic aliphatic C₁₅-C₂₂ hydrocarbon group, R⁵ is a C₁-C₄ saturated alkyl or hydroxyalkyl group, R⁸ is selected from the group consisting of R⁴ and R⁵ groups, and A- is an anion defined as above;
(ii) diamido quaternary ammonium salts having the formula: 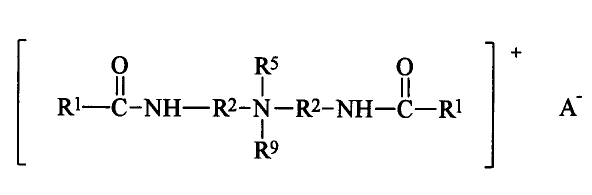
   wherein R¹ is an acyclic aliphatic C₁₅-C₂₁ hydrocarbon group, each R² is the same or different divalent alkylene group having 1 to 3 carbon atoms, R⁵ and R⁹ are C₁-C₄ saturated alkyl or hydroxyalkyl groups, and A- is an anion;
(iii) diamino alkoxylated quaternary ammonium salts having the formula: 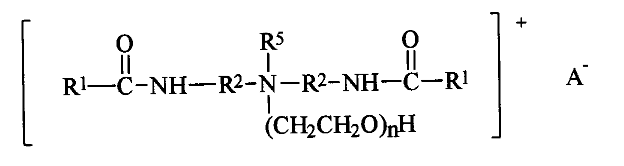
   wherein n is equal to 1 to about 5, and R¹, R², R⁵ and A- are as defined above;
(iv) diester quaternary ammonium (DEQA) compounds having the formula:

   (R)₄₋ₘ - N⁺ - [(CH₂)ₙ - Y - R²]ₘ A⁻

   wherein
   each Y = -O-(O)C-, or -C(O)-O-;
   m = 2 or 3;
   each n = 1 to 4;

   each R substituent is a short chain C₁-C₆, preferably C₁-C₃ alkyl or hydroxyalkyl group, e.g., methyl (most preferred), ethyl, propyl, hydroxyethyl, and the like, benzyl, or mixtures thereof;
   each R² is a long chain C₁₀-C₂₂ hydrocarbyl, or substituted hydrocarbyl substituent, preferably C₁₅-C₁₉ alkyl and/or alkenyl, most preferably C₁₅-C₁₈ straight chain alkyl and/or alkenyl; and
   the counterion, A-, can be any softener-compatible anion, for example, chloride, bromide, methylsulfate, formate, sulfate, nitrate and the like; and
(v) mixtures thereof.

Examples of Component (c) are the well-known dialkyldi methylammonium salts such as ditallowdimethylammonium chloride, ditallowdimethylammonium methylsulfate, di(hydrogenatedtallow)dimethylammonium chloride, distearyldimethylammonium chloride, dibehenyldimethylammonium chloride. Di(hydrogenatedtallow)di methylammonium chloride and ditallowdimethylammonium chloride are preferred. Examples of commercially available dialkyldimethyl ammonium salts usable in the present invention are di(hydrogenatedtallow)dimethylammonium chloride (trade name Adogen® 442), ditallowdimethylammonium chloride (trade name Adogen® 470), distearyl dimethylammonium chloride (trade name Arosurf® TA-100), all available from Witco Chemical Company. Dibehenyldimethylammonium chloride is sold under the trade name Kemamine Q-2802C by Humko Chemical Division of Witco Chemical Corporation.

Other examples of Component (c) are methylbis(tallowamidoethyl)(2-hydroxyethyl)ammonium methylsulfate and methylbis(hydrogenated tallowamidoethyl)(2-hydroxyethyl)ammonium methylsulfate; these materials are available from Witco Chemical Company under the trade names Varisoft® 222 and Varisoft® 110, respectively: dimethylstearylbenzyl ammonium chloride sold under the trade names Varisoft® SDC by Witco Chemical Company and Ammonyx® 490 by Onyx Chemical Company; 1-methyl-1-tallowamidoethyl-2-tallowimidazolinium methylsulfate and 1-methyl-1-(hydrogenatedtallowamidoethyl)-2-(hydrogenated tallow)imidazolinium methylsulfate ; they are sold under the trade names Varisoft® 475 and Varisoft® 445, respectively, by Witco Chemical Company.

The following are also non-limiting examples of Component (c) (wherein all long-chain alkyl substituents are straight-chain): 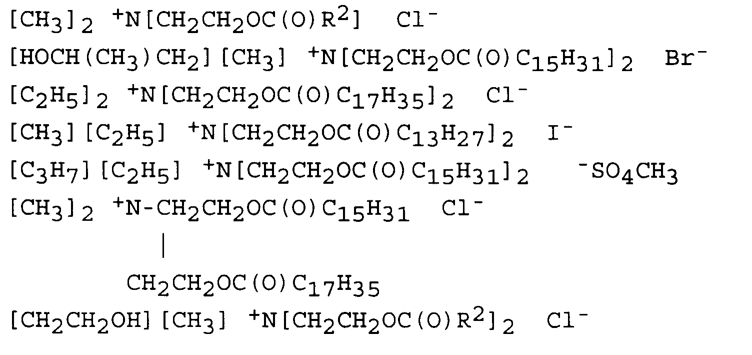 where -C(O)R² is derived from soft tallow and/or hardened tallow fatty acids. Especially preferred is diester of soft and/or hardened tallow fatty acids with di(hydroxyethyl)dimethylammonium chloride, also called di(tallowoyloxyethyl)dimethylammonium chloride.

Since the foregoing compounds (diesters) are somewhat labile to hydrolysis, they should be handled rather carefully when used to formulate the compositions herein. For example, stable liquid compositions herein are formulated at a pH in the range of about 2 to about 5, preferably from about 2 to about 4.5, more preferably from about 2 to about 4. The pH can be adjusted by the addition of a Bronsted acid. Ranges of pH for making stable softener compositions containing diester quaternary ammonium fabric softening compounds are disclosed in U.S. Pat. No. 4,767,547, Straathof and Konig, issued Aug. 30, 1988.

These types of compounds and general methods of making them are disclosed in U.S. Pat. No. 4,137,180, Naik et al., issued Jan. 30, 1979.

A preferred composition contains Component (a) at a level of from about 10% to about 80%, Component (b) at a level of from about 3% to about 40%, and Component (c) at a level of from about 10% to about 80%, by weight of the fabric softening component of the present invention compositions.

An even more preferred composition contains Component (a): the reaction product of about 2 moles of hydrogenated tallow fatty acids with about 1 mole of N-2-hydroxyethylethylenediamine and is present at a level of from about 20% to about 70% by weight of the fabric softening component of the present invention compositions; Component (b): mono(hydrogenated tallow)trimethyl ammonium chloride present at a level of from about 3% to about 30% by weight of the fabric softening component of the present invention compositions; Component (c): selected from the group consisting of di(hydrogenatedtallow)dimethylammonium chloride, ditallowdimethylammonium chloride, methyl-1-tallowamidoethyl-2-tallowimidazolinium methylsulfate, diethanol ester dimethylammonium chloride, and mixtures thereof; wherein Component (c) is present at a level of from about 20% to about 60% by weight of the fabric softening component of the present invention compositions; and wherein the weight ratio of said di(hydrogenated tallow)dimethylammonium chloride to said methyl-1-tallowamidoethyl-2-tallowimidazolinium methylsulfate is from about 2:1 to about 6:1.

In the cationic nitrogenous salts described hereinbefore, the anion A- provides charge neutrality. Most often, the anion used to provide charge neutrality in these salts is a halide, such as chloride or bromide. However, other anions can be used, such as methylsulfate, ethylsulfate, hydroxide, acetate, formate, citrate, sulfate, carbonate, and the like. Chloride and methylsulfate are preferred herein as anion A-.

The amount of fabric softening agent (fabric softener) in liquid compositions of this invention is typically from about 2% to about 50%, preferably from about 4% to about 30%, by weight of the composition. The lower limits are amounts needed to contribute effective fabric softening performance when added to laundry rinse baths in the manner which is customary in home laundry practice. The higher limits are suitable for concentrated products which provide the consumer with more economical usage due to a reduction of packaging and distributing costs.

Fully formulated fabric softening compositions preferably contain, in addition to the hereinbefore described components, one or more of the following ingredients.

Concentrated compositions of the present invention may require organic and/or inorganic concentration aids to go to even higher concentrations and/or to meet higher stability standards depending on the other ingredients. Surfactant concentration aids are typically selected from the group consisting of single long chain alkyl cationic surfactants; nonionic surfactants; amine oxides; fatty acids; or mixtures thereof, typically used at a level of from 0 to about 15% of the composition.

Inorganic viscosity control agents which can also act like or augment the effect of the surfactant concentration aids, include water-soluble, ionizable salts which can also optionally be incorporated into the compositions of the present invention. A wide variety of ionizable salts can be used. Examples of suitable salts are the halides of the Group IA and IIA metals of the Periodic Table of the Elements, e.g., calcium chloride, magnesium chloride, sodium chloride, potassium bromide, and lithium chloride. The ionizable salts are particularly useful during the process of mixing the ingredients to make the compositions herein, and later to obtain the desired viscosity. The amount of ionizable salts used depends on the amount of active ingredients used in the compositions and can be adjusted according to the desires of the formulator. Typical levels of salts used to control the composition viscosity are from about 20 to about 20,000 parts per million (ppm), preferably from about 20 to about 11,000 ppm, by weight of the composition.

Alkylene polyammonium salts can be incorporated into the composition to give viscosity control in addition to or in place of the water-soluble, ionizable salts above. In addition, these agents can act as scavengers, forming ion pairs with anionic detergent carried over from the main wash, in the rinse, and on the fabrics, and may improve softness performance. These agents may stabilize the viscosity over a broader range of temperature, especially at low temperatures, compared to the inorganic electrolytes.

Specific examples of alkylene polyammonium salts include 1-lysine monohydrochloride and 1,5-diammonium 2-methyl pentane dihydrochloride.

Another optional, but preferred, ingredient is a liquid carrier. The liquid carrier employed in the instant compositions is preferably at least primarily water due to its low cost, relative availability, safety, and environmental compatibility. The level of water in the liquid carrier is preferably at least about 50%, most preferably at least about 60%, by weight of the carrier. Mixtures of water and low molecular weight, e.g., <about 200, organic solvent, e.g., lower alcohols such as ethanol, propanol, isopropanol or butanol are useful as the carrier liquid. Low molecular weight alcohols include monohydric, dihydric (glycol, etc.) trihydric (glycerol, etc.), and higher polyhydric (polyols) alcohols.

Still other optional ingredients are Soil Release Polymers, bacteriocides, colorants, perfumes, preservatives, optical brighteners, anti ionisation agents, antifoam agents, and the like.

Enzymes - Enzymes are included in the formulations herein for a wide variety of fabric laundering purposes, including removal of protein-based, carbohydrate-based, or triglyceride-based stains, for example, and for the prevention of refugee dye transfer, and for fabric restoration. The enzymes to be incorporated include proteases, amylases, lipases, cellulases, and peroxidases, as well as mixtures thereof. Other types of enzymes may also be included. They may be of any suitable origin, such as vegetable, animal, bacterial, fungal and yeast origin. However, their choice is governed by several factors such as pH-activity and/or stability optima, thermostability, stability versus active detergents, builders and so on. In this respect bacterial or fungal enzymes are preferred, such as bacterial amylases and proteases, and fungal cellulases.

Enzymes are normally incorporated at levels sufficient to provide up to about 5 mg by weight, more typically about 0.001 mg to about 3 mg, of active enzyme per gram of the composition. Stated otherwise, the compositions herein will typically comprise from about 0.001% to about 5%, preferably 0.01%-2% by weight of a commercial enzyme preparation. Protease enzymes are usually present in such commercial preparations at levels sufficient to provide from 0.005 to 0.1 Anson units (AU) of activity per gram of composition.

Suitable examples of proteases are the subtilisins which are obtained from particular strains of B. subtilis and B. licheniforms. Another suitable protease is obtained from a strain of Bacillus, having maximum activity throughout the pH range of 8-12, developed and sold by Novo Industries A/S under the registered trade name ESPERASE. The preparation of this enzyme and analogous enzymes is described in British Patent Specification No. 1,243,784 of Novo. Proteolytic enzymes suitable for removing protein-based stains that are commercially available include those sold under the tradenames ALCALASE and SAVINASE by Novo Industries A/S (Denmark) and MAXATASE by International Bio-Synthetics, Inc. (The Netherlands). Other proteases include Protease A (see European Patent Application 130,756, published January 9, 1985) and Protease B (see European Patent Application Serial No. 87303761.8, filed April 28, 1987, and European Patent Application 130,756, Bott et al, published January 9, 1985). Other proteases include Protease A (see European Patent Application 130,756, published January 9, 1985) and Protease B (see European Patent Application Serial No. 87303761.8, filed April 28, 1987, and European Patent Application 130,756, Bott et al, published January 9, 1985). Other proteases include Protease A (see European Patent Application 130,756, published January 9, 1985) and Protease B (see European Patent Application Serial No. 87303761.8, filed April 28, 1987, and European Patent Application 130,756, Bott et al, published January 9, 1985). Most preferred is what is called herein "Protease C", which is a variant of an alkaline serine protease from Bacillus, particularly Bacillus lentus, in which arginine replaced lysine at position 27, tyrosine replaced valine at position 104, serine replaced asparagine at position 123, and alanine replaced threonine at position 274. Protease C is described in EP 90915958:4; U.S. Patent No. 5,185,250; and U.S. Patent No. 5,204,015. Also especially preferred are protease which are described in copending application U.S. Serial No. 08/136,797, entitled Protease-containing Cleaning Compositions and copending Application U.S. Serial No. 08/136,626, entitled Bleaching Compositions Comprising Protease Enzymes, which are incorporated herein by reference. Genetically modified variants, particularly of Protease C, are also included herein.

Amylases include, for example, α-amylases described in British Patent Specification No. 1,296,839 (Novo), RAPIDASE, International Bio-Synthetics, Inc. and TERMAMYL, Novo Industries.

The cellulase usable in the present invention include both bacterial or fungal cellulase. Preferably, they will have a pH optimum of between 5 and 9.5. Suitable cellulases are disclosed in U.S. Patent 4,435,307, Barbesgoard et al, issued March 6, 1984, which discloses fungal cellulase produced from Humicola insolens and Humicola strain DSM1800 or a cellulase 212-producing fungus belonging to the genus Aeromonas, and cellulase extracted from the hepatopancreas of a marine mollusk (Dolabella Auricula Solander). Suitable cellulases are also disclosed in GB-A-2.075.028; GB-A-2.095.275 and DE-OS-2.247.832. Cellulases such as CAREZYME (Novo) are especially useful, since they provide additional softening and appearance benefits to fabrics laundered in the present compositions.

Suitable lipase enzymes for detergent usage include those produced by microorganisms of the Pseudomonas group, such as Pseudomonas stutzeri ATCC 19.154, as disclosed in British Patent 1,372,034. See also lipases in Japanese Patent Application 53,20487, laid open to public inspection on February 24, 1978. This lipase is available from Amano Pharmaceutical Co. Ltd., Nagoya, Japan, under the trade name Lipase P "Amano," hereinafter referred to as "Amano-P." Other commercial lipases include Amano-CES, lipases ex Chromobacter viscosum, e.g. Chromobacter viscosum var. lipolyticum NRRLB 3673, commercially available from Toyo Jozo Co., Tagata, Japan; and further Chromobacter viscosum lipases from U.S. Biochemical Corp., U.S.A. and Disoynth Co., The Netherlands, and lipases ex Pseudomonas gladioli. The LIPOLASE enzyme derived from Humicola lanuginosa and commercially available from Novo (see also EPO 341,947) is a preferred lipase for use herein.

Peroxidase enzymes are used in combination with oxygen sources, e.g., percarbonate, perborate, persulfate, hydrogen peroxide, etc. They are used for "solution bleaching," i.e. to prevent transfer of dyes or pigments removed from substrates during wash operations to other substrates in the wash solution. Peroxidase enzymes are known in the art, and include, for example, horseradish peroxidase, ligninase, and haloperoxidase such as chloro-and bromo-peroxidase. Peroxidase-containing detergent compositions are disclosed, for example, in PCT International Application WO 89/099813, published October 19, 1989, by O. Kirk, assigned to Novo Industries A/S. It may be desired to use, in combination with these peroxidases, materials viewed as being peroxidase accelerators such as phenolsulfonate and/or phenothiazine.

A wide range of enzyme materials and means for their incorporation into synthetic detergent compositions are also disclosed in U.S. Patent 3,553,139, issued January 5, 1971 to McCarty et al. Enzymes are further disclosed in U.S. Patent 4,101,457, Place et al, issued July 18, 1978, and in U.S. Patent 4,507,219, Hughes, issued March 26, 1985, both. Enzyme materials useful for liquid detergent formulations, and their incorporation into such formulations, are disclosed in U.S. Patent 4,261,868, Hora et al, issued April 14, 1981.

Enzyme Stabilizers - A preferred optional ingredient for use in the present compositions is enzyme stabilizers. Enzymes for use in detergents can be stabilized by various techniques. Enzyme stabilization techniques are disclosed and exemplified in U.S. Patent 3,600,319, issued August 17, 1971 to Gedge, et al, and European Patent Application Publication No. 0 199 405, Application No. 86200586.5, published October 29, 1986, Venegas. Enzyme stabilization systems are also described, for example, in U.S. Patent 3,519,570. The enzymes employed herein can be stabilized by the presence of water-soluble sources of calcium and/or magnesium ions in the finished compositions which provide such ions to the enzymes. (Calcium ions are generally somewhat more effective than magnesium ions and are preferred herein if only one type of cation is being used.)

Additional stability can be provided by the presence of various other art-disclosed stabilizers, especially borate species: see Severson, U.S. 4,537,706. Typical detergents, especially liquids, will comprise from about 1 to about 30, preferably from about 2 to about 20, more preferably from about 5 to about 15, and most preferably from about 8 to about 12, millimoles of calcium ion per liter of finished composition. This can vary somewhat, depending on the amount of enzyme present and its response to the calcium or magnesium ions. The level of calcium or magnesium ions should be selected so that there is always some minimum level available for the enzyme, after allowing for complexation with builders, fatty acids, etc., in the composition. Any water-soluble calcium or magnesium salt can be used as the source of calcium or magnesium ions, including, but not limited to, calcium chloride, calcium sulfate, calcium malate, calcium maleate, calcium hydroxide, calcium formate, and calcium acetate, and the corresponding magnesium salts. A small amount of calcium ion, generally from about 0.05 to about 0.4 millimoles per liter, is often also present in the composition due to calcium in the enzyme slurry and formula water. In solid detergent compositions the formulation may include a sufficient quantity of a water-soluble calcium ion source to provide such amounts in the laundry liquor. In the alternative, natural water hardness may suffice.

It is to be understood that the foregoing levels of calcium and/or magnesium ions are sufficient to provide enzyme stability. More calcium and/or magnesium ions can be added to the compositions to provide an additional measure of grease removal performance. Accordingly, as a general proposition the compositions herein will typically comprise from about 0.05% to about 2% by weight of a water-soluble source of calcium or magnesium ions, or both. The amount can vary, of course, with the amount and type of enzyme employed in the composition.

The compositions herein may also optionally, but preferably, contain various additional stabilizers, especially borate-type stabilizers. Typically, such stabilizers will be used at levels in the compositions from about 0.25% to about 10%, preferably from about 0.5% to about 5%, more preferably from about 0.75% to about 3%, by weight of boric acid or other borate compound capable of forming boric acid in the composition (calculated on the basis of boric acid). Boric acid is preferred, although other compounds such as boric oxide, borax and other alkali metal borates (e.g., sodium ortho-, meta- and pyroborate, and sodium pentaborate) are suitable. Substituted boric acids (e.g., phenylboronic acid, butane boronic acid, and p-bromo phenylboronic acid) can also be used in place of boric acid. It is to be recognized that such materials may also be used in formulations as the sole stabilizer as well as being used in combination with added calcium and/or magnesium ions.

Finally, it may be desired to add chlorine scavengers, especially to protease-containing compositions, to protect the enzymes from chlorine typically present in municipal water supplies. Such materials are described, for example, in U.S. Patent 4,810,413 to Pancheri et al.

Various other optional adjunct ingredients may also be used to provide fully-formulated detergent compositions. The following ingredients are described for the convenience of the formulator, but are not intended to be limiting thereof.

Detersive Surfactants - Nonlimiting examples of surfactants useful herein typically at levels from about 1% to about 55%, by weight, include the conventional C₁₁-C₁₈ alkyl benzene sulfonates ("LAS") and primary, branched-chain and random C₁₀-C₂₀ alkyl sulfates ("AS"), the C₁₀-C₁₈ secondary (2,3) alkyl sulfates of the formula CH₃(CH₂)ₓ(CHOSO₃⁻M⁺) CH₃ and CH₃(CH₂)_{y}(CHOSO₃⁻M⁺) CH₂CH₃ where x and (y + 1) are integers of at least about 7, preferably at least about 9, and M is a water-solubilizing cation, especially sodium, unsaturated sulfates such as oleyl sulfate, the C₁₀-C₁₈ alkyl alkoxy sulfates ("AEₓS"; especially x up to about 7 EO ethoxy sulfates), C₁₀-C₁₈ alkyl alkoxy carboxylates (especially the EO 1-5 ethoxycarboxylates), the C₁₀₋₁₈ glycerol ethers, the C₁₀-C₁₈ alkyl polyglycosides and their corresponding sulfated polyglycosides, and C₁₂-C₁₈ alpha-sulfonated fatty acid esters. If desired, the conventional nonionic and amphoteric surfactants such as the C₁₂-C₁₈ alkyl ethoxylates ("AE") including the so-called narrow peaked alkyl ethoxylates and C₆-C₁₂ alkyl phenol alkoxylates (especially ethoxylates and mixed ethoxy/propoxy), C₁₂-C₁₈ betaines and sulfobetaines ("sultaines"), C₁₀-C₁₈ amine oxides, and the like, can also be included in the overall compositions. The C₁₀-C₁₈ N-alkyl polyhydroxy fatty acid amides can also be used. Typical examples include the C₁₂-C₁₈ N-methylglucamides. See WO 9,206,154. Other sugar-derived surfactants include the N-alkoxy polyhydroxy fatty acid amides, such as C₁₀-C₁₈ N-(3-methoxypropyl) glucamide. The N-propyl through N-hexyl C₁₂-C₁₈ glucamides can be used for low sudsing. C₁₀-C₂₀ conventional soaps may also be used. If high sudsing is desired, the branched-chain C₁₀-C₁₆ soaps may be used. Mixtures of anionic and nonionic surfactants are especially useful. Other conventional useful surfactants are listed in standard texts.

Builders - Detergent builders can optionally be included in the compositions herein to assist in controlling mineral hardness. Inorganic as well as organic builders can be used. Builders are typically used in fabric laundering compositions to assist in the removal of particulate soils.

The level of builder can vary widely depending upon the end use of the composition and its desired physical form. When present, the compositions will typically comprise at least about 1% builder, preferably from about 1% to about 80%. Liquid formulations typically comprise from about 5% to about 50%, more typically about 5% to about 30%, by weight, of detergent builder. Granular formulations typically comprise from about 1% to about 80%, more typically from about 5% to about 50% by weight, of the detergent builder. Lower or higher levels of builder, however, are not meant to be excluded.

Inorganic or P-containing detergent builders include, but are not limited to, the alkali metal, ammonium and alkanolammonium salts of polyphosphates (exemplified by the tripolyphosphates, pyrophosphates, and glassy polymeric meta-phosphates), phosphonates, phytic acid, silicates, carbonates (including bicarbonates and sesquicarbonates), sulphates, and aluminosilicates. However, non-phosphate builders are required in some locales. Importantly, the compositions herein function surprisingly well even in the presence of the so-called "weak" builders (as compared with phosphates) such as citrate, or in the so-called "underbuilt" situation that may occur with zeolite or layered silicate builders.

Examples of silicate builders are the alkali metal silicates, particularly those having a SiO₂:Na₂O ratio in the range 1.0:1 to 3.2:1 and layered silicates, such as the layered sodium silicates described in U.S. Patent 4,664,839, issued May 12, 1987 to H. P. Rieck. NaSKS-6 is the trademark for a crystalline layered silicate marketed by Hoechst (commonly abbreviated herein as "SKS-6"). Unlike zeolite builders, the Na SKS-6 silicate builder does not contain aluminum. NaSKS-6 has the delta-Na₂Si₂O₅ morphology form of layered silicate. It can be prepared by methods such as those described in German DE-A-3,417,649 and DE-A-3,742,043. SKS-6 is a highly preferred layered silicate for use herein, but other such layered silicates, such as those having the general formula NaMSiₓO₂ₓ₊₁·yH₂O wherein M is sodium or hydrogen, x is a number from 1.9 to 4, preferably 2, and y is a number from 0 to 20, preferably 0 can be used herein. Various other layered silicates from Hoechst include NaSKS-5, NaSKS-7 and NaSKS-11, as the alpha, beta and gamma forms. As noted above, the delta-Na₂Si₂O₅ (NaSKS-6 form) is most preferred for use herein. Other silicates may also be useful such as for example magnesium silicate, which can serve as a crispening agent in granular formulations, as a stabilizing agent for oxygen bleaches, and as a component of suds control systems.

Examples of carbonate builders are the alkaline earth and alkali metal carbonates as disclosed in German Patent Application No. 2,321,001 published on November 15, 1973.

Aluminosilicate builders are useful in the present invention. Aluminosilicate builders are of great importance in most currently marketed heavy duty granular detergent compositions, and can also be a significant builder ingredient in liquid detergent formulations. Aluminosilicate builders include those having the empirical formula:

M_{z/n}[(AlO₂)_{z}(SiO₂)_{y}]·xH₂O

wherein z and y are integers usually of at least 6, the molar ratio of z to y is in the range from 1.0 to 0, and x is an integer from 0 to about 264, and M is a Group IA or IIA element, e.g., Na, K, Mg, Ca with valence n.

Useful aluminosilicate ion exchange materials are commercially available. These aluminosilicates can be crystalline or amorphous in structure and can be naturally-occurring aluminosilicates or synthetically derived. A method for producing aluminosilicate ion exchange materials is disclosed in U.S. Patent 3,985,669, Krummel, et al, issued October 12, 1976. Preferred synthetic crystalline aluminosilicate ion exchange materials useful herein are available under the designations Zeolite A, Zeolite P (B), Zeolite MAP and Zeolite X. In an especially preferred embodiment, the crystalline aluminosilicate ion exchange material has the formula:

Na₁₂[(AlO₂)₁₂(SiO₂)₁₂]·xH₂O

wherein x is from about 20 to about 30, especially about 27. This material is known as Zeolite A. Dehydrated zeolites (x = 0 - 10) may also be used herein. Preferably, the aluminosilicate has a particle size of about 0.1-10 microns in diameter.

Organic detergent builders suitable for the purposes of the present invention include, but are not restricted to, a wide variety of polycarboxylate compounds. As used herein, "polycarboxylate" refers to compounds having a plurality of carboxylate groups, preferably at least 3 carboxylates. Polycarboxylate builder can generally be added to the composition in acid form, but can also be added in the form of a neutralized salt. When utilized in salt form, alkali metals, such as sodium, potassium, and lithium, or alkanolammonium salts are preferred.

Included among the polycarboxylate builders are a variety of categories of useful materials. One important category of polycarboxylate builders encompasses the ether polycarboxylates, including oxydisuccinate, as disclosed in Berg, U.S. Patent 3,128,287, issued April 7, 1964, and Lamberti et al, U.S. Patent 3,635,830, issued January 18, 1972. See also "TMS/TDS" builders of U.S. Patent 4,663,071, issued to Bush et al, on May 5, 1987. Suitable ether polycarboxylates also include cyclic compounds, particularly alicyclic compounds, such as those described in U.S. Patents 3,923,679; 3,835,163; 4,158,635; 4,120,874 and 4,102,903.

Other useful detergency builders include the ether hydroxypolycarboxylates, copolymers of maleic anhydride with ethylene or vinyl methyl ether, 1, 3, 5-trihydroxy benzene-2, 4, 6-trisulphonic acid, and carboxymethyloxysuccinic acid, the various alkali metal, ammonium and substituted ammonium salts of polyacetic acids such as ethylenediamine tetraacetic acid and nitrilotriacetic acid, as well as polycarboxylates such as mellitic acid, pyromellitic, succinic acid, oxydisuccinic acid, polymaleic acid, benzene 1,3,5-tricarboxylic acid, carboxymethyloxysuccinic acid, and soluble salts thereof.

Citrate builders, e.g., citric acid and soluble salts thereof (particularly sodium salt), are polycarboxylate builders of particular importance for heavy duty liquid detergent formulations due to their availability from renewable resources and their biodegradability. Citrates can also be used in granular compositions, especially in combination with zeolite and/or layered silicate builders. Oxydisuccinates are also especially useful in such compositions and combinations.

Also suitable in the detergent compositions of the present invention are the 3,3-dicarboxy-4-oxa-1,6-hexanedioates and the related compounds disclosed in U.S. Patent 4,566,984, Bush, issued January 28, 1986. Useful succinic acid builders include the C₅-C₂₀ alkyl and alkenyl succinic acids and salts thereof. A particularly preferred compound of this type is dodecenylsuccinic acid. Specific examples of succinate builders include: laurylsuccinate, myristylsuccinate, palmitylsuccinate, 2-dodecenylsuccinate (preferred), 2-pentadecenylsuccinate, and the like. Laurylsuccinates are the preferred builders of this group, and are described in European Patent Application 86200690.5/0,200,263, published November 5, 1986.

Other suitable polycarboxylates are disclosed in U.S. Patent 4,144,226, Crutchfield et al, issued March 13, 1979 and in U.S. Patent 3,308,067, Diehl, issued March 7, 1967. See also Diehl U.S. Patent 3,723,322.

Fatty acids, e.g., C₁₂-C₁₈ monocarboxylic acids such as oleic acid and/or its salts, can also be incorporated into the compositions alone, or in combination with the aforesaid builders, especially citrate and/or the succinate builders, to provide additional builder activity. Such use of fatty acids will generally result in a diminution of sudsing, which should be taken into account by the formulator.

In situations where phosphorus-based builders can be used, and especially in the formulation of bars used for hand-laundering operations, the various alkali metal phosphates such as the well-known sodium tripolyphosphates, sodium pyrophosphate and sodium orthophosphate can be used. Phosphonate builders such as ethane-1-hydroxy-1,1-diphosphonate and other known phosphonates (see, for example, U.S. Patents 3,159,581; 3,213,030; 3,422,021; 3,400,148 and 3,422,137) can also be used.

Bleaching Compounds - Bleaching Agents and Bleach Activators - The detergent compositions herein may optionally contain bleaching agents or bleaching compositions containing a bleaching agent and one or more bleach activators. When present, bleaching agents will typically be at levels of from about 1% to about 30%, more typically from about 5% to about 20%, of the detergent composition, especially for fabric laundering. If present, the amount of bleach activators will typically be from about 0.1% to about 60%, more typically from about 0.5% to about 40% of the bleaching composition comprising the bleaching agent-plus-bleach activator.

The bleaching agents used herein can be any of the bleaching agents useful for detergent compositions in textile cleaning or other cleaning purposes that are now known or become known. These include oxygen bleaches as well as other bleaching agents. Perborate bleaches, e.g., sodium perborate (e.g., mono- or tetra-hydrate) can be used herein.

Another category of bleaching agent that can be used without restriction encompasses percarboxylic acid bleaching agents and salts thereof. Suitable examples of this class of agents include magnesium monoperoxyphthalate hexahydrate, the magnesium salt of metachloro perbenzoic acid, 4-nonylamino-4-oxoperoxybutyric acid and diperoxydodecanedioic acid. Such bleaching agents are disclosed in U.S. Patent 4,483,781, Hartman, issued November 20, 1984, U.S. Patent Application 740,446, Burns et al, filed June 3, 1985, European Patent Application 0,133,354, Banks et al, published February 20, 1985, and U.S. Patent 4,412,934, Chung et al, issued November 1, 1983. Highly preferred bleaching agents also include 6-nonylamino-6-oxoperoxycaproic acid as described in U.S. Patent 4,634,551, issued January 6, 1987 to Burns et al.

Peroxygen bleaching agents can also be used. Suitable peroxygen bleaching compounds include sodium carbonate peroxyhydrate and equivalent "percarbonate" bleaches, sodium pyrophosphate peroxyhydrate, urea peroxyhydrate, and sodium peroxide. Persulfate bleach (e.g., OXONE, manufactured commercially by DuPont) can also be used.

A preferred percarbonate bleach comprises dry particles having an average particle size in the range from about 500 micrometers to about 1,000 micrometers, not more than about 10% by weight of said particles being smaller than about 200 micrometers and not more than about 10% by weight of said particles being larger than about 1,250 micrometers. Optionally, the percarbonate can be coated with silicate, borate or water-soluble surfactants. Percarbonate is available from various commercial sources such as FMC, Solvay and Tokai Denka.

Mixtures of bleaching agents can also be used.

Peroxygen bleaching agents, the perborates, the percarbonates, etc., are preferably combined with bleach activators, which lead to the *in situ* production in aqueous solution (i.e., during the washing process) of the peroxy acid corresponding to the bleach activator. Various nonlimiting examples of activators are disclosed in U.S. Patent 4,915,854, issued April 10, 1990 to Mao et al, and U.S. Patent 4,412,934. The nonanoyloxybenzene sulfonate (NOBS) and tetraacetyl ethylene diamine (TAED) activators are typical, and mixtures thereof can also be used. See also U.S. 4,634,551 for other typical bleaches and activators useful herein.

Highly preferred amido-derived bleach activators are those of the formulae:

R¹N(R⁵)C(O)R²C(O)L or R¹C(O)N(R⁵)R²C(O)L

wherein R¹ is an alkyl group containing from about 6 to about 12 carbon atoms, R² is an alkylene containing from 1 to about 6 carbon atoms, R⁵ is H or alkyl, aryl, or alkaryl containing from about 1 to about 10 carbon atoms, and L is any suitable leaving group. A leaving group is any group that is displaced from the bleach activator as a consequence of the nucleophilic attack on the bleach activator by the perhydrolysis anion. A preferred leaving group is phenyl sulfonate.

Preferred examples of bleach activators of the above formulae include (6-octanamido-caproyl)oxybenzenesulfonate, (6-nonanamidocaproyl)oxybenzenesulfonate, (6-decanamidocaproyl)oxybenzenesulfonate, and mixtures thereof as described in U.S. Patent 4,634,551, incorporated herein by reference.

Another class of bleach activators comprises the benzoxazin-type activators disclosed by Hodge et al in U.S. Patent 4,966,723, issued October 30, 1990, incorporated herein by reference. A highly preferred activator of the benzoxazin-type is:

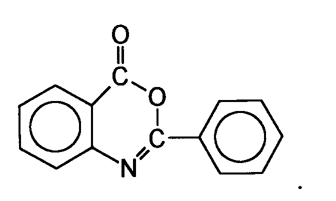

Still another class of preferred bleach activators includes the acyl lactam activators, especially acyl caprolactams and acyl valerolactams of the formulae: 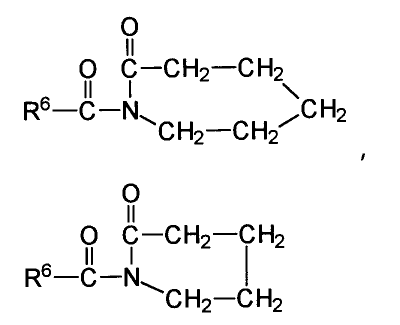 wherein R⁶ is H or an alkyl, aryl, alkoxyaryl, or alkaryl group containing from 1 to about 12 carbon atoms. Highly preferred lactam activators include benzoyl caprolactam, octanoyl caprolactam, 3,5,5-trimethylhexanoyl caprolactam, nonanoyl caprolactam, decanoyl caprolactam, undecenoyl caprolactam, benzoyl valerolactam, octanoyl valerolactam, decanoyl valerolactam, undecenoyl valerolactam, nonanoyl valerolactam, 3,5,5-trimethylhexanoyl valerolactam and mixtures thereof. See also U.S. Patent 4,545,784, issued to Sanderson, October 8, 1985, which discloses acyl caprolactams, including benzoyl caprolactam, adsorbed into sodium perborate.

Bleaching agents other than oxygen bleaching agents are also known in the art and can be utilized herein. One type of non-oxygen bleaching agent of particular interest includes photoactivated bleaching agents such as the sulfonated zinc and/or aluminum phthalocyanines. See U.S. Patent 4,033,718, issued July 5, 1977 to Holcombe et al. If used, detergent compositions will typically contain from about 0.025% to about 1.25%, by weight, of such bleaches, especially sulfonate zinc phthalocyanine.

If desired, the bleaching compounds can be catalyzed by means of a manganese compound. Such compounds are well known in the art and include, for example, the manganese-based catalysts disclosed in U.S. Pat. 5,246,621, U.S. Pat. 5,244,594; U.S. Pat. 5,194,416; U.S. Pat. 5,114,606; and European Pat. App. Pub. Nos. 549,271A1, 549,272A1, 544,440A2, and 544,490A1; Preferred examples of these catalysts include Mn^{IV}₂(u-O)₃(1,4,7-trimethyl-1,4,7-triazacyclononane)₂(PF₆)₂, Mn^{III}₂(u-O)₁(u-OAC)₂(1,4,7-trimethyl-1,4,7-triazacyclononane)₂-(ClO₄)₂, Mn^{IV}₄(u-O)₆(1,4,7-triazacyclononane)₄(ClO₄)₄, Mn^{III}Mn^{IV}₄(u-O)₁(u-OAc)₂₋(1,4,7-trimethyl-1,4,7-triazacyclononane)₂(ClO₄)₃, Mn^{IV}(1,4,7-trimethyl-1,4,7-triazacyclononane)-(OCH₃)₃(PF₆), and mixtures thereof. Other metal-based bleach catalysts include those disclosed in U.S. Pat. 4,430,243 and U.S. Pat. 5,114,611. The use of manganese with various complex ligands to enhance bleaching is also reported in the following United States Patents: 4,728,455; 5,284,944; 5,246,612; 5,256,779; 5,280,117; 5,274,147; 5,153,161; and 5,227,084.

As a practical matter, and not by way of limitation, the compositions and processes herein can be adjusted to provide on the order of at least one part per ten million of the active bleach catalyst species in the aqueous washing liquor, and will preferably provide from about 0.1 ppm to about 700 ppm, more preferably from about 1 ppm to about 500 ppm, of the catalyst species in the laundry liquor.

Other preferred optional ingredients include polymeric soil release agents, materials effective for inhibiting the transfer of dyes from one fabric to another during the cleaning process (i.e., dye transfer inhibiting agents), polymeric dispersing agents, suds suppressors, optical brighteners or other brightening or whitening agents, chelating agents, fabric softening clay, anti-static agents, other active ingredients, carriers, hydrotropes, processing aids, dyes or pigments, solvents for liquid formulations, solid fillers for bar compositions, etc.

Liquid detergent compositions can contain water and other solvents as carriers. Low molecular weight primary or secondary alcohols exemplified by methanol, ethanol, propanol, and isopropanol are suitable. Monohydric alcohols are preferred for solubilizing surfactant, but polyols such as those containing from 2 to about 6 carbon atoms and from 2 to about 6 hydroxy groups (e.g., 1,3-propanediol, ethylene glycol, glycerine, and 1,2-propanediol) can also be used. The compositions may contain from 5% to 90%, typically 10% to 50% of such carriers.

Granular detergents can be prepared, for example, by spray-drying (final product density about 520 g/l) or agglomerating (final product density above about 600 g/l) the Base Granule. The remaining dry ingredients can then be admixed in granular or powder form with the Base Granule, for example in a rotary mixing drum, and the liquid ingredients (e.g., nonionic surfactant and perfume) can be sprayed on.

The detergent compositions herein will preferably be formulated such that, during use in aqueous cleaning operations, the wash water will have a pH of between about 6.5 and about 11, preferably between about 7.5 and 10.5. Laundry products are typically at pH 9-11. Techniques for controlling pH at recommended usage levels include the use of buffers, alkalis, acids, etc., and are well known to those skilled in the art.

The following examples illustrate the esters and compositions of this invention, but are not intended to be limiting thereof.

### EXAMPLES

In all these examples, betaine ester of an alcohol means a betaine ester with a trimethylammonium quaternary center (acohoxycarbonyl-N,N,N-trimethylmethanaminium)

### Hydrolysis profile of the betaine ester of geraniol

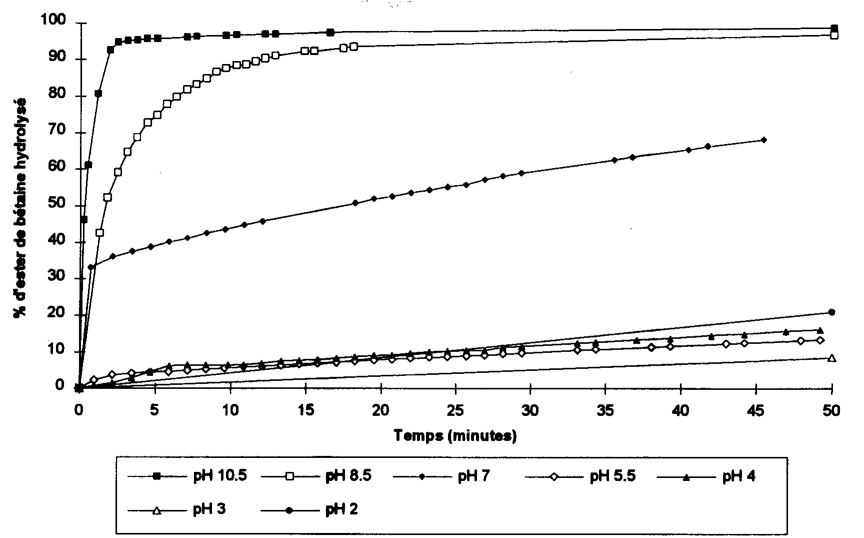

Test realised under pH-stat configuartion, at controlled temperature (40°C). Concentration in betaine ester used 1.5.10⁻² mol/l

The hydrolysis profile of the betaine ester of geraniol is very pH dependent and similar to the one reported in the art.

### Examples of applications in a fabric-softener matrix

In these following tests, the intensity of the perfume has been evaluated by trained perfumers and allocated an intensity rating varying between 0 (no odour detected) and 100 (perfection). The higher the rating, the stronger the odour.
In this instance, a trained perfumer is defined as a person having at least 6 months training with demonstrated evidence of olfactive sensitivity.

| **Intensity rating** | **Intensity on fabric** |
|---|---|
| 100 | Excellent |
| 75 | Very good |
| 50 | Good |
| 25 | Fair |
| 0 | None |

### Example 1

Perfume intensity on fabric using betaine esters versus the free alcohol: test realised in a beaker

### 1) Geraniol

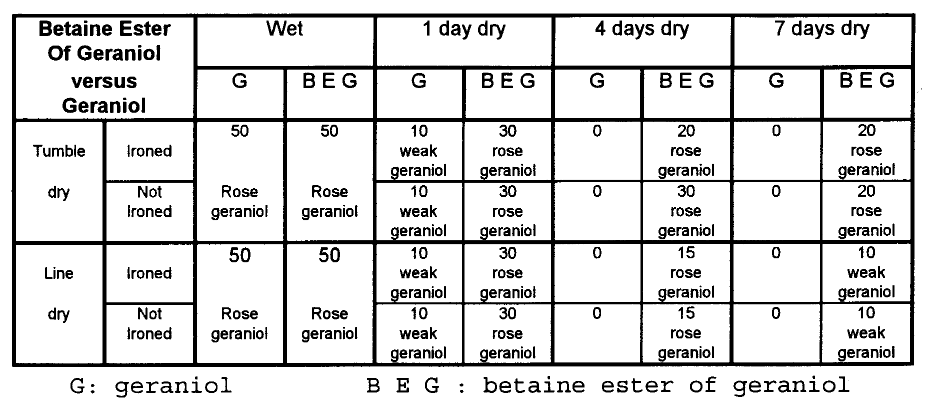

In both cases, 100 ppm of geraniol or betaine ester of geraniol are used. Geraniol or betaine ester of geraniol are added to an unperfumed matrx of fabric-softener Test done by adding the mixture of unperfumed fabric-softener + (G or B E G) to 2 litres of water and stir for 5 minutes (magnetic stirrer). Fabric used: cotton terry towels.

### 2) Citronellol 1

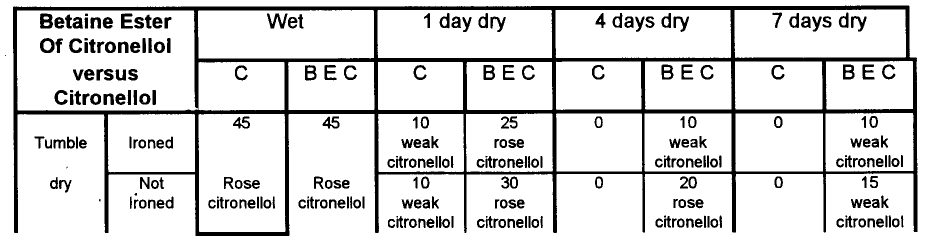

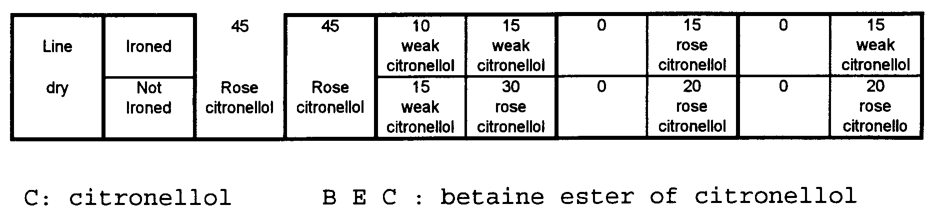

In both cases, 100 ppm of citronellol or betaine ester of citronellol are used. Citronellol or betaine ester of citronellol are added to an unperfumed matrx of fabric softener.
Test done by adding the mixture of unperfumed fabric-softener + (C or B E C) to 2 litres of water and stir for 5 minutes (magnetic stirrer). Fabric used: cotton terry towels

### Example 2)

Perfume intensity on fabric using betaine ester of geraniol versus the free geraniol: test realised under real wash conditions

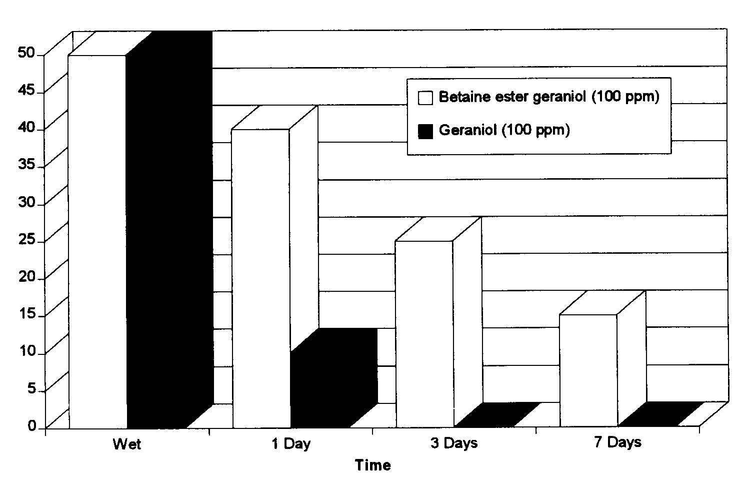

In both cases, 100 ppm of geraniol or betaine ester of geraniol are used. Geraniol or betaine ester of geraniol are added to an unperfumed matrx of fabric-softener.
Test done by adding the mixture unperfumed fabric-softener + (G or B E G) to the rinse cycle of a wash. Fabric used in the load: clean cotton terry towels

### Example 3)

Long lasting high perfume intensity on fabric by adding betaine ester of geraniol on top of current perfume fabric-softener versus the current perfume only or with a similar level of 10 fold higher level of geraniol.

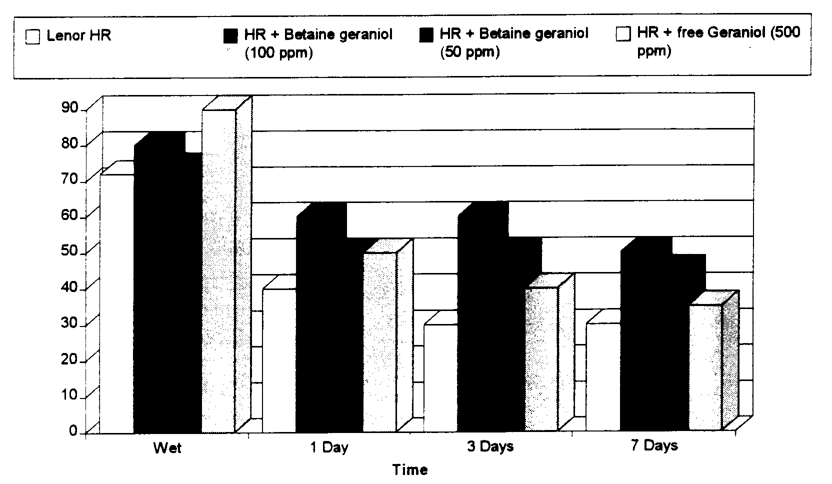

Betaine ester allows us to achieve an higher intensity on fabric, for at least a week, compared to the current perfume used in fabric-softeners. Moreover, using a level of free geraniol ten times higher does not provide the same benefit due to the high volatility of this perfume alcohol. This higher perfume intensity translates into a high consumer preference for the fabric treated with HR+ betaine ester of geraniol

| **Survey done on 20 people using the towels from the previous graph.** **Question: Which towel is the most perfumed?** | | | |
|---|---|---|---|
| | HR + 50 ppm Betaine geraniol | Lenor HR only | None |
| After 1 day | 18 | 2 | 0 |
| After 3 days | 15 | 1 | 4 |
| After 7 days | 19 | 1 | 0 |

### Example 4)

Using betaine ester on top of an actual perfume leads to an improved longevity of the perfume intensity whatever the perfume alcohol used to prepare the betaine ester.

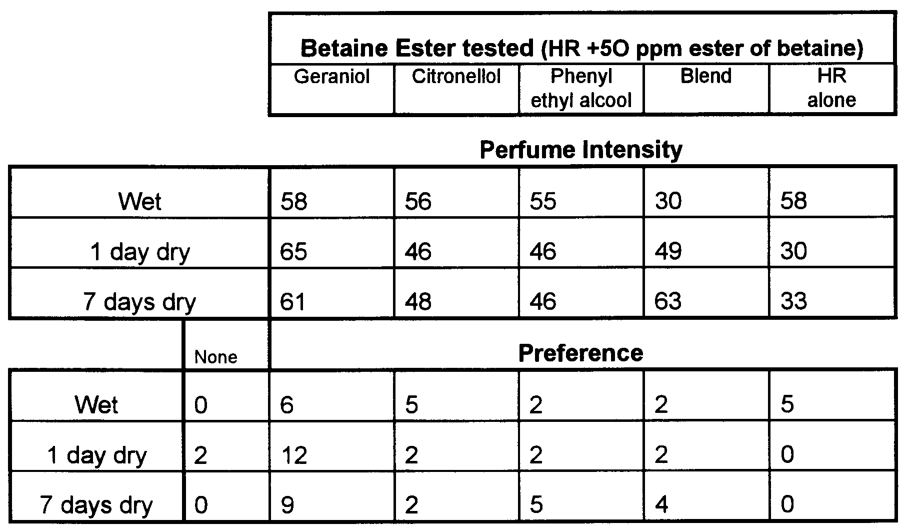

Preference test done on 20 people. Question asked: which towel do you think is the more perfumed? Possiblity to answer "no difference" (column none)
The blend is a mixture of four different betaine esters (phenylethylalcool, geraniol, citronellol and phenoxanol)

### Example 5

In the literature betaine esters are prepared by first preparing the halogenoacetate ester, preferably chloroacetate ester, and then quaternizing the ester with the required tertiary amine.

The first step, the synthesis of halogenoacetate esters by reaction of an alcohol with an halogenoacetic acid halide is widely described in the prior art for primary alcohols or phenols.
Typically, the alcohol is stirred with chloroacetyl chloride, in presence of a catalyst such as a tertiary amines (triethylamine, tributylamine, etc...), sterically hindered secondary amines (diisopropylamine), pyridine and substituted pyridine, in particular 4-diloweralkylaminopyridine such as 4-dimethylaminopyridine, quaternary ammonium salts(tetramethylammonium bromide or chloride, etc.), quaternary phosphonium salts (tetrabutylphosphonium chloride, etc.).
This catalyst can be used at a level as low as 0.1% and up to slightly above a stoichiometric quantity compared to chloroacetyl chloride or the alcohol
The molar ratio of the alcohol to the chloroacetyl chloride normally varies between 0.9 and 1.1.

When such a process is used with sterically unhindered alcohols (such as primary perfume alcohols, phenols and menthol, a secondary alcohol), the expected chloroacetate ester is quickly obtained, in very good yields and purity. Equimolar quantities of alcohol/phenol, chloroacetyl chloride and pyridine are stirred at room temperature for 4 hours, in a nonpolar to moderately polar aprotic solvent such as hexane, toluene, dichloromethane or chloroform.
But when this same process is reapplied for the synthesis of chloroacetate esters of a very sterically hindered alcohol (linalool, a tertiary alcohol), a far higher temperature and a longer reaction time is needed to achieve a similar yield. Moreover, the purity of the reaction mixture obtained is far lower than for a primary alcohol or a phenol. With a tertiary alcohol, side reactions occurs to a substantial extent. For the esterification of linalool with chloroacetyl chloride, these side-reactions products account for around 20% of all products detected, in the reaction mixture.

In the prior art, no mention is made of the esterification route to prepare the halogenoacetate ester of an hindered alcohol (and especially tertiary alcohols).
It is found that replacing chloroacetyl chloride by chloroacetic anhydride greatly accelerates the kinetics of esterification and improves the selectivity of the reaction. With chloroacetic anhydride and pyridine, linalyl chloroacetate is obtained in 90% yield after only 2h30 hours at room temperature. Moreover, no side reaction products could then be detected by GC/MS and 1H/13 C NMR in the final mixture.
The molar ratio of chloroacetic anhydride to sterically hindered alcohols is between 0.95 and 1.5 preferably 0.95 and 1.10
The molar ratio of catalyst to sterically hindered alcohols is between 0.95 and 1.5 preferably 0.95 and 1.10

If the halogen on the methylene group of the halogenoacetate ester does not have to be a chloride, then using bromoacetyl bromide rather than chloroacetyl chloride is far more appropriate. As for chloroacetic anhydride, it is found that replacing chloroacetyl chloride by bromoacetyl bromide accelerates the kinetic of esterification and improves the selectivity of the reaction, no side reaction product being detected. With pyridine as catalyst, linalyl bromoacetate was obtained in 95% yield after only 5 hours at 50°C. Similar results were obtained with tetrahydrolinalool and 1,2-dihydromyrcenol.

Both chloroacetic anhydride and bromoacetyl bromide can be used to prepare respectively the chloroacetate and bromoacetate esters of any class of alcohol, not only tertiary alcohols but also primary and secondary alcohols as well as phenols. In both cases the use of a catalyst as previously described leads to an improved rate of esterification and a higher selectivity of reaction.

### Citronellyl Chloroacetate

Chloroacetyl chloride (0.08 mol, 6.4 ml) was mixed with sodium-dried toluene (30 ml), in a 150 ml conical flask, cooled on a cold water-bath. To this solution were added dropwise a mixture of citronellol (0.08 mol, 14.8 ml), pyridine (0.08 mol, 6.4 ml) in toluene (10 ml), the total addition time being about 15-30 minutes. The dropping funnel was fitted with a calcium chloride drying tube. The reaction mixture was left to stirr over the cold water-bath for three hours. Then, the white precipitate of pyridinium chloride was filtered off and the reaction mixture was washed with distiled water (2x50 ml), dried over MgSO₄ and the solvent removed under reduced pressure, yielding citronellyl chloroacetate as a slightly yellow oil (18.35 g, 99%)
C₁₂H₂₁ClO₂ M= 232.75 g/mol
IR: n C=O 1760 and 1736 cm⁻¹, n C=C 1605 cm⁻¹, n O=C-O-C 1289 cm⁻¹, n CO-O-C 1179 cm⁻¹, n C-Cl 731 cm⁻¹,
δH: (270 MHz, CDCl₃) 0.9 (3H, d, CH₃), 1.00-1.80 (CH₂, CH, m, 5H), 1.60 and 1.69 (CH₃-C=, s,6H), 2.00 (=CH-CH₂, m, 2H), 4.07 (CH2OOCCH2Cl, s, 2H), 4.20 (CH2-OOC, t, 2H), 5.08 (=CH, t, J 5.5 Hz, 1H)

### Citronellyl Betainate

To a solution of citronellyl chloroacetate (0.079 mol, 18.35 g) in toluene (80 ml), cooled over a salted ice bath was added trimethyl amine (0.24 mol, 22 ml). The reaction mixture was stirred for 6 hours at around 0oC and then for an extra 18 hours at room temperature, yielding a white solid which was isolated on a glass filter and washed carefully with ether (3x100 ml). The product was recrystallized from acetonitrile yielding a fine white solid (18.5 g, 80.3%).
C₁₅H₃₀ClNO₂ M= 291.9 g/mol mp 68-69°C
δH: (270 MHz, CDCl₃) 0.9 (3H, d, CH₃), 1.00-1.80 (CH₂, CH, 5H), 1.60 and 1.69 (CH₃-C=, s,6H), 2.00 (=CH-CH₂, m, 2H), 3.65 (CH3-N⁺, s, 9H), 4.20 (CH2-OOC, t, 2H), 4.98 (CH2OOCCH2N⁺, s, 2H), 5.08 (=CH,t, J 7.5 Hz, 1H)
IS/MS (C₁₅H₃₀NO₂)⁺ M= 256.4 m/z= 256

### Geranyl Chloroacetate

Chloroacetyl chloride (0.4 mol, 32 ml) was mixed with dichloromethane (250 ml), in a 500 ml conical flask, cooled on a cold water-bath. To this solution were added dropwise a mixture of geraniol (0.4 mol, 70 ml), pyridine (0.4 mol, 32 ml) in dichloromethane (100 ml), the total addition time being about 15-30 minutes. The dropping funnel was fitted with a calcium chloride drying tube. The reaction mixture was left to stirr over the cold water-bath for three hours. Then, the white precipitate of pyridinium chloride was filtered off and the reaction mixture was washed with distiled water (3x350 ml), dried over MgSO₄ and the solvent removed under reduced pressure, yielding geranyl chloroacetate as a colourless oil (86.5 g , 93.7%)
C₁₂H₁₉ClO₂ M= 230.73 g/mol
IR: n C=O 1762 and 1739 cm⁻¹, n C=C 1670 cm⁻¹ and 1605 cm⁻¹, n O=C-O-C 1285 cm⁻¹, n CO-O-C 1168 cm⁻¹, n C-Cl 728 cm⁻¹,
δH: (270 MHz, CDCl₃) 1.60 (CH₃-C=, s, 3H), 1.68 and 1.72 (CH₃-C=, s, 6H), 2.07 (=CH-CH₂, =C(CH₃)-CH₂, m, 4H), 4.07 (CH₂OOCCH₂Cl, s, 2H), 4.71 (=CH-CH₂-OOC, d, J 7Hz, 2H), 5.08 (=CH, t, J 5.5 Hz, 1H), 5.35 (=CH-CH₂OOC, t, J 8 Hz, 1H)
δC/DEPT: (70 MHz, CDCl₃) 16.2 17.4 25.4 CH₃-C=, 26.0 =CH-CH₂-CH₂, 39.3 =C(CH₃)-CH₂, 40.7 CH₂OOCCH₂Cl, 62.7 =CH-CH₂-OOC, 117.3 123.4 =CH, 131.6 =C(CH₃)₂, 143.2 =C(CH₃)-CH₂, 167.0 C=O

### Geranyl bromoacetate

Bromoacetyl bromide (0.055 mol, 4.74 ml) was mixed with dichloromethane (80 ml), in a 150 ml conical flask, cooled on a cold water-bath. To this solution were added dropwise a mixture of geraniol (0.055 mol, 9.6 ml), pyridine (0.055 mol, 4.4 ml) in dichloromethane (20 ml), the total addition time being about 15-30 minutes. The dropping funnel was fitted with a calcium chloride drying tube. The reaction mixture was left to stirr over the cold water-bath for five hours. Then, the white precipitate of pyridinium chloride was filtered off and the reaction mixture was washed with distiled water (3x150 ml), dried over MgSO₄ and the solvent removed under reduced pressure, yielding geranyl bromoacetate as a dark brown oil (14.2 g , 88.5%)
C₁₂H₁₉BrO₂ M= 275.19 g/mol
IR: n C=O 1762 and 1737 cm⁻¹, n C=C 1669 cm⁻¹ and 1637 cm⁻¹, n C-O ester 1281 cm⁻¹, n C-Br 675 cm⁻¹,
δH: (270 MHz, CDCl₃) 1.60 (CH₃-C=, s, 3H), 1.68 and 1.72 (CH₃-C=, s, 6H), 2.07 (=CH-CH₂, =C(CH₃)-CH₂, m, 4H), 3.84 (CH₂OOCCH₂Br, s, 2H), 4.68 (=CH-CH₂-OOC, d, J 7Hz, 2H), 5.07 ((CH₃)₂C=CH, t, J 5.5 Hz, 1H), 5.35 (=CH-CH₂OOC, t, J 7 Hz, 1H)
δC/DEPT: (70 MHz, CDCl₃) 16.4 17.5 25.5 CH₃-C=, 25.9 CH₂OOCCH₂Br, 26.1 =CH-CH₂-CH₂, 39.4 =C(CH₃)-CH₂, 62.9 =CH-CH₂-OOC, 117.3 =CH-CH₂OOC, 123.5 (CH₃)₂C=CH, 131.7 =C(CH₃)₂, 143.3 =C(CH₃)-CH₂, 167.0 C=O

### H-C COSY:

Direct coupling of the signals at 1.60 1.68 and 1.72 ppm (1H) with 16.3 17.5 and 25.5 ppm (13C)
Direct coupling of the multiplet at 2.07 ppm (1H) with 26.1 and 39.3 ppm (13C)
Direct coupling of the singulet at 3.84 ppm (1H) with 25.9 ppm (13C)
Direct coupling of the doublet at 4.68 ppm (1H) with 62.9 ppm (13C)
Direct coupling of the multiplet at 5.07 ppm (1H) with 123.5 ppm (13C)
Direct coupling of the triplet at 5.32 ppm (1H) with 117.3 ppm (13C)

### Geranyl a-Chlorophenylacetate

a-Chlorophenylacetyl chloride (0.1 mol, 14.54 ml) was mixed with dichloromethane (100 ml), in a 250 ml conical flask, cooled on a cold water-bath. To this solution was added dropwise a mixture of geraniol (0.1 mol, 17.5 ml), pyridine (0.1 mol, 8 ml) in dichloromethane (25 ml), the total addition time being about 15-30 minutes. The dropping funnel was fitted with a calcium chloride drying tube. The reaction mixture was left to stirr over the cold water-bath for four hours. Then, the white precipitate of pyridinium chloride was filtered off and the reaction mixture was washed with distiled water (3x150 ml), dried over MgSO₄ and the solvent removed under reduced pressure, yielding geranyl a-chlorophenylacetate as a light yellow oil (27.6 g, 90.1%)
C₁₈H₂₃ClO₂ M= 306.83 g/mol
IR: n C=O 1753 and 1739 cm⁻¹, n C=C 1668 cm⁻¹ and 1601 cm⁻¹, X O=C-O-C 1283 cm⁻¹, n CO-O-C 1155 cm⁻¹, n C-Cl 728 cm⁻¹,
δH: (270 MHz, CDCl₃) 1.57 (CH₃-C=, s, 3H), 1.63 and 1.66 (CH₃-C=, s, 6H), 2.04 (=CH-CH₂, =C(CH₃)-CH₂, m, 4H), 4.64 (=CH-CH₂-OOC, m, 2H), 5.05 (=CH, t, J 6.6 Hz, 1H), 5.35 (=CH-CH₂OOC, t, J 6.6 Hz, 1H), 5.34 (CH₂OOCCHClPh, s, 1H), 7.29-7.35 (H aromatic, m, 3H), 7.45-7.48 (H aromatic, m, 2H)
δC/DEPT: (70 MHz, CDCl₃) 16.0 17.4 25.4 CH₃-C=, 26.0 =CH-CH₂-CH₂, 39.2 =C(CH₃)-CH₂, 58.9 CH₂OOCCHClPh, 62.9 =CH-CH₂-OOC, 117.2 123.4 =CH geranyl, 127.7 128.5 128.9 =CH aromatic, 131.5 =C(CH₃)₂, 135.7 C-CHCl aromatic, 143.2 =C(CH₃)-CH₂, 168.0 C=O

### Geranyl Betainate (geranyloxycarbonyl-N,N,N-trimethylmethanaminium chloride)

Trimethyl amine (0.6 mol, 55 ml) was added to a solution of geranyl chloroacetate (0.375 mol, 86.5g), in acetone (300 ml), cooled over a salted ice bath. The reaction mixture was stirred for 6 hours at around 0^{o}C and for an extra 18 hours at room temperature. Then, more trimethylamine (0.44 mol, 40 ml) was added and the reaction mixture was stirred for an extra 48 hours at room temperature, yielding a white gel which was isolated by centrifugation and washed carefully with ether (3x100 ml). The product was recrystallized from acetonitrile yielding geranyl betainate as a fine white solid (74.6 g, 68.6%).
C₁₅H₂₈ClNO₂ M= 289.9 g/mol mp 92°C
δH: (270 MHz, CDCl₃) 1.61 (CH₃-C=, s, 3H), 1.69 and 1.72 (CH₃-C=, s, 6H), 2.07 (=CH-CH₂, =C(CH₃)-CH₂, m, 4H), 3.67 (CH3-N⁺, s, 9H), 4.71 (=CH-CH₂-OOC, d, J 7.5 Hz, 2H), 5.04 (CH₂OOCCH₂N⁺, s, 2H), 5.08 (=CH, t, J 5.5 Hz, 1H), 5.31 (=CH-CH₂OOC, t, J 8 Hz, 1H)
δC: (70 MHz, CDCl₃) 16.5 17.7 25.6 26.2 CH₃-C= =CH-CH₂-CH₂, 39.5 =C(CH₃)-CH₂, 54.1 (CH₃)₂-N⁺, 63.0 63.4 =CH-CH₂-OOC CH₂OOCCH₂N⁺, 116.6 123.5 =CH, 132.0 =C(CH₃)₂, 144.3 =C(CH₃)-CH₂, 164.8 C=O
IS/MS (C₁₅H₂₈NO₂)⁺ M= 256.4 Found m/z= 254

| | | | |
|---|---|---|---|
| Anal Calcd for C₁₅H₂₈ClNO₂ | C 62.16 | H 9.74 | N4.83 |
| C₁₅H₂₈ClNO₂.H2O | C 58.52 | H 9.82 | N4.55 |
| Found: | C 59.77 | H 9.85 | N4.61 |

### Geranyloxycarbonyl-N,N,N-trimethylmethanaminium bromide

Trimethylamine (0.15 mol, 13.75 ml) is added to a solution of geranyl bromoacetate (0.15 mol, 41.28 g), in acetone (300 ml), cooled over a salted ice bath. The reaction mixture is stirred for 3 hours at room temperature after which the mixture has completely turned to a solid mass. Then, more trimethylamine (0.15 mol, 13.75 ml) and acetone (100 ml) are added and the reaction mixture is warmed up to 35-40°C and maintained at this temperature for one hour. The solvent is then removed under vacuum, yielding a white gel which is stirred overnight in ether (250 ml). The white solid formed is filtered, washed with more ether(3*100 ml) and recrystallized from petroleum ether 60-80°C/ethanol (75%/25% v/v), yielding the product as a fine white solid (34.25 g, 68.3%).
Spectra as before.

### Geranyloxycarbonyl-N-butyl-N,N-dimethylmethanaminium bromide

Geranylbromoacetate (0.0545 mol, 15g) was mixed with N,N-dimethylbutylamine (0.109 mol, 15.26 ml), in chloroform (100 ml). The reaction mixture is stirred at room temperature for 24 hours, during which it turns dark brown. Then, the solution is dissolved in buffered acidic water (pH=3) and washed with diethyl ether (2*50 ml). The aqueous phase is then extracted with chloroform (3*50 ml). The chloroform phases are combined, dried over MgSO₄ and concentrated under vacuum, yielding geranyloxycarbonyl-N-butyl-N,N-dimethylmethanaminium bromide as a brown oil (17.5 g, 85%).
C₁₈H₃₄BrNO₂ M= 376.4 g/mol
δH: (270 MHz, CDCl₃) 0.99 (CH₃CH₂, t, J 7.5 Hz, 3H), 1.42 (CH₃CH₂, q, J 7.5 Hz, 2H), 1.60-1.85 (CH₃-C=, ⁺NCH₂CH₂, 11H), 2.07 (=CH-CH₂, =C(CH₃)-CH₂, m, 4H), 3.64 (CH3-N⁺, s, 6H), 3.86 (CH₂CH₂N⁺, t, J 9 Hz, 2H), 4.71 (=CH-CH₂-OOC, d, J 7.5 Hz, 2H), 4.88 (CH₂OOCCH₂N⁺, s, 2H), 5.07 (=CH, t, J 5.5 Hz, 1H), 5.31 (=CH-CH₂OOC, t, J 8 Hz, 1H)
δC: (70 MHz, CDCl₃) 13.5 CH₃CH₂, 16.2 17.6 19.4 24.7 25.6 26.1 CH₂ CH₃-C= =CH-CH₂-CH₂, 39.5 =C(CH₃)-CH₂, 51.7 (CH₃)₂-N⁺, 61.2 63.3 64.4 CH₂CH₂N⁺ =CH-CH₂-OOC CH₂OOCCH₂N⁺, 116.5 123.4 =CH, 131.9 =C(CH₃)₂, 144.3 =C(CH₃)-CH₂, 164.5 C=O
IS/MS (C₁₈H₃₄NO₂)⁺ M= 298.4 Found m/z= 298

### Geranyloxycarbonyl-N,N-dimethyl-N-propanolmethanaminium bromide

Geranyl bromoacetate (0.018 mol, 5g) was mixed with N,N-dimethylpropanolamine(0.018 mol, 2.13 ml), in chloroform (30 ml). The reaction mixture is stirred at room temperature for 48 hours. After removal of the solvent, the oil is disolved in buffered acidic water (pH=3, 75 ml) and washed with diethyl ether (3*100 ml). The aqueous phase is then saturated with sodium chloride and extracted with dichloromethane (3*150 ml). The dichloromethane phases are combined, dried over MgSO₄, filtered and the solvent is removed under reduced pressure, yielding geranylethyloxycarbonyl-N,N-dimethyl-N-propanolmethanaminium bromide as a yellow oil (3.9 g , 57.3%)
C₁₇H₃₂BrNO₃ M= 378.35 g/mol
δH: (270 MHz, CDCl₃) 1.61 (CH₃-C=, s, 3H), 1.69 and 1.72 (CH₃-C=, s, 6H), 2.07 (=CH-CH₂, =C(CH₃)-CH₂, ⁺NCH₂-CH₂-CH₂OH, m, 6H), 3.56 (CH3-N⁺, s, 6H), 3.72 (CH₂CH₂N⁺, t, J 8 Hz, 2H), 4.01 (CH₂OH , t, J 5.5 Hz, 2H), 4.40 (CH₂OH, m, 1H), 4.68 (CH₂OOCCH₂N⁺, s, 2H), 4.72 (=CH-CH₂-OOC, d, J 7.5 Hz, 2H), 5.08 (=CH, t, J 5.5 Hz, 1H), 5.31 (=CH-CH₂OOC, t, J 8 Hz, 1H)

### Geranyloxycarbonyl-pyridylmethanaminium bromide (N 940 p 45)

Geranylbromoacetate (0.0727 mol, 2g) was mixed with pyridine (0.109 mol, 1.76 ml), in chloroform (50 ml). The reaction mixture is stirred at room temperature for 120 hours after which the chloroform is removed under vacuum. The yellow loil obtained is then carefully washed with ether (2*50 ml) and under vacuum, yielding geranyloxycarbonyl-pyridylmethanaminium bromide as a yellow gum (2.3 g, 89%).
C₁₇H₂₄BrNO₂ M= 354.3 g/mol
δH: (270 MHz, CDCl₃) 1.60 (CH₃-C=, s, 3H), 1.67 and 1.70 (CH₃-C=, s, 6H), 2.06 (=CH-CH₂, =C(CH₃)-CH₂, m, 4H), 4.74 (=CH-CH₂-OOC, d, J 7 Hz, 2H), 5.07 (=CH, t, J 5.5 Hz, 1H), 5.34 (=CH-CH₂OOC, t, J 8 Hz, 1H), 6.27 (CH₂OOCCH₂N⁺, s, 2H), 8.14 (d d, J1 6.5 Hz, J2 7.5 Hz, 2H pyridine), 8.61 (t, J 8 Hz, 1H pyridine), 9.48 (d, J 5.5 Hz, 2H pyridine)

### Phenylethyl chloroacetate

Chloroacetyl chloride (0.2 mol, 16 ml) was mixed with dichloromethane (80 ml), in a 150 ml conical flask, cooled on a cold water-bath. To this solution were added dropwise a mixture of phenylethyl alcohol (0.2 mol, 24 ml), pyridine (0.2 mol, 16 ml) in dichloromethane (20 ml), the total addition time being about 15-30 minutes. The reaction mixture was left to stirr over the cold water-bath for three hours. Then, the white precipitate of pyridinium chloride was filtered off and the reaction mixture was washed with distiled water (3x100 ml), dried over MgSO₄ and the solvent removed under reduced pressure, yielding phenylethyl chloroacetate as a brown oil (36.5 g , 93.5%)
C₁₀H₁₁ClO₂ M= 198.65 g/mol
IR: n C=O 1758 and 1739 cm⁻¹, n C=C aromatic 1605 cm⁻¹, n O=C-O-C 1285 cm⁻¹, n CO-O-C 1172 cm⁻¹, n C-Cl 751 cm⁻¹,
δH: (270 MHz, CDCl₃) 2.97 (Ar-CH₂, t, J 7 Hz, 2H), 4.03 (CH₂OOCCH₂Cl, s, 2H), 4.39 (CH₂-OOC, t, J 7Hz, 2H), 7.23 7.29 7.31 (H aromatic, m, 5H)
δC: (70 MHz, CDCl₃) 33.4 Ar-CH₂, 39.7 CH₂OOCCH₂Cl, 64.8 CH₂-OOC, 125.6 C2/C6 aromatic, 127.7 C4 aromatic, 128 C3/C5 aromatic, 136.8 C1 aromatic, 166 C=O ester

### Phenylethyl Betainate (phenylethyloxycarbonyl-N,N,N-trimethylmethanaminium chloride)

Trimethyl amine (0.3 mol, 27.5 ml) was added to a solution, cooled over a salted ice bath, of phenyethylyl chloroacetate (0.184 mol, 36.5g) in ether (75 ml). The reaction mixture was stirred for 6 hours at around 0^{o}C and then for an extra 12 hours at room temperature, yielding a white precipitate which was isolated on a glass filter and washed carefully with ether (3x100 ml). The product was recrystallized from acetonitrile yielding a fine white solid (41.8g, 87.6%).
C₁₃H₂₀ClNO₂ M= 259.8 g/mol mp 159 °C
δH: (270 MHz, CDCl₃) 2.97 (Ar-CH₂, t, J 7 Hz, 2H), 3.56 ((CH₃)₃N⁺, s, 9H), 4.41 (CH₂-OOC, t, J 7Hz, 2H), 5.08 (CH₂OOCCH₂N⁺, s, 2H), 7.23 7.29 7.33 (H aromatic, m, 5H)
δC: (70 MHz, CDCl₃) 34.8 Ar-CH₂, 54.2 (CH₃)₃N⁺, 64.4 CH₂-OOC, 66.6 CH₂OOCCH₂N⁺, 126.9 C2/C6 aromatic, 128.7 C4 aromatic, 129 C3/C5 aromatic, 137 C1 aromatic, 164.8 C=O ester

| | | | |
|---|---|---|---|
| Anal Calcd for C₁₃H₂₀ClNO₂ | C 60.58 | H 7.82 | N 5.43 |
| C₁₃H₂₀ClNO₂.H₂O | C 56.62 | H 8.04 | N 5.08 |
| Found: | C 60.48 | H 8.58 | N 5.35 |

### Phenylethyloxycarbonyl-N,N-dimethyl-N-propanolmethanaminium chloride

Phenylethylchloroacetate (0.04 mol, 7.96g) was mixed with N,N-dimethylpropanolamine(0.04 mol, 4.7 ml), in diethyl ether (50 ml). The reaction mixture is stirred at room temperature for 48 hours during which dark brown gum forms at the bottom of the flask. The ether is quickly removed and the gum is carefully washed with ether (2*50 ml). It is then disolved in buffered acidic water (pH=3, 75 ml) and washed with ether (3*75 ml). The aqueous phase is then saturated with sodium chloride and extracted with dichloromethane (3*75 ml). The dichloromethane phases are combined, dried over MgSO₄, filtered and the solvent is removed under reduced pressure, yielding phenylethyloxycarbonyl-N,N-dimethyl-N-propanolmethanaminium chloride as a brown gum (7.2 g , 59.6%)
C₁₅H₂₄ClNO₃ M= 301.8 g/mol
δH: (270 MHz, CD₃OD) 1.92 (HOCH₂CH₂, m, 2H), 2.98 (Ar-CH₂, t, J 7 Hz, 2H), 3.42 ((CH₃)₃N⁺, s, 6H), 3.62 (CH₂CH₂N⁺, t, J 8 Hz, 2H), 3.85 (CH₂OH , t, j 5.5 Hz, 2H), 4.48 (CH₂-OOC, t, J 7Hz, 2H), 4.71 (CH₂OOCCH₂N⁺, s, 2H), 7.23 7.29 7.33 (H aromatic, m, 5H)
δC: (70 MHz, CDCl₃) 25.9 CH₂CH₂OH, 34.8 Ar-CH₂, 51.5 (CH₃)₂N⁺, 57.9 CH₂OH, 61.1 63.7 and 66.4 CH₂-OOC CH₂OOCCH₂N⁺ CH₂CH₂N⁺, 126.9 C2/C6 aromatic, 128.7 C4 aromatic, 129 C3/C5 aromatic, 137 C1 aromatic, 164.6 C=O ester

### 2-Phenoxyethyl chloroacetate

Chloroacetyl chloride (0.1 mol, 8 ml) was mixed with dichloromethane (30 ml), in a 150 ml conical flask, cooled on a cold water-bath. To this solution were added dropwise a mixture of 2-phenoxyethanol (0.1 mol, 12.5 ml), pyridine (0.1 mol, 8 ml) in dichloromethane (20 ml), the total addition time being 30 minutes. The reaction mixture was left to stir over the cold water-bath for four hours. Then, the white precipitate of pyridinium chloride was filtered off and the reaction mixture was washed with distiled water (3x50 ml), dried over MgSO₄ and the solvent removed under reduced pressure, yielding 2-phenoxyethyl chloroacetate as a slightly yellow oil (19.85 g , 83.6%)
C₁₀H₁₁ClO₃ M= 237.6 g/mol
δH: (270 MHz, CDCl₃) 4.10 (CH₂OOCCH₂Cl, s, 2H), 4.18 (Ar-O-CH₂, t, J 4.5 Hz, 2H), 4.52 (CH₂-OOC, t, J 4.5Hz, 2H), 6.89 (H2/H6 aromatic, d, J 8.5 Hz, 2H), 6.97 (H4 aromatic, t, J 6 Hz, 1H), 7.29 (H3/H5 aromatic, m, 2H)

### 2-Phenoxyethyl Betainate (2-phenoxyethyloxycarbonyl-N,N,N-trimethylmethanaminium chloride)

Trimethyl amine (0.15 mol, 13.5 ml) was added to a solution, cooled over a salted ice bath, of 2-phenoxyethyl chloroacetate (0.084 mol, 19.85 g) in ether (75 ml). The reaction mixture was stirred for 5 hours at around 0^{o}C and then for an extra 48 hours at room temperature, during which the reaction mixture turns to a white solid which was isolated on a glass filter and washed carefully with ether (3x100 ml), then stirred for an hour in ether. Finally, the product was recrystallized twice from acetonitrile yielding a fine white solid (17.8g, 77.8%).
C₁₃H₂₀ClNO₃ M= 273.8 g/mol mp 160 °C
δH: (270 MHz, CDCl₃) 3.56 ((CH₃)₃N⁺, s, 9H), 4.20 (, t, J 4.5 Hz, 2H), 4.53 (CH₂-OOC, t, J 4.5Hz, 2H), 5.20 (CH₂OOCCH₂N⁺, s, 2H), 6.89 (H2/H6 aromatic, d, J 8.5 Hz, 2H), 6.97 (H4 aromatic, t, J 6 Hz, 1H), 7.29 (H3/H5 aromatic, m, 2H)
δC: (70 MHz, CDCl₃) 54.2 (CH₃)₃N⁺, 62.9 64.6 65.1 CH₂OOCCH₂N⁺ CH₂-OOC Ar-O-CH₂, 114.6 C2/C6 aromatic, 121.5 C4 aromatic, 129.7 C3/C5 aromatic, 158.1 C1 aromatic, 164.9 C=O ester
IS/MS (C₁₃H₂₀NO₃)⁺ M= 238.3 g/mol Found m/z= 238

| | | | |
|---|---|---|---|
| Anal Calcd for C₁₃H₂₀ClNO₄ | C 57.04 | H 7.36 | N 5.12 |
| C₁₃H₂₀ClNO₄.H₂O | C 53.51 | H 7.60 | N 4.80 |
| Found: | C 55.59 | H 7.42 | N 4.81 |

### Farnesyl chloroacetate

Chloroacetyl chloride (0.1 mol, 8 ml) was mixed with dichloromethane (75 ml), in a 150 ml conical flask, cooled on a cold water-bath. To this solution was added dropwise a mixture of farnesol (67.5 mmol, 15g), pyridine (67.5 mmol, 5.45 ml) in dichloromethane (25 ml), the total addition time being 30 minutes. The reaction mixture was left to stir over the cold water-bath for four hours. Then, the white precipitate of pyridinium chloride was filtered off and the reaction mixture was washed with distiled water (3x100 ml), dried over MgSO₄ and the solvent removed under reduced pressure, yielding farnesyl chloroacetate as a slightly yellow oil (20.1 g, quantitative)
C₁₇H₂₇ClO₂ M= 298.85 g/mol
δH: (270 MHz, CDCl₃) 1.59 1.68 1.72 (CH₃-C=, 12H), 1.85-2.20 (=C-CH₂-CH₂, m, 8H), 4.05 (CH₂OOCCH₂Cl, s, 2H), 4.70 (=CH-CH₂-OOC, d, J 7Hz, 2H), 5.08 (=CH, m, 2H), 5.35 (=CH-CH₂OOC, t, J 6 Hz, 1H)
δC: (70 MHz, CDCl₃) 15.9 16.4 17.6 23.3 25.6 CH₃, 26.7 31.9 32.1 39.6 CH₂, 40.9 CH₂OOCCH₂Cl, 62.9 CH₂-OOC, 117.3 123.4 124.2 131.2 135.5 143.5 C=C, 167.2 C=O ester

### Farnesyloxycarbonyl-N,N-dimethyl-N-propanolmethanaminium chloride

Farnesylchloroacetate (67.5 mmol, 20.1g) was mixed with N,N-dimethylpropanolamine(67.5 mol, 7.9 ml), in chloroform (100 ml). The reaction mixture is stirred at room temperature for 48 hours. The solvent is rotaevaporated and the yellow gum obtained is then disolved in buffered acidic water (pH=3, 75 ml) and washed with petroleum ether 40-60^{o}C (3*75 ml). The aqueous phase is then saturated with sodium chloride and extracted with chloroform (3*75 ml). The organic phases are combined, dried over MgSO₄, filtered and the solvent is removed under reduced pressure, yielding farnesyloxycarbonyl-N,N-dimethyl-N-propanolmethanaminium chloride as a brown gum (11.5 g , 42.3%)
C₂₂H₄₀ClNO₃ M= 402.02 g/mol
δH: (270 MHz, CDCl₃) 1.59 1.68 1.72 (CH₃-C=, 12H), 1.85-2.20 (=C-CH₂-CH₂, m, 10H), 3.48 ((CH₃)₃N⁺, s, 6H), 3.66 (CH₂CH₂N⁺, m, 2H), 3.91 (CH₂OH , m, 2H), 4.56 (CH₂OOCCH₂N⁺, s, 2H), 4.65 (=CH-CH₂-OOC, d, J 7Hz, 2H), 5.08 (=CH, m, 2H), 5.26 (=CH-CH₂OOC, t, J 6 Hz, 1H)
δC: (70 MHz, CDCl₃) 15.9 16.4 17.6 23.3 25.6 CH₃, 25.9 26.7 31.9 32.1 39.6 CH₂, 51.5 (CH₃)₂N⁺, 58.1 CH₂OH, 61.1 63.1 and 63.6 CH₂-OOC CH₂OOCCH₂N⁺ CH₂CH₂N⁺, 117.3 123.4 124.2 131.2 135.5 143.5 C=C, 167.2 C=O ester

### Cis-3-hexenyl chloroacetate

Chloroacetyl chloride (0.1 mol, 8 ml) was mixed with dichloromethane (30 ml), in a 150 ml conical flask, cooled on a cold water-bath. To this solution were added dropwise a mixture of cis-3-hexenol (0.1 mol, 11.8 ml), pyridine (0.1 mol, 8 ml) in dichloromethane (20 ml), the total addition time being 15 minutes. The reaction mixture was stirred over the cold water-bath for one hour, then at room temperature for 24 hours and finally at reflux for 6 hours. Then, the white precipitate of pyridinium chloride was filtered off and the reaction mixture was washed with distiled water (3x50 ml), dried over MgSO₄ and the solvent removed under reduced pressure, yielding cis-3-hexenyl chloroacetate as a yellow liquid (15.1 g , 85.5%)
C₈H₁₃ClO₂ M= 176.6 g/mol
IR: n C=O 1738 cm⁻¹, n C=C 1611 cm⁻¹, n O=C-O-C 1282 cm⁻¹, n CO-O-C 1169 cm⁻¹, n C-Cl 756 cm⁻¹,
δH: (270 MHz, CDCl₃) 0.98 (CH₃, t, J 8 Hz, 3H), 2.07 (CH₃CH₂CH=, quartet, J 7 Hz, 2H), 2.43 (=CHCH₂CH₂OOC, quintet, J 7 Hz, 2H), 4.06 (CH₂OOCCH₂Cl, s, 2H), 4.20 (CH₂-OOC, t, J 7Hz, 2H), 5.30 (=CH, m, 1H), 5.52 (=CH, m, 1H)

### Cis-3-hexenyl Betainate (cis-3-hexenyloxycarbonyl-N,N,N-trimethylmethanaminium chloride)

Trimethyl amine (0.12 mol, 11 ml) was added to a solution, cooled over a salted ice bath, of cis-3-hexenyl chloroacetate (0.0855 mol, 15.1 g) in ether (100 ml). The reaction mixture was stirred for 6 hours at around 0^{o}C and then for an extra 48 hours at room temperature, during which a grey solid precipitated out of solution and was isolated on a glass filter. It was then washed carefully with ether (3x100 ml), then stirred for an hour in ether (100 ml) and dried under vacuum, yielding a lightly grey solid (9.45g, 46.9 %).
C₁₁H₂₂ClNO₂ M= 235.75 g/mol
δH: (270 MHz, CDCl₃) 0.98 (CH₃, t, J 8 Hz, 3H), 2.07 (CH₃CH₂CH=, quartet, J 7 Hz, 2H), 2.43 (=CHCH₂CH₂OOC, quintet, J 7 Hz, 2H), 3.56 ((CH₃)₃N⁺, s, 9H), 4.18 (CH₂-OOC, t, J 7Hz, 2H), 5.09 (CH₂OOCCH₂N⁺, s, 2H), 5.30 (=CH, m, 1H), 5.52 (=CH, m, 1H)
δC: (70 MHz, CDCl₃) 14.2 CH₃, 20.6 C=C-CH₂CH₂-OOC, 26.3 CH₃CH₂-C=C, 54.2 (CH₃)₃N⁺, 63 CH₂-OOC-, 66 CH₂OOCCH₂N⁺, 122.4 and 135.5 two CH=, 164.9 C=O ester
   IS/MS (C₁₁H₂₂NO₂)⁺ M= 200.3 g/mol Found m/z= 200

### 3-Methyl-5-phenyl-1-pentanyl chloroacetate (Phenoxanyl chloroacetate)

Chloroacetyl chloride (0.1 mol, 8 ml) was mixed with dichloromethane (80 ml), in a 150 ml conical flask, cooled on a cold water-bath. To this solution were added dropwise a mixture of 3-methyl-5-phenyl-1-pentanol (0.1 mol, 18.6 ml), pyridine (0.1 mol, 8 ml) in dichloromethane (20 ml), the total addition time being 15 minutes. The reaction mixture was left to stir over the cold water-bath for four hours. Then, the white precipitate of pyridinium chloride was filtered off and the reaction mixture was washed with distiled water (3x100 ml), dried over MgSO₄ and the solvent removed under reduced pressure, yielding 3-methyl-5-phenyl-1-pentanyl chloroacetate as a colourless liquid (24.1 g , 94.6%)
C₁₄H₁₉ClO₂ M= 254.76 g/mol
IR: n C=O 1761 cm⁻¹, n C=C aromatic 1604 cm⁻¹, n O=C-O-C 1295 cm⁻¹, n CO-O-C 1181 cm⁻¹, n C-Cl 747 cm⁻¹,
δH: (270 MHz, CDCl₃) 0.99 (CH₃, d, J 6 Hz, 3H), 1.48-1.85 ( two CH₂ and one CH, 5 H), 2.63 (Ar-CH₂, m, 2H), 4.04 (CH₂OOCCH₂Cl, s, 2H), 4.25 (CH₂-OOC, t, J 8Hz, 2H), 7.15-7.35 (H aromatic, m, 5H)
δC: (70 MHz, CDCl₃) 19.8 CH₃, 29.7 33 .2 35.5 two CH₂ aliphatic and the CH, 38.5 Ar-CH₂, 41.5 CH₂OOCCH₂Cl, 64.4 CH₂-OOC, 125.6 C4 aromatic, 128.3 4 aromatic carbons C2/C3/C5/C6, 142.5 C1 aromatic, 167.5 C=O ester

### 3-Methyl-5-phenyl-1-pentanyl Betainate (3-methyl-5-phenyl-1-pentanyloxycarbonyl-N,N,N-trimethylmethanaminium chloride)

Trimethyl amine (0.15 mol, 13.5 ml) was added to a solution, cooled over a salted ice bath, of 3-methyl-5-phenyl-1-pentanyl chloroacetate (0.0946 mol, 24.1 g) in ether (75 ml). The reaction mixture was stirred for 6 hours at around 0^{o}C and then for an extra 12 hours at room temperature, yielding a white precipitate which was isolated on a glass filter and washed carefully with ether (3x100 ml). The product was recrystallized from acetonitrile yielding a fine white solid (15.9 g, 53.5 %).
C₁₇H₂₈ClNO₂ M= 313.9 g/mol mp 134 °C
δH: (270 MHz, CDCl₃) 0.98 (CH₃, d, J 6 Hz, 3H), 1.48-1.85 ( two CH₂ and one CH, 5 H), 2.63 (Ar-CH₂, m, 2H), 3.64 ((CH₃)₃N⁺, s, 9H), 4.22 (CH₂-OOC, t, J 8Hz, 2H), 5.02 (CH₂OOCCH₂N⁺, s, 2H), 7.15-7.35 (H aromatic, m, 5H)
IS/MS (C₁₇H₂₈NO₂)⁺ M= 278.4 g/mol Found m/z= 278

| | | | |
|---|---|---|---|
| Anal Calcd for C₁₇H₂₈ClNO₂ | C 65.06 | H 8.99 | N 4.46 |
| C₁₇H₂₈ClNO₂.H₂O | C 61.52 | H 9.11 | N 4.22 |
| Found: | C 64.95 | H 9.03 | N 4.29 |

### 2,4-Dimethyl-3-cyclohexene-1-methanyl chloroacetate (Floralyl chloroacetate)

Chloroacetyl chloride (0.1 mol, 8 ml) was mixed with dichloromethane (80 ml), in a 150 ml conical flask, cooled on a cold water-bath. To this solution were added dropwise a mixture of 2,4-dimethyl-3-cyclohexene-1-methanol (0.1 mol, 14.9 ml), pyridine (0.1 mol, 8 ml) in dichloromethane (20 ml), the total addition time being 15 minutes. The reaction mixture was left to stir over the cold water-bath for four hours. Then, the white precipitate of pyridinium chloride was filtered off and the reaction mixture was washed with distiled water (3x100 ml), dried over MgSO₄ and the solvent removed under reduced pressure, yielding 3-methyl-5-phenyl-1-pentanyl chloroacetate as a yellow oil (20.5 g , 94.6%)
C₁₁H₁₇ClO₂ M= 216.71 g/mol
IR: n C=O 1759 cm⁻¹, n C=C 1677 cm⁻¹, n O=C-O-C 1290 cm⁻¹, n CO-O-C 1177 cm⁻¹, n C-Cl 791 cm⁻¹,

The commercial 2,4-dimethyl-3-cyclohexene-1-methanol (floralol) used is a mixture of two stereoisomers, noted A and B in a proportion of 50-75% A and 25-50% B. An 1H nmr analysis of the starting floralol showed that this particular sample had a 60%/40% A/B composition.
δH: (270 MHz, CDCl₃) 0.86 (CH₃ _{B}, d, J 7 Hz, 3HB), 1.01 (CH₃ _{A}, d, J 7 Hz, 3HA),1.40-1.60 ( CH₂ CHCH₂O, m, 2 HA + 2 HB), 1.64 (CH₃ C=, s, 3 HA + 3 HB), 1.80-2.40 ( CH₂ C= and two CH, m, 4 HA + 4 HB), 4.07 and 4.08 (CH₂OOCCH₂Cl, two s, 2 HA + 2 HB), 4.12 (CH₂-OOC, d, J 8Hz, 2HA), 4.24 (CH₂-OOC, d, J 8Hz, 2HB), 5.17 (=CH, m, 1HA), 5.31 (=CH, m, 1HB)

### 2,4-Dimethyl-3-cyclohexene-1-methanyl Betainate (2,4-Dimethyl-3-cyclohexene-1-methanyloxycarbonyl-N,N,N-trimethylmethanaminium chloride)

Trimethyl amine (0.3 mol, 27.5 ml) was added to a solution, cooled over a salted ice bath, of 2,4-dimethyl-3-cyclohexene-1-methanyl chloroacetate (0.0946 mol, 20.5g) in ether (75 ml). The reaction mixture was stirred for 6 hours at around 0^{o}C and then for an extra 48 hours at room temperature, yielding a white precipitate which was isolated on a glass filter and washed carefully with ether (3x100 ml). The product was recrystallized from acetonitrile yielding a fine white solid (9.05 g, 34.7 %).
C₁₄H₂₆ClNO₂ M= 254.76 g/mol M= 275.8 g/mol
mp 133-145°C
δH: (270 MHz, CDCl₃) 0.86 (CH₃ _{B}, d, J 7 Hz, 3HB), 1.01 (CH₃ _{A}, d, J 7 Hz, 3HA),1.40-1.60 ( CH₂ CHCH₂O, m, 2 HA + 2 HB), 1.64 (CH₃ C=, s, 3 HA + 3 HB), 1.80-2.40 ( CH₂ C= and two CH, m, 4 HA + 4 HB), 3.69 ((CH₃)₃N⁺, s, 9 HA + 9 HB), 4.12 (CH₂-OOC, m, 2HA), 4.27 (CH₂-OOC,m, 2HB), 5.10 (CH₂OOCCH₂N⁺, s, 2 HA + 2 HB), 5.16 (=CH, m, 1HA), 5.30 (=CH, m, 1HB)
IS/MS (C₁₄H₂₆NO₂)⁺ M= 240.4 g/mol Found m/z= 240

| | | | |
|---|---|---|---|
| Anal Calcd for C₁₄H₂₆ClNO₂ | C 60.97 | H 9.50 | N 5.08 |
| C₁₄H₂₆ClNO₂.H₂O | C 57.23 | H 9.60 | N 4.77 |
| Found: | C 59.44 | H 9.46 | N 5.03 |

### 2,4-dichlorobenzyle Chloroacetate

Same experimental as for geranyl chloroacetate. Colourless oil (21.5 g, quantitative).
C₉H₅Cl₃O₂ M= 251.5 g/mol
IR: n C=O 1763 cm⁻¹ , n C=C 1593 cm-1
δH: (270 MHz, CDCl₃) 4.02 (ArCH₂OOCCH₂Cl, s, 2H), 5.40 (ArCH₂OOC, s, 2H), 7.20-7.40 (H benzyl, m, 3H)
δC: (70 MHz, CDCl₃) 40.5 ArCH₂-Cl, 64.1 ClCH₂COOCH₂Ar, 127.1 129.2 130.7 131.9 134.2 and 134.8 aromatic carbons, 166.7 ClCH₂COOCH₂Ar

### (-) Menthyl chloroacetate

Chloroacetyl chloride (0.064 mol, 5.1 ml) was mixed with dichloromethane (80 ml), in a 150 ml conical flask, cooled on a cold water-bath. To this solution were added dropwise a mixture of menthol (0.064 mol, 10g), pyridine (0.064 mol, 5.1ml) in dichloromethane (20 ml), the total addition time being 15 minutes. The reaction mixture was left to stir over the cold water-bath for six hours. Then, the white precipitate of pyridinium chloride was filtered off and the reaction mixture was washed with 5% w/w sodium hydrogen carbonate (100 ml), then distiled water (2x100 ml), dried over MgSO₄ and the solvent removed under reduced pressure, yielding menthyl chloroacetate as a dark yellow oil (13.85 g, 93%)
C₁₂H₂₁ClO₂ M= 232.75 g/mol
IR: n C=O 1759 and 1740 cm⁻¹
δH: (270 MHz, CDCl₃) 0.76 (CH₃, d, J 6.8 Hz, 3H), 0.91 ((CH₃)₂-CH, m, 6H), 1.03 ( CH₂, m, 2H), 1.40-150 ( CH CH₂, m, 3H), 1.68 ( CH₂, m, 2H), 1.86 ( CH, m, 1H), 2.02 ( CH, m, 1H), 4.04 (CHOOCCH₂Cl, s, 2H), 4.77 (CH-OOC, t d, J₁ 11 Hz, J₂ 4.6 Hz, 1H)
δC/DEPT: (70 MHz, CDCl₃) 16.2 20.6 21.9 CH₃, 23.3 34.0 CH₂, 26.2 31.3 46.9 CH, 40.5 41.1 CH₂-CHOOC CHOOCCH₂Cl, 76.4 CH-OOC, 166.8 C=O ester

### (-)Menthyl Betainate (menthyloxycarbonyl-N,N,N-trimethylmethanaminium chloride)

Trimethyl amine (0.27 mol, 25 ml) was added to a solution, cooled over a salted ice bath, of the menthyl chloroacetate (0.064 mol, 15.1 g) in toluene (80 ml). The reaction mixture was stirred for 6 hours at around 0^{o}C and then for an extra 12 hours at room temperature, yielding a white precipitate which was isolated on a glass filter, washed carefully with ether (3x100 ml) and dried under vacuum (16 g, 85.2%).
C₁₅H₃₀ClNO₂ M= 292.9 g/mol mp 212-217^{O}C
δH: (90 MHz, CDCl₃) 0.76 (CH₃, d, J 6.8 Hz, 3H), 0.91 ((CH₃)₂-CH, m, 6H), 1.03 ( CH₂, m, 2H), 1.40-150 ( CH CH₂, m, 3H), 1.68 ( CH₂, m, 2H), 1.86 ( CH, m, 1H), 2.02 ( CH, m, 1H), 4.85 (CHOOCCH₂N⁺, s, 2H), 5.27 (CH-OOC, t d, J₁ 11 Hz, J₂ 4.6 Hz, 1H)

| | | | |
|---|---|---|---|
| Anal Calcd for C₁₅H₃₀ClNO₂: | C 61.73 | H 10.36 | N 4.80 |
| C₁₅H₃₀ClNO₂.H₂O | C 58.14 | H 10.41 | N 4.52 |
| Found: | C 58.16 | H 10.34 | N 4.52 |

### (-)Menthyl pyridinoacetate

Menthyl chloroacetate (7.1 g, 0.03 mol) was stirred in anhydrous pyridine (12 ml, 0.15 mol) at 50^{O}C for 5 hours. Solid began to appear after 1.5 hours. The mixture was cooled to a solid mass which was filtered with ether and washed with the same solvent. Drying gave a white product (8.1 g, 86.3%).
C₁₇H₂₆ClNO₂ M= 311.9 g/mol mp 215-218^{O}C
δH: (270 MHz, CDCl₃) 0.76 (CH₃, d, J 6.8 Hz, 3H), 0.91 ((CH₃)₂-CH, m, 6H), 1.03 ( CH₂, m, 2H), 1.40-150 ( CH CH₂, m, 3H), 1.68 ( CH₂, m, 2H), 1.86 ( CH, m, 1H), 2.02 ( CH, m, 1H), 5.27 (CH-OOC, t d, J₁ 11 Hz, J₂ 4.6 Hz, 1H), 6.31 (CHOOCCH₂N⁺, s, 2H), 8.14 (d d, J1 6.5 Hz, J2 7.5 Hz, 2H pyridine), 8.61 (t, J 8 Hz, 1H pyridine), 9.48 (d, J 5.5 Hz, 2H pyridine)

| | | | |
|---|---|---|---|
| Anal Calcd for C₁₇H₂₆ClNO₂: | C 65.49 | H 8.35 | N 4.49 |
| Found: | C 65.28 | H 8.34 | N 4.49 |

### (-)Menthyloxycarbonyl-N-butyl-N,N-dimethylmethanaminium chloride

Menthylchloroacetate (0.0595 mol, 13.85g) was mixed with N,N-dimethylbutylamine (0.13 mol, 18 ml), in chloroform (100 ml). The reaction mixture turns instantly dark brown and is stirred at room temperature for 48 hours. Then, all the chloroform except 20 ml is rotaevaporated and the remaining chloroformic solution is added dropwise over 30 minutes to petroleum ether 40-60^{o}C (300 ml), with a vigorous stirring. In contact with the petroleum ether, a white precipitate forms and is filtered off at the end of addition on a sinter glass 4. The fine white solid recovered is stirred in petroleum ether 40-60^{o}C (100 ml) overnight, filtered off and dried under vacuum yielding menthyloxycarbonyl-N-butyl-N,N-dimethylmethanaminium chloride as a white solid (17.7 g, 89.1%).
C₁₈H₃₆ClNO₂ M= 333.95 g/mol mp 204 °C
δH: (270 MHz, CDCl₃) 0.76 (CH₃, d, J 6.8 Hz, 3H), 0.80-1.1 ((CH₃)₂-CH CH₃CH CH₂, m, 11H), 1.40-150 1.68 2.02 ( CH₂ CH, m, 11H), 3.68 and 3.70 (CH3-N⁺, s, 6H), 3.83 (CH₂CH₂N⁺, t, J 7.5 Hz, 2H), 4.77 (CH-OOC, t d, J₁ 11 Hz, J₂ 4.6 Hz, 1H), 4.57-4.98 (CHOOCCH₂N⁺, quartet, 2H),
δC/DEPT: (70 MHz, CDCl₃) 13.6 CH₃CH₂, 16.0 20.7 21.8 other CH₃, 19.5 23.0 24.8 33.8 CH₂, 26.1 31.4 CH, 40.5 CH₂-CHOOC, 46.6 CH-CHOOC, 52.1 (CH₃)₂-N⁺, 60.8 64.1 CH₂CH₂N⁺ CHOOCCH₂N⁺, 76.5 CH-OOC, 164.3 C=O ester

### Eugenyl chloroacetate

Chloroacetyl chloride (0.0914 mol, 7.3 ml) was mixed with dichloromethane (75 ml), in a 250 ml conical flask, cooled on a cold water-bath. To this solution was added dropwise a mixture of eugenol (0.0914 mol, 15.0 g), pyridine (0.0914 mol, 7.4 ml) in dichloromethane (75 ml), the total addition time being 30 minutes. The reaction mixture was left to stir over the cold water-bath for four hours during which it has taken a light-yellow colour. Then, the white precipitate of pyridinium chloride was filtered off and the reaction mixture was washed with distiled water (3x100 ml), dried over MgSO₄ and the solvent removed under reduced pressure, yielding eugenyl chloroacetate as a light yellow liquid (20.83 g, 93.4%)
C₁₂H₁₄ClO₃ M= 241.7 g/mol
δH: (270 MHz, CDCl₃) 3.33 (ArCH₂HC=, d, J 6.5Hz, 2H), 3.75 (ArOCH₃, s, 3H), 4.28 (ArOOCCH₂Cl, s, 2H), 5.11 (=CH₂, m, 2H), 5.94 (CH₂CH=CH₂, m, 1H), 6.75 (C-H aromatic, m, 2H), 6.94 (C-H aromatic, d, 8.3 Hz, 1H)
δC: (70 MHz, CDCl₃) 40.3 41.0 ArOOCCH₂Cl and ArCH₂HC=, 56.0 ArOCH₃, 113.1 120.9 122.4 C-H aromatic , 116.5 CH₂CH=CH₂, 137.2 CH₂CH=CH₂, 137.8 CCH₂CH=, 139.9 COCH₃, 150.9 C-OC=O aromatic, 165.9 C=O ester

### Vanilin chloroacetate

Chloroacetyl chloride (0.999 mol, 7.86 ml) was mixed with dichloromethane (75 ml), in a 250 ml conical flask, cooled on a cold water-bath. To this solution was added dropwise a mixture of vanilin (0.099 mol, 15.0 g), pyridine (0.099 mol, 8.0 ml) in dichloromethane (50 ml), the total addition time being 30 minutes. The reaction mixture was left to stir over the cold water-bath for four hours during which it has taken a light-yellow colour. Then, the reaction mixture was washed with distiled water (3x100 ml), dried over MgSO₄ and the solvent removed under reduced pressure, yielding vanilinchloroacetate as a yellow viscous oil (21.2 g, 93.7%)
C₁₀H₉ClO₄ M= 228.63 g/mol mp 55°C
δH: (270 MHz, CDCl₃) 3.90 (ArOCH₃, s, 3H), 4.37 (ArOOCCH₂Cl, s, 2H), 7.26 (C-H aromatic, d, 8 Hz, 1H), 7.45-7.50 (C-H aromatic, m,2H), 9.95 (CO-H, s, 1H)
δC: (70 MHz, CDCl₃) 40.4 ArOOCCH₂Cl, 56.1 ArOCH₃, 111.0 123.0 124.6 C-H aromatic, 135.5 COCH₃, 144.2 C-COH, 151.6 C-OC=O , 164.8 C=O ester, 190.9 C=O aldehyde

### 2,4-dichlorophenyl chloroacetate

Chloroacetyl chloride (0.08 mol, 6.4 ml) was mixed with sodium dried toluene (30 ml), in a 150 ml conical flask, cooled on a cold water-bath. To this red solution were added dropwise a mixture of 2,4-dichlorophenol (0.08 mol, 13.06g), pyridine (0.08 mol, 6.4 ml) in sodium dried toluene (40 ml), the total addition time being 30 minutes. The reaction mixture was left to stir over the cold water-bath for four hours during which it has taken a dark-yellow colour. Then, the white precipitate of pyridinium chloride was filtered off and the reaction mixture was washed with distiled water (3x100 ml), dried over MgSO₄ and the solvent removed under reduced pressure, yielding 2,4-dichlorophenyl chloroacetate as sharp yellow needles.
C₈H₅Cl₃O₂ M= 239.49 g/mol
IR: n C=O 1790 cm⁻¹, n C=C aromatic 1585 cm⁻¹, n O=C-O-C 1218 cm⁻¹, n CO-O-C 1126 cm⁻¹, n C-Cl 747 and 803 cm⁻¹,
δH: (270 MHz, CDCl₃) 4.32 (ArOOCCH₂Cl, s, 2H), 7.02 (C6-H aromatic, 1H), 7.14 (C5-H aromatic, 1H), 7.36 (C3-H aromatic, 1H)
δC: (70 MHz, CDCl₃) 39.8 ArOOCCH₂Cl, 123.6 127.6 129.8 C-H aromatic (C3/C5/C6), 127.1 and 132 C-Cl aromatic (C2/C4), 144.5 C-O aromatic (C1), 164.6 C=O ester

### 2,4-Dichlorophenyl Betainate (2,4-dichlorophenyloxycarbonyl -N,N,N-trimethylmethanaminium chloride)

Trimethyl amine (0.16 mol, 15 ml) was added to a solution, cooled over a salted ice bath, of the 2,4-dichlorophenyl chloroacetate previously obtained, in ether (100 ml). The reaction mixture was stirred for 6 hours at around 0^{o}C and then for an extra 12 hours at room temperature, yielding a white precipitate which was isolated on a glass filter and washed carefully with ether (100 ml). The mother liquor was then cooled overnight at 4oC. Then, more white solid was isolated on a glass filter. Both solid fractions were combined and washed carefully with ether (3x100 ml), then dried under vaccum, yielding 2,4-dichlorophenyl betainate as a fine white dust (20.8g, 87% for the two steps)
C₁₁H₁₄Cl₃NO₂ M= 298.6 g/mol mp 124-126°C
δH: (270 MHz, DMSO-d6) 3.42 ((CH₃)₃N⁺, s, 9H), 5.17 (ArOOCCH₂N⁺, s, 2H), 7.58 (C6-H aromatic, 1H), 7.65 (C5-H aromatic, 1H), 7.85 (C3-H aromatic, 1H)
δC: (70 MHz, DMSO-d6) 53.4 (CH₃)₃N⁺, 62.2 ArOOCCH₂N⁺, 125.6 129 129.8 C-H aromatic (C3/C5/C6), 127 and 132 C-Cl aromatic (C2/C4), 144.5 C-O aromatic (C1), 163.3 C=O ester

### 4-Chloro-3,5-dimethylphenyl chloroacetate

Chloroacetyl chloride (0.1 mol, 8ml) was mixed with ether (30 ml), in a 250 ml conical flask, cooled on a cold water-bath. To this solution was added dropwise a mixture of 4-chloro-3,5-dimethylphenol (0.1 mol, 15.66g), pyridine (0.1 mol, 8 ml) in ether (100 ml), the total addition time being 30 minutes. The reaction mixture was left to stir over the cold water-bath for three hours. Then, the white precipitate of pyridinium chloride was filtered off and the reaction mixture was washed with distiled water (3x100 ml), dried over MgSO₄ and the solvent removed under reduced pressure, yielding 4-Chloro-3,5-dimethylphenyl chloroacetate as a grey solid (29.3g, 89.8%).
C₁₀H₁₀Cl₂O₂ M= 233.09 g/mol PF: 42°C
δH: (270 MHz, CDCl₃) 2.37 (CH₃, s, 6H), 4.27 (ArOOCCH₂Cl, s, 2H), 6.92 (H aromatic, s, 2H).
δC: (70 MHz, CDCl₃) 21.2 Ar-CH₃ (two C), 41.2 ArOOCCH₂Cl, 121.2 C-H aromatic (C2/C6), 132.7 C-Cl aromatic (C4), 138.2 C-CH₃ aromatic (C3/C5), 148.3 C-O aromatic (C1), 166.4 C=O ester

### 4-Chloro-3,5-dimethylphenyl Betainate (4-chloro-3,5-dimethylohenyloxycarbonyl-N N,N-trimethylmethanaminium chloride)

Trimethyl amine (0.2 mol, 18 ml) was added to a solution, cooled over a salted ice bath, of the 4-chloro-3,5-dimethylphenyl chloroacetate (mol, g) in chloroform (200 ml). The reaction mixture was stirred for 6 hours at around 0^{o}C and then for an extra 12 hours at room temperature, yielding a white precipitate which was isolated on a glass filter, washed carefully with ether (3x100 ml) and recrystallized from acetonitrile, yielding 4-chloro-3,5-dimethylphenyl betainate as a fine white dust (18.1g, %)
C₁₃H₁₉Cl₂NO₂ M= 292.2 g/mol mp 169 °C
δH: (270 MHz, CD₃OD) 2.38 (CH₃, s, 6H), 3.41 ((CH₃)₃N⁺, s, 9H), 4.92 (ArOOCCH₂N⁺, s, 2H), 7.03 (H aromatic, s, 2H).
δC: (70 MHz, CD₃OD) 21 Ar-CH₃ (two C), 56 (CH₃)₃N⁺, 64.5 ArOOCCH₂N⁺, 121.5 C-H aromatic (C2/C6), 133.5 C-Cl aromatic (C4), 139 C-CH₃ aromatic (C3/C5), 148 C-O aromatic (C1), 166 C=O ester

### Triclosan chloroacetate

Triclosan : 2,4,4'-trichloro-2'-hydroxydiphenyl ether
Chloroacetyl chloride (0.1 mol, 8ml) was mixed with dichloromethane (50 ml), in a 250 ml conical flask, cooled on a cold water-bath. To this solution was added dropwise a mixture of 2,4,4'-trichloro-2'-hydroxydiphenyl ether (0.1 mol, 29g), pyridine (0.1 mol, 8 ml) in dichloromethane (100 ml), the total addition time being 30 minutes. The reaction mixture was left to stir over the cold water-bath for five hours. Then, the white precipitate of pyridinium chloride was filtered off and the reaction mixture was washed with distiled water (3x200 ml), dried over MgSO₄ and the solvent removed under reduced pressure, yielding triclosan chloroacetate as a white solid which is subsequently dried under vacuum (36.2g, 98.9%)
C₁₄H₈Cl₄O₃ M= 366.03 g/mol
IR: n C=O 1788 cm⁻¹, n C=C aromatic 1592 and 1582 cm⁻¹, n O=C-O-C 1216 cm⁻¹, n CO-O-C 1123 cm⁻¹, n C-Cl 765 and 812 cm⁻¹,
δH: (270 MHz, CDCl₃) 4.26 (ArOOCCH₂Cl, s, 2H), 6.80 (H aromatic, d, J 8.5 Hz, 1H), 6.91 (H aromatic, d, J 8.5 Hz, 1H), 7.15-7.25 (H aromatic, m, 3H), 7.47 (H aromatic, d, J 2.5 Hz, 1H).
δC: (70 MHz, CDCl₃) 20.8 Ar-CH₃ (two C), 40.1 ArOOCCH₂Cl, 119.8 120.9 124 127.6 128.3 130.4 C-H aromatic (C2/C6), 126.5 129.2 and 130 C-Cl aromatic (C2/C4/C4'), 138.1 C-CH₃ aromatic (C3/C5), 140.9 146.7 and 150.6 C-O-Ar (two C) and C-OOC, 165 C=O ester

### Triclosan Betainate

Trimethyl amine (0.15 mol, 13.5 ml) was added to a solution, cooled over a salted ice bath, of the triclosan chloroacetate (0.0989 mol, 36.2 g) in ether (250 ml). The reaction mixture was stirred for 6 hours at around 0^{o}C and then for an extra 12 hours at room temperature, yieding a white precipitate which was isolated on a glass filter, washed carefully with ether (3x100 ml) and recristallized from acetonitirle, yielding triclosan betainate as a fine white dust (19.24g, 45.8 %)
C₁₇H₁₇Cl₄NO₃ M= 425.1 g/mol mp 167°C
δH: (270 MHz, CDCl₃) 3.69 ((CH₃)₃N⁺, s, 9H), 5.51 (ArOOCCH₂N⁺, s, 2H), 6.73 (H triclosan, d, J 8.5 Hz, 1H), 6.96 (H triclosan, d, J 8.5 Hz, 1H), 7.15-7.35 (H triclosan, m, 3H), 7.47 (H triclosan, d, J 2.5 Hz, 1H)
δC: (70 MHz, CDCl₃) 53.2 (CH₃)₂-N⁺, 62.3 ArOOCCH₂N⁺, 119.8 121.9 124.3 127.9 129 and 130.7 C-H aromatic (C2/C6), 125.5 128.3 and 129.2 C-Cl aromatic (C2/C4/C4'), 138.1 C-CH₃ aromatic (C3/C5), 139.9 146.5 and 150.0 C-O-Ar (two C) and C-OOC, 165 C=O ester

| | | | |
|---|---|---|---|
| Anal Calcd for C₁ ₇H₁₇Cl₄NO₃ | C 48.03 | H 4.03 | N 3.29 |
| C₁₇H₁₇Cl₄NO₃.H₂O | C 46.08 | H 4.32 | N 3.16 |
| Found: | C 47.89 | H 3.92 | N 3.31 |

### Linalyl Chloroacetate

### a) From chloroacetyl chloride

Chloroacetyl chloride (0.04 mol, 3.2 ml) was mixed with dry toluene (25 ml), in a 150 ml conical flask. To this solution was added dropwise a mixture of linalool (0.04 mol, 7.2 ml), pyridine (0.04 mol, 3.2 ml) in dry toluene (10 ml), the total addition time being about 15-30 minutes. The dropping funnel was fitted with a calcium chloride drying tube. The reaction mixture was left to stir at 50°C for 16 hours. Then, the white precipitate of pyridinium chloride was filtered off and the dark solution recovered was washed with a 5% solution of sodium hydrogen carbonate (60 ml), distilled water (2x60 ml), dried over MgSO₄ and the solvent removed under reduced pressure, yielding a dark brown oil which was analysed by 1H/13C NMR and GC/MS.

### b) From chloroacetic anhydride

Experiment as before except chloroacetic anhydride (6.83 g, 0.04 mol) is used instead of chloroacetyl chloride.The solvent used is dichloromethane rather than toluene.
Conditions of reaction: 2h30 at room temperature
Reaction yields a slightly brown oil

### Linalyl chloroacetate

C₁₂H₁₉ClO₂ M= 230.73 g/mol
IR: n C=O 1739 cm⁻¹, n C=C 1683 cm⁻¹ and 1643 cm⁻¹
δH: (270 MHz, CDCl₃) 1.57 1.58 and 1.67 (CH₃-COOC (CH₃)₂C=, trois s, 9H), 1.70-2.20 (=CH-CH₂, OOCC(CH₃)-CH₂, m, 4H), 4.00 (COOCCH₂Cl, s, 2H), 5.00-5.25 (CH₂= (CH₃)₂C=CH, m, 3H), 5.85-5.98 (CH₂=CH, m, 1H)
δC/DEPT/H-C COSY: (70 MHz, CDCl₃) 17.1 CH₃-C=, 21.9 =CH-CH₂, 23.0 CH₃-COOC, 25.2 CH₃-C=, 39.0 CH₂-C(CH₃)-OOC, 41.3 COOCCH₂Cl, 84.9 COOCCH₂Cl, 113.6 CH=CH₂, 123.2 CH =C(CH₃)₂, 131.5 CH =C(CH₃)₂, 140.3 CH=CH₂, 165.4 C=O

### H-C COSY:

Direct coupling of the singlets at 1.57 and 1.58 ppm (1H) with 17.1 and 23.0 ppm (13C).
Direct coupling of the singlet at 1.67 ppm (1H) with 25.2 ppm (13C).
Direct coupling of 1.70-2.05 ppm (1H) with 21.9 and 39.0 ppm (13C).
Direct coupling of the singlet at 4.00 ppm (1H) with 41.3 ppm (13C).
Direct coupling of 5.0-5.25 ppm (1H) with 113.6 and 123.2 ppm (13C).
Direct coupling of 5.89-5.98 ppm (1H) with 140.3 ppm (13C).

MS 136 (7%),121 (27%),93 (^{°}OOCCH₂Cl, 100%), 91 (28%), 80 (31%), 77 (32%), 69 (84%), 67 (26%)

### Linalyl Bromoacetate

Procedure as before except chloroacetyl chloride is replaced by bromoacetyl bromide
Conditions: 5 hours at 50°C in dry toluene.
Linalyl bromoacetate is recovered as a slightly brown oil.

C₁₂H₁₉BrO₂ M= 275.19 g/mol
δH: (270 MHz, CDCl₃) 1.55 1.59 and 1.66 (CH₃-COOC (CH₃)₂C=, m, 9H), 1.70-2.05 (=CH-CH₂, OOCC(CH₃)-CH₂, m, 4H), 3.73 (COOCCH₂Br, s, 2H), 5.00-5.25 (CH₂= and (CH₃)₂C=CH, m, 3H), 5.89-5.98 (CH₂=CH, m, 1H)
δC/DEPT/H-C COSY: (70 MHz, CDCl₃) 17.2 CH₃-C=, 21.9 =CH-CH₂, 23.0 CH₃-C=, 25.3 CH₃-COOC, 26.7 C(CH₃)OOCCH₂Br, 39.1 CH₂-C(CH₃)-OOC, 84.6 COOCCH₂Br, 113.5 CH=CH₂, 123.3 CH =C(CH₃)₂, 131.4 CH =C(CH₃)₂, 140.4 CH=CH₂, 165.2 C=O

### H-C COSY:

Direct coupling of the singlets at 1.55 1.59 and 1.66 ppm (1H) with 17.2 23.0 and 25.3 ppm (13C)
Direct coupling of 1.70-2.05 ppm (1H) with 21.9 and 39.1 ppm (13C)
Direct coupling of singlet at 3.73 ppm (1H) with 26.7 ppm (13C)
Direct coupling of 5.0-5.25 ppm (1H) with 113.5 and 123.3 ppm (13C)
Direct coupling of 5.89-5.98 ppm (1H) with 140.4 ppm (13C)

MS 195 (4%), 136 (^{°}OOCCH₂Br, 67%) with 137 (43%) and 138 (32%), 123 (74%), 121 (96%), 107 (28%), 95 (79%), 93 (100%), 91 (81%), 80 (81%), 77 (67%), 69 (76%), 67 (72%), 55 (64%), 53 (64%)

| Amine | Conditions | Solvent | Composition of the mixture (as assessed by GC/MS and 1H/13C NMR) |
|---|---|---|---|
| | | | |

| With chloroacetyl chloride | | | |
|---|---|---|---|
| 1 Eq pyridine | 4H at R.T. | Toluene | 45% linalyl chloroacetate |
| | | | 50% linalool |
| | | | 5%side-reactions products |
| | | | 75% linalyl chloroacetate |
| | 4H at R.T. + 16h at 50^{o}C | | 5-10% linalool |
| | | | 15 - 20% side-reactions products |
| | | | As before |
| | 40hat 40^{o}C | CH₂Cl₂ | |
| 1 Eq 4-DMAP | 6h at R.T. | CH₂Cl₂ | 20%linalyl chloroacetate |
| | | | 75-80%% linalool |
| | | | ∼5% sidE-reactions products |
| 1 Eq pyridine | 16 h at 60^{o}C | CHCl₃ | No ester formed, total isomerisation of linalool in geraniol |

| With chloroacetic anhydride | | | |
|---|---|---|---|
| 1 Eq pyridine | 2 h 30 at R.T. | CH₂Cl₂ | 90% linalyl chloroacetate |
| | | | 10% linalool( |
| | | | No side-reactions products detected |

| With bromoacetyl bromide | | | |
|---|---|---|---|
| 1 Eq pyridine | 5 h at 50°C | Toluene | 95% linalyl bromoacetate |
| | | | 5% linalool |
| | | | No side-reactions products detected |
| Eq: equivalent | | | |

### Application to other sterically hindered alcohols

| Esterifcation of the tertiary alcohol with bromoacetyl bromide (1.1 equivalent) | | | |
|---|---|---|---|
| Tertiary Alcohol and Amine used | Conditions | Solvent | Composition of the final reaction mixture (as assessed by GC/MS and 1H/13C NMR) |
| 1 Eq linalool + 1 Eq pyridine | 5 h at 50°C | Toluene | 95% linalyl bromoacetate |
| | | | 5% linalool |
| | | | No side-reactions products detected |
| 1 Eq tetrahydro linalool + 1 Eq pyridine | 5 h at 50°C | Toluene | >95% tetrahydrolinalyl bromoacetate |
| | | | <5% tetrahydrolinalool |
| | | | No side-reactions products detected |
| 1 Eq 1,2-dihydromyrcenol + 1 Eq pyridine | 7 h at 50°C | Toluene | 80-85% 1,2-dihydromyrcenyl bromoacetate |
| | | | 15-20% 1,2-dihydromyrcenol |
| | | | No side-reactions products detected |

### Tetrahydrolinalyle Bromoacetate

C₁₂H₂₃BrO₂ M= 279.22 g/mol
δH: (270 MHz, CDCl₃) 0.85-0.90 (CH₃, m, 9H), 1.13-1.33 (CH₂, m, 4H), 1.42 (CH₃-C-O-OCCH₂Br, s, 3H), 1.50-1.60 (CH, m, 1H), 1.68-1.96 (CH₂, m, 4H), 3.74 (COOCCH₂Br, s, 2H) δC/DEPT/H-C COSY: (70 MHz, CDCl₃) 7.8 22.4 CH₃, 22.9 CH₃-C-O-OCCH₂Br, 21.1 30.6 CH₂, 27.3 C(CH₃)OOCCH₂Br, 27.6 (CH₃)₂CH, 37.7 39.1 CH₂, 87.6 COOCCH₂Br, 165.9 C=O

### H-C COSY:

Direct coupling of the multiplet at 0.85-0.90 ppm (1H) with 7.7 et 22.4 ppm (13C)
Direct coupling of the multiplet at 1.13-1.33 ppm (1H) with 21.1 et 39.0 ppm (13C)
Direct coupling of the singulet at 1.42 ppm (1H) with 22.9 ppm (13C)
Direct coupling of the multiplet at 1.50-1.60 ppm (1H) with 27.6 ppm (13C)
Direct coupling of the multiplet at 1.68-1.96 ppm (1H) with 30.6 et 37.7 ppm (13C)
Direct coupling of the singulet at 3.74 ppm (1H) with 27.3 ppm (13C)

### 1,2-dihydromyrcenyl Bromoacetate

C₁₂H₂₁BrO₂ M= 277.2 g/mol
δH: (270 MHz, CDCl₃) 0.98 (CH₃-CH, d, 6.9 Hz, 3H), 1.20-1.35 (CH₂, m, 4H), 1.44 ((CH₃)₂C-OOC, s, 6H), 1.70-1.80 (CH₂, m, 2H), 2.1-2.2 (CH₃-CH, m, 1H), 3.72 (COOCCH₂Br, s, 2H), 4.88-4.98 (CH₂=, m, 2H), 5.61-5.71 (CH₂=CH, m, 1H)
δC/DEPT/H-C COSY:
   (70 MHz, CDCl₃) 20.2 CH₃-CH, 21.3 CH₂, 25.3 (CH₃)₂C-OOC, 27.5 C(CH₃)₂OOCCH₂Br, 36.6 40.5 CH₂, 37.5 CH₃-CH, 85.0 COOCCH₂Br, 112.6 CH=CH₂, 144.4 CH =CH₂, 166.1 C=O

### H-C COSY:

Direct coupling of the doublet at 0.98 ppm (1H) with 20.2 ppm (13C)
Direct coupling of the singulet at 1.44 ppm (1H) with 25.3 ppm (13C)
Direct coupling of the multiplet at 2.1-2.2 ppm (1H) with 37.5 ppm (13C)
Direct coupling of the singulet at 3.72 ppm (1H) with 27.5 ppm (13C)
Direct coupling of the multiplet at 4.88-4.98 ppm (1H) with 112.6 ppm (13C)
Direct coupling of the multiplet at 5.61-5.71 ppm (1H) with 144.4 ppm (13C)

### Second step

### Quaternisation of a tertiary amine with the halogenoacetate ester to prepare a betaine ester.

The reaction conditions for the quaternisation step with trimethylamine are various. Due to the great volatility of trimethylamine generally an excess of trimethylamine is used (usually 2 to 5 equivalents). The solvent can be either non-polar , ranging from petroleum ether/alkane to toluene and halogenated solvents such as chloroform or polar (alcohol, acetonitrile, acetone, dimethylformamide or dimethylsulfoxide). An aprotic polar solvent to a non-polar is prefered because the rate of quaternisation is normally faster in such a solvent but it is not primordial. The temperature of the reaction can be between -78°C and the refluxed temperature of the solvent, but preferentially the reaction is performed at room temperature.
When the reaction is performed at room temperature, the quaternisation is easily performed with various amines such as pyridine, dimethylbutylamine, dimethylpropanolamine. Tertiary ethanolamines can not generally be used because a transesterification takes place, yielding a salt of 2-oxomorpholinium instead of the expected betaine ester.

For more hindered or more hydrophobic tertiary amines (such as triethylamine, dimethylbenzylamine, dimethylalkylamine with an alkyl chain greater than 4 carbons), very specific conditions of reaction are needed to yield the expected betaine ester only.
In the prior art, quaternisation of such a tertiary amine with an halogenoacetate is only reported for the quaternisation methyl or ethyl chloroacetate with triethylamine or various dimethylalkylamines with an alkyl chain greater than 10 carbons.

R. P. Bell and F. J. Lindars (R. P. Bell and F. J. Lindars, J. Chem. Soc, 1954, 4601-4) have reported the synthesis of ethyloxycarbony-N,N,N-triethyl-methanaminium chloride by either stirring a stoichiometric amount of ethylchloroacetate and triethylamine, for seven hours in refluxing benzene or three weeks in ether at room temperature.

H. A. Al-Lohedan, C. A. Bunton and L. S. Romsted (A. Al-Lohedan, C. A. Bunton and L. S. Romsted, J. Phys. Chem., 1981, 85, 2123-9)have reported the synthesis of methyloxycarbony-N,N-dimethyl-N-dodecyl or hexadecylmethanaminium chloride by refluxing in methanol for 48 hours a stoichiometric mixture of methyl chloroacetate and dimethyldodecyl or hexadecylamine.

No special precautions or possible side-reactions are reported in these two references.

But, if similar conditions are applied for the quaternisation of various halogenoacetate esters of perfume alcohols such as geraniol or 2-phenoxyethanol, with tertiary amines such as triethylamine, dimethylbenzylamine or dimethylalkylamine with an alkyl chain greater than 4 carbons, some side reactions also occur and the expected betaine ester is only recovered in very poor yields.

It is found that the expected betaine ester is formed in solution but that if any unreacted tertiary amine is also present in solution, it will attack in solution the desired betaine ester and leads to a breakdown of the ester bond between the C and the O of the starting alcohol. The betaine can be considered to act as an alkylating agent. The products following this degradation are a betaine and a quaternary ammonium salt, formed by alkylation of the starting tertiary amine with the alkyl chain of the starting perfume alcohol.

### Mechanism of degradation

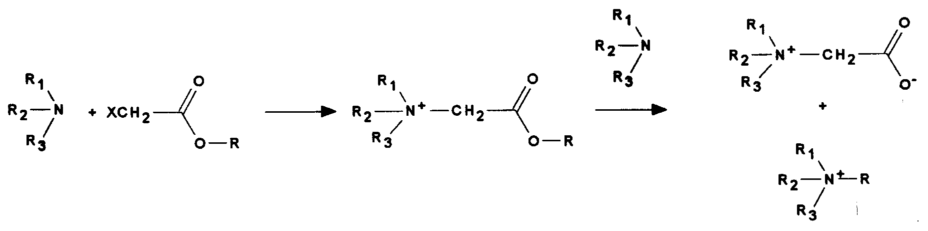

This degradation is favoured by a high temperature of reaction, a solvent of low polarity and a high concentration of tertiary amine in solution compared to the one of betaine ester and starting halogenoacetate ester.

The prefered conditions to minimize the occurence of this side-reaction is to run the experiment at a temperature as low as possible, preferably room temperature, in a polar solvent such as alcohol, acetonitrile, acetone, DMF or DMSO. The amount of tertiary amine and its mode of addition is also crucial to minimise the occurence of this side-reaction. The halogenoacetate ester must be used at least in equimolar or in excess of the tertiary amine (between 1 to 10 equivalents of halogenoacetate ester preferentially 1 to 2 equivalents per equivalent of tertiary amine).

Using these conditions, salts of geranyloxycarbonyl-N,N,N-triethylmethanaminium, and of various geranyloxycarbonyl-N,N-dimethyl-N-alkylmethanaminium have been easily prepared in very high yields. The degradation products are not detected in the various geranyloxycarbonyl-N,N-dimethyl-N-alkylmethanaminium and are present at a level below 5% for geranyloxycarbonyl-N,N,N-triethylmethanaminium.

### Geranyloxycarbonyl-N,N-dimethyl-N-benzyl-methanaminium bromide

Geranylbromoacetate (0.073 mol, 20g) is mixed with N,N-dimethylbenzylamine (0.146 mol, 21.8 ml), in acetone (150 ml). The reaction mixture is stirred at room temperature for 4 hours. Then, after removal of the solvent, the brown oil is carefully washed with petroleum spirit 40-60°C (150 ml) and diethyl ether (150 ml). The oil is then dissolved in buffered acidic water (pH=3) and extracted with chloroform (3*150 ml). The chloroform phases are combined, dried over MgSO₄ and concentrated under vacuum
C₂₁H₃₂BrNO₂ M= 410.4 g/mol
δH: (270 MHz, CDCl₃) 1.61 (CH₃-C=, s, 3H), 1.69 and 1.72 (CH₃-C=, s, 6H), 2.07 (=CH-CH₂, =C(CH₃)-CH₂, m, 4H), 3.57 (CH3-N⁺, s, 6H), 4.69 (=CH-CH₂-OOC, d, J 7.5 Hz, 2H), 4.78 (CH₂OOCCH₂N⁺, s, 2H), 5.08 (=CH, t, J 5.5 Hz, 1H), 5.29 (=CH-CH₂OOC, t, J 8 Hz, 1H), 5.33 (Ar-CH₂N⁺, s, 2H), 7.40-7.50 (H aromatic, m, 3H), 7.60-7.70 (H aromatic, m, 2H)

### H-H COSY:

Coupling between the signals at 1.61 1.69 and 1.72 ppm and the multiplet at 2.07 ppm and the two triplets at 5.08 and 5.29 ppm
Coupling between the singulet at 2.07 ppm anb the triplet at 5.08 ppm
Coupling between the doublet at 4.69 ppm and the triplet at 5.29 ppm
Coupling between the multiplet at 7.4-7.5 ppm and the one at 7.6-7.7 ppm

δC/DEPT/H-C COSY: (70 MHz, CDCl₃) 16.3 17.5 25.4 CH₃-C=, 25.7 =CH-CH₂-CH₂, 39.4 =C(CH₃)-CH₂, 50.1 (CH₃)₂-N⁺, 62.3 =CH-CH₂OOCCH₂N⁺(CH₃)₂CH₂Ph, 63.2 =CH-CH₂OOCCH₂N⁺(CH₃)₂CH₂Ph, 67.0 ROOCCH₂-N⁺(CH₃)₂CH₂Ph, 116.5 =CH-CH₂OOCCH₂N⁺, 123.3 CH=C(CH₃)₂, 126.6 C-CH₂ benzylic, 128.9 130.3 and 132.8 CH benzylic, 132.2 =C(CH₃)₂, 144.0 =C(CH₃)-CH₂, 164.6 C=O
IS/MS (C₂₁H₃₂NO₂)⁺ M= 330.5 Found m/z= 330

### Geranyloxycarbonyl-N,N,N-triethylmethanaminium bromide

Triethylamine (1.7 ml, 12.5 mmol) is slowly added portionwise over 2 hour to geranyl bromoacetate (3.44 g, 12.5 mmol), in acetone (20 ml). After an overnight stirring at room temperature, the acetone is evaporated under vacuum. The product is purified by column chromatography. Geranyl bromoacetate in excess is eluted first with chloroform then geranyloxycarbonyl-N,N,N-triethylmethanaminium bromide is eluted with ethanol.
C₁₈H₃₄BrNO₂ M= 376.38 g/mol
δH/ H-H COSY: (270 MHz, CDCl₃) 1.45 (CH₃CH₂N⁺, t, J 7.25 Hz, 9H), 1.61 1.68 1.72 (CH₃-C=, three s 9H), 2.07 (=CH-CH₂, =C(CH₃)-CH₂, m, 4H), 3.81 (CH₃CH₂N⁺, quartet, J 7.25 Hz, 6H), 4.54 (CH₂OOCCH₂N⁺, s, 2H), 4.73 (=CH-CH₂-OOC, d, J 7.3 Hz, 2H), 5.07 (=CH, m, 1H), 5.32 (=CH-CH₂OOC, t, J 7.25 Hz, 1H)
δC/DEPT H-C COSY: (70 MHz, CDCl₃) 8.1 CH₃CH₂N⁺, 16.2 17.3 25.3 CH₃-C=, 25.8 =CH-CH₂-CH₂, 39.1 =C(CH₃)-CH₂, 54.8 CH₃CH₂-N⁺, 55.8 CH₂OOCCH₂N⁺, 63.1 =CH-CH₂-OOC, 116.2 =CH-CH₂-OOC, 123.0 CH₃)₂C=CH, 131.6 =C(CH₃)₂, 144.2 =C(CH₃)-CH₂, 163.9 C=O

### Geranyloxycarbonyl-N,N-dimethyl-N-dodecylmethanaminium bromide

Dimethyldodecylamine (2.75 ml, 10 mmol) is slowly added portionwise over 1 hour to geranyl bromoacetate (3.44 g, 12.5 mmol), in acetone (20 ml). After two hours stirring at room temperature, the acetone is evaporated under vacuum. The brown solid recovered is triturated ine ether (100 ml), until completetly converted into a fine white solid. This solid is filtered off, washed carefully with ether (2* 20 ml) and recrystallised from ether/acetone (75%/25% v/v)..
Yield 3.81 g, 78.3%
C₂₆H₅₀BrNO₂ M= 488.59 g/mol mp: 92.5°C
δH/ H-H COSY: (270 MHz, CDCl₃) 0.88 (CH₃CH₂, t, J 6.9 Hz, 3H), 1.25 (CH₂, m, 16H), 1.33 (CH₂CH₂CH₂N⁺ m, 2H), 1.61 1.69 1.72 (CH₃-C=, three s, 9H), 1.80 (⁺NCH₂CH₂, m, 2H), 2.07 (=CH-CH₂, =C(CH₃)-CH₂, m, 4H), 3.64 (CH3-N⁺, s, 6H), 3.80 (CH₂CH₂N⁺, m, 2H), 4.71 (=CH-CH₂-OOC, d, J 7.3 Hz, 2H), 4.90 (CH₂OOCCH₂N⁺, s, 2H), 5.07 (=CH, m, 1H), 5.31 (=CH-CH₂OOC, t, J 7.5 Hz, 1H)
δC/DEPT H-C COSY: (70 MHz, CDCl₃) 13.8 CH₃CH₂, 16.3 17.4 25.4 CH₃-C=, 22.3 22.6 25.8 25.9 28.8 28.98 29.03 29.12 29.25 31.55 CH₂ =CH-CH₂-CH₂, 39.2 =C(CH₃)-CH₂, 51.5 (CH₃)₂-N⁺, 60.9 CH₂OOCCH₂N⁺, 63.1 =CH-CH₂-OOC, 64.3 CH₂CH₂N⁺, 116.4 =CH-CH₂-OOC, 123.2 CH₃)₂C=CH, 131.6 =C(CH₃)₂, 144.0 =C(CH₃)-CH₂, 164.3 C=O

### Geranyloxycarbonyl-N,N-dimethyl-N-octylmethanaminium bromide

Same experimental as for geranyloxycarbonyl-N,N-dimethyl-N-dodecylmethanaminium Bromide.
Reaction done on 10 mmol of dimethyloctylamine.
Yield of geranyloxycarbonyl-N,N-dimethyl-N-octylmethanaminium bromide (2.95 g, 68.2%) white solid.
C₂₂H₄₂BrNO₂ M= 432.48 g/mol mp: 92°C
δH/ H-H COSY: (270 MHz, CDCl₃) 0.88 (CH₃CH₂, t, J 6.9 Hz, 3H), 1.25 (CH₂, m, 8H), 1.33 (CH₂CH₂CH₂N⁺ m, 2H), 1.61 1.69 1.72 (CH₃-C=, three s 9H), 1.77 (⁺NCH₂CH₂, m, 2H), 2.07 (=CH-CH₂, =C(CH₃)-CH₂, m, 4H), 3.65 (CH3-N⁺, s, 6H), 3.85 (CH₂CH₂N⁺, m, 2H), 4.71 (=CH-CH₂-OOC, d, J 7.3 Hz, 2H), 4.87 (CH₂OOCCH₂N⁺, s, 2H), 5.07 (=CH, m, 1H), 5.31 (=CH-CH₂OOC, t, J 7.5 Hz, 1H)
δC/DEPT H-C COSY: (70 MHz, CDCl₃) 13.8 CH₃CH₂, 16.4 17.5 25.5 CH₃-C=, 22.4 22.7 25.9 26.1 28.8 28.9 31.4 CH₂ =CH-CH₂-CH₂, 39.4 =C(CH₃)-CH₂, 51.6 (CH₃)₂-N⁺, 61.1 CH₂OOCCH₂N⁺, 63.2 =CH-CH₂-OOC, 64.4 CH₂CH₂N⁺, 116.5 =CH-CH₂-OOC, 123.3 CH₃)₂C=CH, 131.8 =C(CH₃)₂, 144.2 =C(CH₃)-CH₂, 164.2 C=O

### Geranyloxycarbonyl-N,N-dimethyl-N-(2-palmitoylethyl) methanaminium bromide

(2-dimethylamino)ethyl palmitoate(Me2NCH2CH2OOCC15H31, 3.28 g, 10 mmol) is slowly added portionwise over 1 hour to geranyl bromoacetate (3.44 g, 12.5 mmol), in acetone (20 ml). After two hours stirring at room temperature, the acetone is evaporated under vacuum. The product is purified by column chromatography. Geranyl bromoacetate in excess is eluted first with chloroform then geranyloxycarbonyl-N,N,N-triethylmethanaminium bromide is eluted with ethanol.
Yield 0.80g, 13.3% of a brown oil.
C₃₂H₆₀BrNO₄ M= 602.74 g/mol
δH: (270 MHz, CDCl₃) 0.88 (CH₃CH₂, t, J 6.9 Hz 6.3 Hz, 3H), 1.26 (CH₂, m, 24H), 1.61 1.69 1.72 (CH₃-C=, three s 9H), 1.77 (CH₂CH₂COOCH₂, m, 2H), 2.07 (=CH-CH₂, =C(CH₃)-CH₂, m, 4H), 2.31 (CH₂CH₂COOCH₂, t 7.9 Hz 7.2 Hz, 2H), , 3.83 (CH3-N⁺, s, 6H), 4.35 (COOCH₂CH₂N⁺Me₂, m, 2H), 4.56 (COOCH₂CH₂N⁺Me₂,m, 2H), 4.72 (=CH-CH₂-OOC, d, J 7.25 Hz, 2H), 4.91 (CH₂OOCCH₂N⁺, s, 2H), 5.07 (=CH, m, 1H), 5.31 (=CH-CH₂OOC, t, J 6.9 Hz 7.2 Hz, 1H)
δC/DEPT H-C COSY: (70 MHz, CDCl₃) 14.0 CH₃CH₂, 16.5 17.6 25.6 CH₃-C=, 22.6 24.5 26.1 29.03 29.20 29.25 29.38 29.48 29.86 31.8 CH₂, 33.8 CH₂CH₂COOCH₂, 39.5 =C(CH₃)-CH₂, 52.7 (CH₃)₂-N⁺, 57.4 COOCH₂CH₂N⁺Me₂, 62.0 =CH-CH₂OOCCH₂N⁺, 62.5 COOCH₂CH₂N⁺Me₂, 63.4 =CH-CH₂-OOC, 116.5 =CH-CH₂-OOC, 123.3 CH₃)₂C=CH, 131.9 =C(CH₃)₂, 144.3 =C(CH₃)-CH₂, 164.6 =CH-CH₂-OOC, 172.5 COOCH₂CH₂N⁺Me₂

### Linalyloxycarbonyl-N,N-dimethyldodecylmethanaminium bromide

Same experimental as for geranyloxycarbonyl-N,N-dimethyl-N-dodecylmethanaminium Bromide.
Reaction done on 10 mmol of dimethyldodecylamine.
After evaporation of acetone, the brown oil recovered is purified by column chromatography. linalyloxycarbonyl-N,N-dimethyldodecylmethanaminium bromide is eluted first with petroleum ether 40-60°C then linalyl bromoacetate in excess is eluted with ethanol.
Yield of linalyloxycarbonyl-N,N-dimethyldodecylmethanaminium bromide (2.84g, 38.8%, brown oil).
C₂₆H₅₀BrNO₂ M= 488.59 g/mol
CCM: eluant petroleum ether 40-60°C R_{f} = 0.92
δH/ H-H COSY: (270 MHz, CDCl₃) 0.88 (CH₃CH₂, t, J 6.9 Hz 6.3 Hz, 3H), 1.25 (CH₂, m, 16H), 1.33 (CH₂CH₂CH₂N⁺ m, 2H), 1.59 and 1.68 (CH₃-COOC (CH₃)₂C=, m, 9H), 1.80-2.00 (⁺NCH₂CH₂ =CH-CH₂ and OOCC(CH₃)-CH₂, m, 6H), 3.62 (CH3-N⁺, s, 6H), 3.77 (CH₂CH₂N⁺, m, 2H), 4.70 (CH₂OOCCH₂N⁺, s, 2H), 5.05 ((CH₃)₂C=CH, m, 1H), 5.20-5.28 (=CH₂, m, 2H), 5.93-6.03 (CH₂=CH, m, 1H)
δC/DEPT H-C COSY: (70 MHz, CDCl₃) 14.1 CH₃CH₂, 17.7 CH₃-C=, 22.4 =CH-CH₂, 23.0 CH₃-C=, 25.7 (CH₃)-COOC, 22.7 26.1 29.16 29.34 29.45 29.59 and 31.9 CH₂, 39.2 CH₂-C(CH₃)-OOC, 52.1 (CH₃)₂-N⁺, 61.3 C(CH₃)OOCCH₂N⁺, 64.6 CH₂CH₂N⁺, 115.3 CH=CH₂, 123.0 CH =C(CH₃)₂, 132.5 CH =C(CH₃)₂, 139.8 CH=CH₂, 163.3 C=O

### Tetrahydrolinalyloxycarbonyl-N,N-dimethyl-N-dodecylmethanaminium

Experimental: as for geranyloxycarbonyl-N,N-dimethyl-N-dodecylmethanaminium.
Done with dimethyldodecylamine (14.6 ml, 53 mmol) tetrahydrolinalyl bromoacetate (17.7 g, 63 mmol, 1.2 eq, purity > 95% by GC/MS)
Acetone (100 ml in total)
Reaction time: 2h30 at room temperature
Recristallised in ether/ethanol (90%/10% v/v)
Yield: 23.35 g (89.4%) of a fine white solid
C₂₆H₅₄BrNO₂ M= 492.62 g/mol PF: 111°C
δH: (270 MHz, CDCl₃) 0.85-0.90 (CH₃, m, 12H), 1.13-1.33 (CH₂, m, 22H), 1.42 (CH₃-C-O-OCCH₂N⁺, s, 3H), 1.50-1.60 (CH, m, 1H), 1.68-2.0 (CH₂, m, 6H), 3.66 (CH3-N⁺, s, 6H), 3.81 (CH₂CH₂N⁺, m, 2H), 4.62 (COOCCH₂N⁺, s, 2H)
δC/DEPT/H-C COSY: (70 MHz, CDCl₃) 7.9 13.8 22.3 CH₃, 23.0 CH₃-C-O-OCCH₂N⁺, 21.2 22.4 22.7 25.8 28.9 29.0 29.07 29.14 29.27 30.5 37.6 38.8 CH₂, 27.4 (CH₃)₂CH, 51.7 (CH₃)₂-N⁺, 60.9 C(CH₃)OOCCH₂N⁺, 64.0 CH₂CH₂N⁺, 90.6 C(CH₃)OOCCH₂N⁺, 163.2 C=O

### H-C COSY: (major peaks)

Direct coupling of the multiplet at 0.85-0.90 ppm (1H) with 7.7 13.8 and 22.3 ppm (13C)
Direct coupling of the singulet at 1.42 ppm (1H) with 23.0 ppm (13)
Direct coupling of the multiplet at 1.5-1.6 ppm (1H) with 27.4 ppm (13C)
Direct coupling of the singulet at 3.66 ppm (1H) with 51.7 ppm (13C)
Direct coupling of the multiplet at 3.81 ppm (1H) with 64.0 ppm (13C)
Direct coupling of the singulet at 4.62 ppm (1H) with 60.9 ppm (13C)

### 1,2-dihydromyrcenyloxycarbonyl-N,N-dimethyl-N-dodecylmethanaminium bromide

Experimental: as for geranyloxycarbonyl-N,N-dimethyl-N-dodecylmethanaminium.
Done with dimethyldodecylamine (13 ml, 47.2 mmol).
1,2-dihydromyrcenyl bromoacetate as previously described (starting from 70.7 mmol of 1,2-dihydromyrcenol)(80% 1,2-dihydromyrcenyl bromoacetate and 20% 1,2-dihydromyrcenol by GC/MS).
After evaporation of acetone, the brown oil recovered is purified by column chromatography. 1,2-dihydromyrcenyloxycarbonyl-N,N-dimethyldodecylmethanaminium bromide (12.7g, 46%) is eluted first with chloroform then 1,2-dihydromyrcenyl bromoacetate (3.68g) in excess is eluted with ethanol.
C₂₆H₅₂BrNO₂ M= 490.6 g/mol
δH: (270 MHz, CDCl₃) 0.88 (CH₃-(CH₂)₁₁N⁺, t, 6.7 Hz, 3H), 0.98 (CH₃-CH, d, 6.9 Hz, 3H), 1.20-1.35 (CH₂, m, 22H), 1.47 ((CH₃)₂C-OOC, s, 6H), 1.70-1.80 (CH₂, m, 4H), 2.1-2.2 (CH₃-CH, m, 1H), 3.64 (CH3-N⁺, s, 6H), 3.83 (CH₂CH₂N⁺, m, 2H), 4.57 (COOCCH₂N⁺, s, 2H), 4.88-4.98 (CH₂=, m, 2H), 5.59-5.71 (CH₂=CH, m, 1H)
δC/DEPT/H-C COSY: (70 MHz, CDCl₃) 13.7 CH₃-(CH₂)₁₁, 19.9 CH₃-CH, 21.2 22.2 22.6 CH₂, 25.4 (CH₃)₂C-OOC, 25.9 28.7 28.90 28.95 29.05 29.15 31.5 CH₂, 36.2 40.3 CH₂, 37.1 CH₃-CH, 51.6 (CH₃)₂-N⁺, 61.0 C(CH₃)OOCCH₂N⁺, 64.1 CH₂CH₂N⁺, 87.5 C(CH₃)₂OOCCH₂N⁺, 112.4 CH=CH₂, 143.9 CH =CH₂, 163.1 C=O

### H-C COSY: (major peaks)

Direct coupling of the triplet at 0.88 ppm (1H) with 13.7 ppm (13C)
Direct coupling of the doublet at 0.98 ppm (1H) with 19.9 ppm (13C)
Direct coupling of the singulet at 1.47 ppm (1H) with 25.4 ppm (13C)
Direct coupling of the multiplet at 2.1-2.2 ppm (1H) with 37.1 ppm (13C)
Direct coupling of the singulet at 3.64 ppm (1H) with 51.6 ppm (13C)
Direct coupling of the multiplet at 3.83 ppm (1H) with 64.1 ppm (13C)
Direct coupling of the singulet at 4.57 ppm (1H) with 61.0 ppm (13C)
Direct coupling of the multiplet at 4.88-4.98 ppm (1H) with 112.4 ppm (13C)
Direct coupling of the multiplet at 5.59-5.71 ppm (1H) with 143.9 ppm (13C)

| **Solvent** | **Conditions** | **Proportions of each compounds in the final reaction mixture** |
|---|---|---|
| Toluene excess geranyl bromo acetate (1.25 eq) | 1h30 at 50°C | ∼65 % geranyl bromoacetate : starting product |
| | | 25 % geranyloxycarbonyl-N,N,N-triethylmethanaminium : desired product |
| | | ∼3 % N-geranyl-N,N,N-triethylammonium : degradation product |
| | 3h30 at 50°C | 40 % geranyl bromoacetate |
| | | 40 % geranyloxycarbonyl-N,N,N-triethylmethanaminium |
| | | 20 % N-geranyl-N,N,N-triethylammonium |
| | 4h30 at 50°C | 25 % geranyl bromoacetate |
| | | 45 % geranyloxycarbonyl-N,N,N-triethylmethanaminium |
| | | 30 % N-geranyl-N,N,N-triethylammonium |
| | 4h30at 50°C+ 16 h at R.T. | as after 4h30 at 50°C |
| | 4h30at 50°C+ 40 h at R.T. | as after 4h30 at 50°C |
| Acetone | 1h30 at 50°C | ∼60 % geranyl bromoacetate |
| | | 30 % geranyloxycarbonyl-N,N,N-triethylmethanaminium |
| | | ∼10 % N-geranyl-N,N,N-triethylammonium |
| | 6h at 50°C | 30 % geranyl bromoacetate |
| | | 50 % geranyloxycarbonyl-N,N,N-triethylmethanaminium |
| | | 20 % N-geranyl-N,N,N-triethylammonium |
| | 18h at 50°C | Only traces of geranyloxycarbonyl-N,N,N-triethylmethanaminium 7 times more N-geranyl-N,N,N-triethylammonium than geranyloxycarbonyl-N,N,N-triethylmethanaminium |
| Acetone Excess geranyl bromo acetate (1.25 eq) | 3h30 at R.T. | ∼25 % geranyl bromoacetate |
| | | 70 % geranyloxycarbonyl-N,N,N-triethylmethanaminium |
| | | 1-5 % N-geranyl-N,N,N-triethylammonium |
| | 6h at R.T. | 15 % geranyl bromoacetate |
| | | 85 % geranyloxycarbonyl-N,N,N-triethylmethanaminium |
| | | 1-5 % N-geranyl-N,N,N-triethylammonium |
| | 18h at R.T. | 7 % geranyl bromoacetate |
| | | 92 % geranyloxycarbonyl-N,N,N-triethylmethanaminium |
| | | ∼1 % N-geranyl-N,N,N-triethylammonium |
| **Note:** all quaternary ammonium salts are bromide salts. | | |

### Example 6

### STABILITY PROFILE OF THE C12 BEG

As for the BEG, the C12 BEG shows a profile of hydrolysis very dependent on pH. Very importantly, its rate of hydrolysis at pH neutral (∼7) is far faster than for a normal ester.
C₁₂ BEG is : 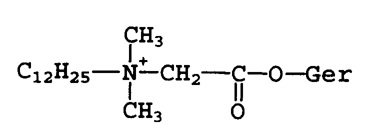 BEG is : 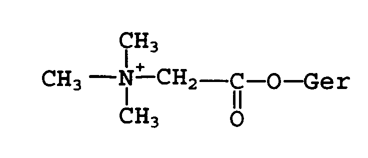 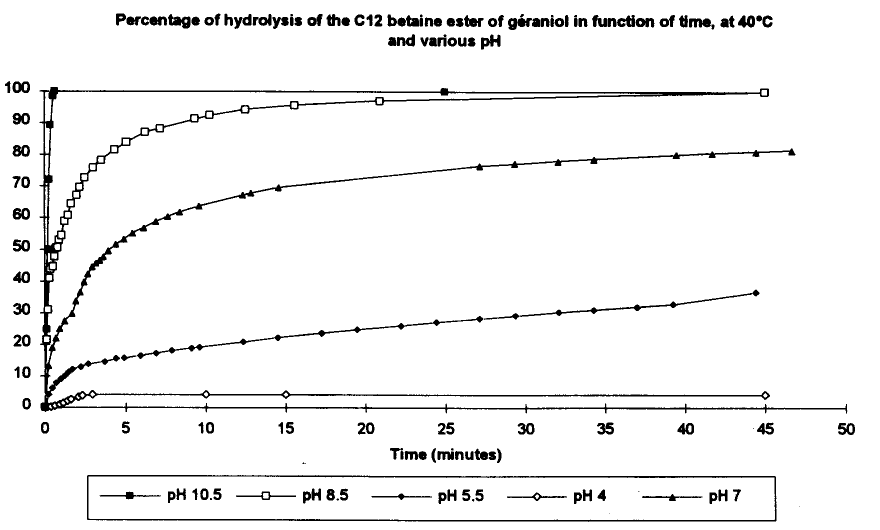 Concentration of C12 BEG used: 1.25 10⁻² mol/l

### Example 7

### STABILISATION OF A BETAINE ESTER BY ANIONIC SURFACTANTS ENABLING ITS USE IN A DETERGENT MATRIX

Betaine esters are far more unstable under alkaline conditions than normal esters. Using betaine esters for applications where the pH is highly alkaline is difficult if only the pH is considered. But betaine esters are greatly stabilised by anionic surfactants. Under the same conditions of pH and temperature, the rate of hydrolysis of a betaine ester in the presence of anionic surfactants is far lower than in pure water. This stabilisation is large enough to use betaine esters through the wash.

### STABILISATION OF THE C12 BEG BY ADDITION OF VARIOUS QUANTITIES OF ANIONIC SURFACTANTS

### EXAMPLE a: ALKYLSULFATES, ALKYLSULFONATES AND FATTY ACIDS SALTS, ALL STABLISED THE C12 BEG TOWARDS SAPONIFICATION

### a) AT pH 8.5 and 40°C

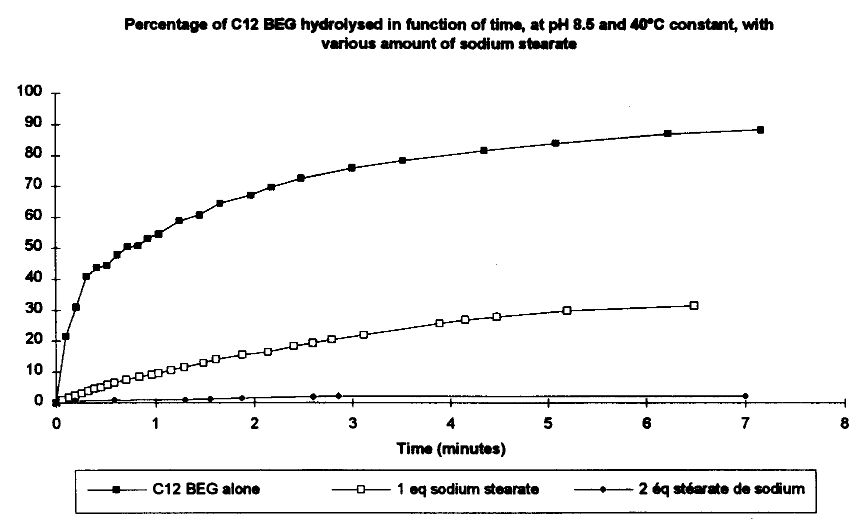

Test conditions: C12 BEG added to a solution (pH 10.5) of sodium stearate in distilled water. pH and temperature maintained constant all along the test. Percentage of C12 BEG hydrolysed assessed using an automatic pH stat titrator.
- Concentrations used:: C12 BEG 1.25 10⁻² mol/l
- Sodium stearate:: 1 equivalent 1.25 10⁻² mol/l
2 equivalents 2.5 10⁻² mol/l

| Sample (done at 40°C and pH 8.5) | k_{obs}(min⁻¹) at pH 8.5 | k(mol⁻ ¹.l.min⁻¹) | t_{1/2} (min) pH 8.5 |
|---|---|---|---|
| C12 BEG alone | >0.3 | >100 000 | <2 minutes |
| With 1 equivalent sodium stearate | 0.0643 | 20 333 | 11 |
| With 2 equivalent sodium stearate | 0.00286 | 904 | 243 |

### b) At pH 10.5 and 40°C

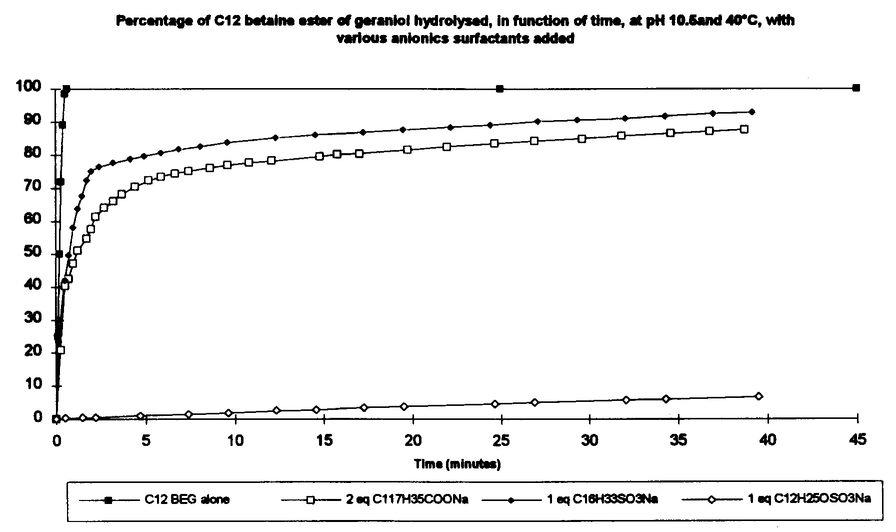

Conditions: as before

| Sample (done at 40°C and pH 10.5) | k_{obs}(min⁻¹) at pH 8.5 | k(mol⁻ ¹.l.min⁻¹) | t_{1/2} (min) pH 10.5 |
|---|---|---|---|
| C12 BEG alone | >30 | >100 000 | < 2 seconds |
| With 1 equivalent sodium hexadecylsulfonate | 0.5 | 1580 | 1.4 |
| With 2 equivalents sodium stearate | 0.4 | 1265 | 1.7 |
| With 1 equivalent sodium dodecylsulfate | 0.0017 | 5.3 | 414 |

### EXAMPLE b: BEG IS ALSO STABILISED TOWARDS SAPONIFICATION BY ADDITION OF ANIONIC SURFACTANTS

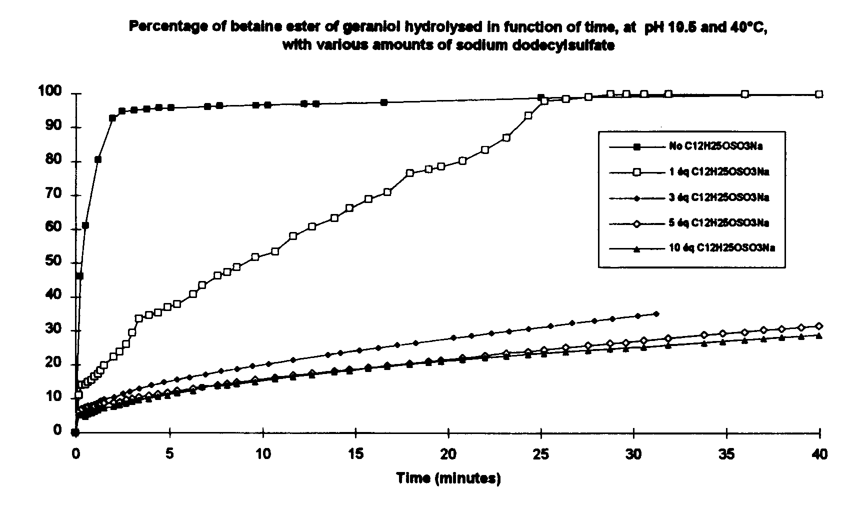

Conditions: as before

| Sample (done at 40°C and pH10.5) | k_{obs}(min⁻¹) at pH 10.5 | k(mol⁻ ¹.l.min⁻¹) | t_{1/2} (min) at pH 10.5 |
|---|---|---|---|
| BEG alone | 1.25 | 3950 | ∼30 seconds |
| BEG with 1 equivalent of sodium dodecylsulfate | 0.064 | 202 | 10.8 |
| BEG with 3 equivalents of sodium dodecylsulfate | 0.0111 | 35 | 63 |
| BEG with 5 equivalents of sodium dodecylsulfate | 0.0074 | 23.5 | 93 |
| BEG with 10 equivalents of sodium dodecylsulfate | 0.0067 | 21 | 104 |

### EXAMPLE c

### STABILISATION OF THE C12 BETAINE ESTER OF GERANIOL BY REPLACING THE HALOGENE COUNTER-ION BY A MORE HYDROPHOBIC ANIONIC SURFACTANT AND STABILISATION OF THIS NEW ION PAIR C12 BEG/DODECYLSULFATE [C12BEG/DS] BY ADDITION OF MORE ANIONIC SURFACTANTS

### At high betaine ester concentration (1.25 10⁻² mol/l)

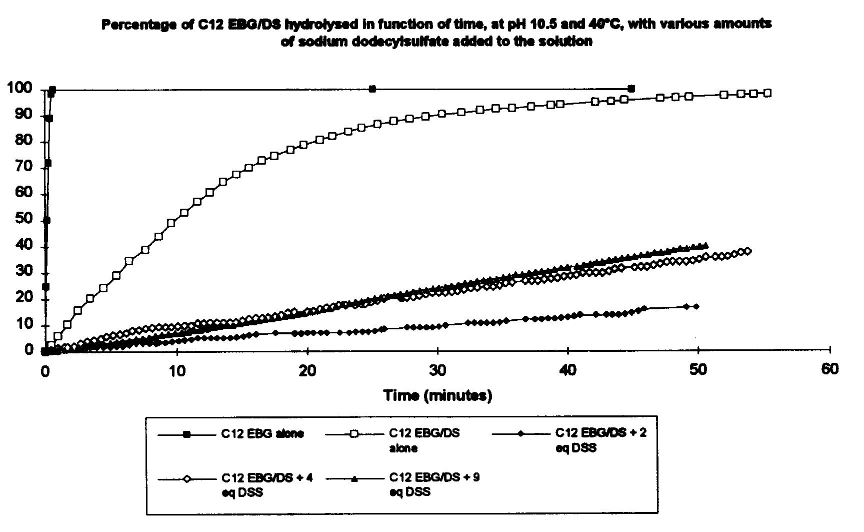

| Sample (done at 40°C and pH10.5) | k _{obs} (min⁻¹) | k (mol⁻ ¹.l.min⁻¹) | t_{1/2} (min) |
|---|---|---|---|
| C12 BEG alone | >30 | >100 000 | <2 seconds |
| C12 BEG/DS (1.25 10⁻² mol/l) alone | 0.0724 | 229 | 9.6 |
| C12 BEG/DS with 2 equivalents of sodium dodecylsulfate | 0.0034 | 10.6 | 206 |
| C12 BEG/DS with 4 equivalents of sodium dodecylsulfate | 0.0083 | 26.1 | 84 |
| C12 BEG/DS with 9 equivalents of sodium dodecylsulfate | 0.0104 | 32.8 | 67 |

### At a lower betaine ester concentration (10⁻³ mol/l)

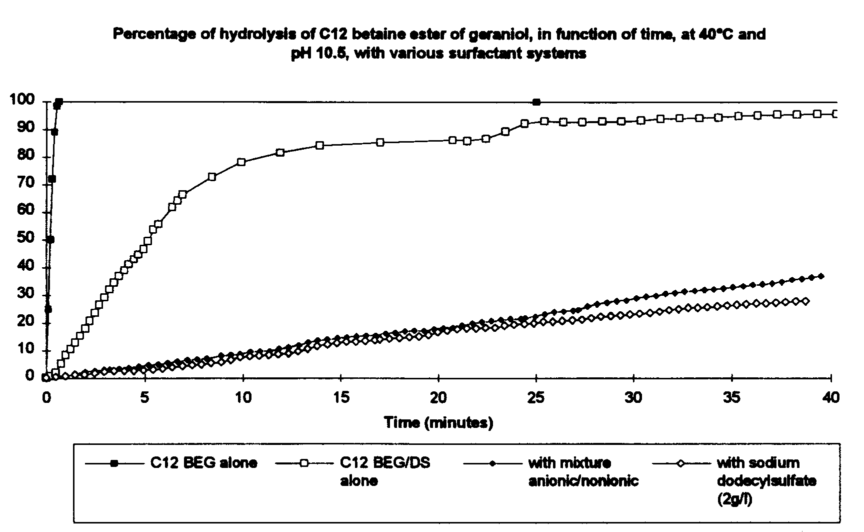

Hydrolysis rate determined with a Radiometer pH stat.
Temperature (40°C) and pH (10.5) maintained constant. Concentration in C12 BEG or new ion pair C12 BEG/DS: 10⁻³ mol/l.
Mixture anionic/nonionic: sodium dodecylsulfate (0.43 g/l), sodium stearate (0.013 g/l), alkylethoxylate (Dobanol 45 7 from Shell, 0.38 g/l), N-cocoyl-N-methylglucamine (0.16 g/l).
Kinetics of hydrolysis of C12 BEG/DS, with or without surfactants all follow a first order in [C12BEG/DS].

| Sample (done at 40°C and pH10.5) | k _{obs} (min⁻¹) | k(mol⁻ ¹.l.min⁻¹) | t_{1/2} (min) |
|---|---|---|---|
| C12 BEG alone | >5 | >15000 | <10 seconds |
| C12 BEG/DS (10⁻³ mol/l) alone | 0.134 | 423.7 | 5.2 |
| With mixture anionic/nonionic | 0.01169 | 37 | 59 |
| With sodium dodecylsulfate only | 0.0095 | 28.6 | 77 |

### Example 8

### TYPICAL FABRIC SOFTENER COMPOSITIONS WHEREIN THE BETAINE ESTERS ACCORDING TO THE INVENTION ARE USED COMPRISE THE FOLLOWING MAIN INGREDIENTS

| | |
|---|---|
| DEEDMAC (1) | 6.7% |
| MP10 antifoam emulsion^{R} | 0.05% |
| HCl | 0.09% |
| Dye | 0.05% |
| Perfume | 0.20% |
| Water | Up to 100% |

| | |
|---|---|
| (1) DEEDMAC = N,N-di(tallowoyl-oxy-ethyl)-N,N-dimethylammonium chloride | |

or

| | |
|---|---|
| DEEDMAC (1) | 22.5% |
| MP10 antifoam emulsion^{R} | 0.1% |
| HCl | 0.06% |
| Soil release polymer | 0.5% |
| PEG 4000 | 1.2% |
| Dye | 0.25% |
| CaCL2 | 0.9% |
| Perfume | 0.7% |
| Water | Up to 100% |

| | |
|---|---|
| (1) DEEDMAC = N,N-di(tallowoyl-oxy-ethyl)-N,N-dimethylammonium chloride | |

### Example 9

### A TYPICAL HARD SURFACE CLEANING COMPOSITION WHEREIN THE BETAINE ESTERS ACCORDING TO THE INVENTION ARE USED COMPRISING THE FOLLOWING MAIN INGREDIENTS

| | |
|---|---|
| Nonionic surfactants | 16% |

| Anionic surfactants | |
|---|---|
| - Sodium parafin sulfonate | 3% |
| - C8 alkyl sodium sulfate | 2% |

| Hydrotrope | |
|---|---|
| - Sodium cumene sulfonate | 3% |

| Buffer | |
|---|---|
| - Potassium carbonate | 2% |

| Foam control | |
|---|---|
| - Fatty acid | 0.8% |
| Perfume | 0.8% |
| Geraniol dodecyl dimethyl betaine | 1% |

This formulation was used to clean a ceramic tile (30x30cm) which was then rinsed with water and left to dry. The odour of the tile was then graded by panelists on a scale of 1-5 (very weak, weak, moderate, strong and very strong) over a period of several hours. The identical formulation but containing no betaine was used as a reference and graded at the same time (a grade of 1 is considered to be consumer noticeable).
The results of this testing showed a significant odour longevity benefit for the formulation containing the betaine.

## Claims

1. A composition comprising a compound of general formula selected from: 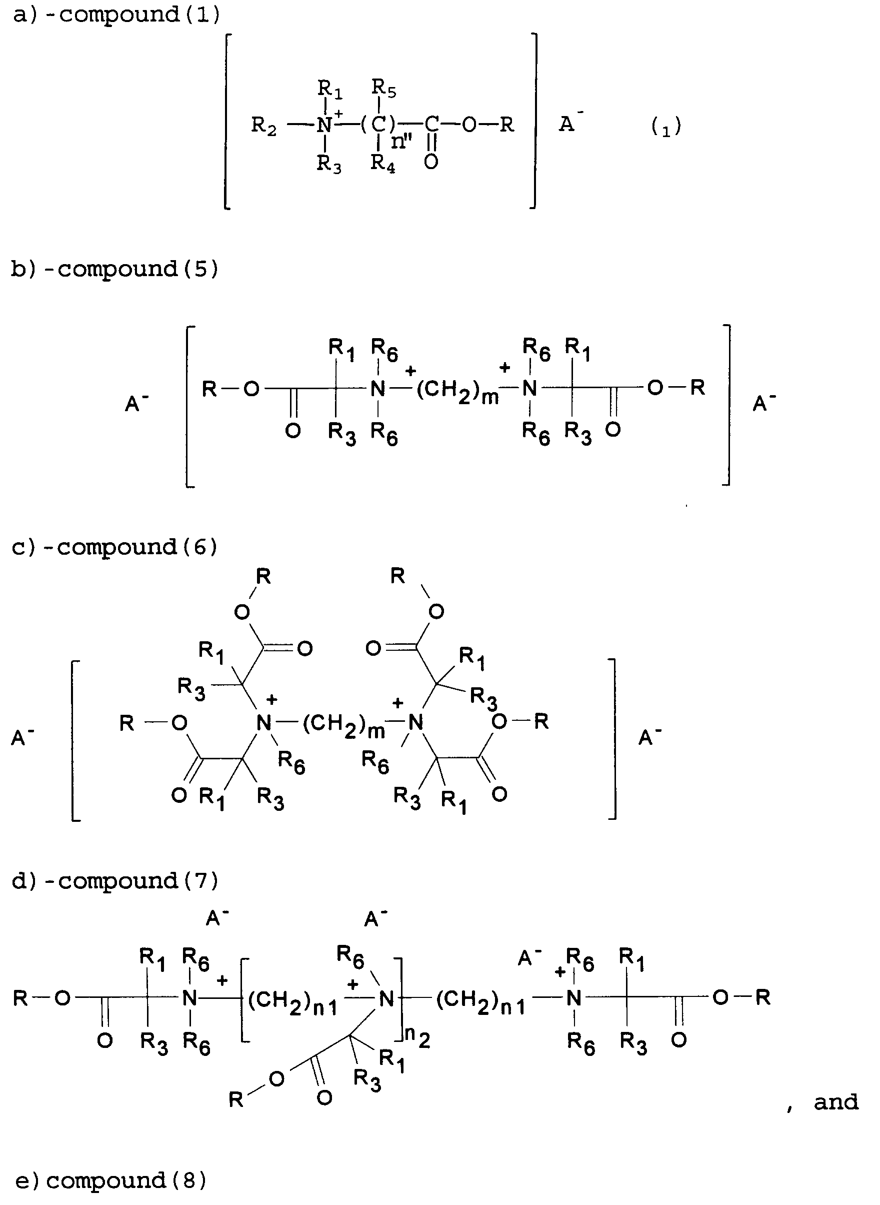 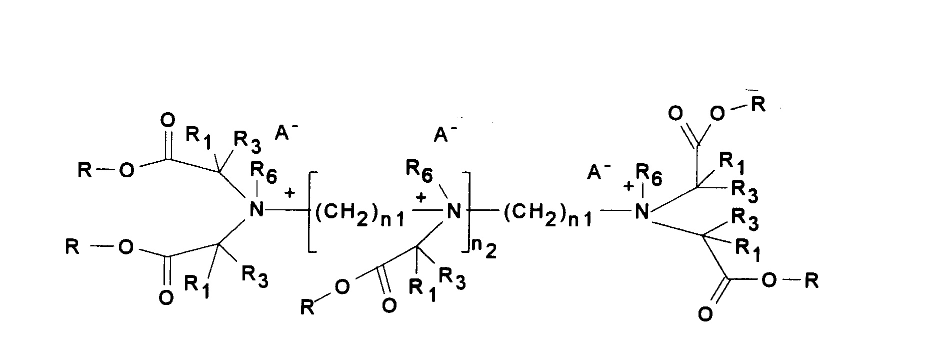 wherein
each R, independently, is derived from an alcohol of more than four (4) carbon atoms of synthetic or natural origin;
each R₁, R₂, R₃, independently is hydrogen, alkyl, hydroxyalkyl, aryl, phenyl, -(CH₂)_{n'}-O-CO-R' with n'≧1,
preferably 2 or 3, and R' is a C₁-C₂₀ (un)saturated alkyl chain, preferably C₇-C₂₀, or a -(CH₂)ₙ-CO-OR'' group wherein R'' is derived from an alcohol of synthetic or natural origin and n is 1, 2 or 3, preferably 1;
each R₄, R₅, independly, is a hydrogen, alkyl, hydroxyalkyl, aryl, phenyl or -(CH₂)ₙ-CO-OR'', with R'' is derived from an alcohol and
n is an integer preferably 0, 1 or 2;
each A is a compatible anion and
n'' is an integer having the value of 1, 2 or 3, preferably 1, and
wherein in b), c), d) and/or e)
each R₆, independently is hydrogen, alkyl, hydroxyalkyl, aryl or phenyl,
each m is an integer of value equal or greater than 1,
each n1 is an integer lying in the range of 1 to 4,
n2 is an integer lying in the range of 1 to 6,
said compound having an efficient deposition to a surface followed by delayed activated-release of the R-group and/or R'' group.

2. A composition according to claim 1 wherein the compound is: 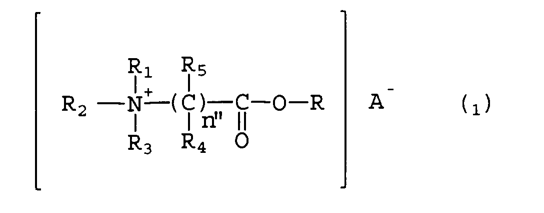 wherein
R₁, R₂ and R₃ is methyl;
R₄ and R₅ is hydrogen,
R is derived from an alcohol of more than four (4) carbon atoms of synthetic or natural origin and
n'' has the integer of 1.

3. A composition according to claim 1 wherein the compound is: 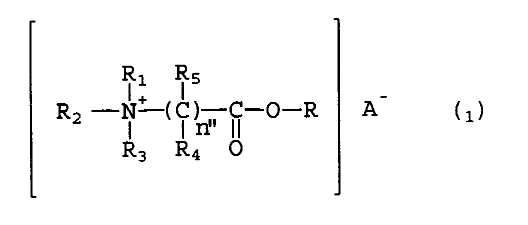
wherein R₁ and R₂ are methyl, R₃ is an alkyl/alkenyl chain preferably butyl, octyl, dodecyl or benzyl, or an aryl/phenyl chain, or a pyridine derivative, R₄ and R₅ is hydrogen, R is derived from an alcohol of more than four (4) carbon atoms of synthetic or natural origin and n'' has the integer of 1.

4. A composition according to claim 1 wherein the compound is: 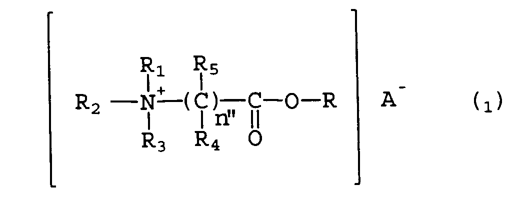
wherein R₁, R₂, R₃ is ethyl, R₄ and R₅ is hydrogen, R is derived from an alcohol of more than four (4) carbon atoms of synthetic or natural origin and n'' has the integer of 1.

5. A composition according to claim 1 wherein the compound is: 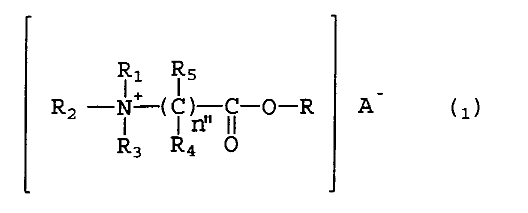 wherein
R₁ and R₂ is (the same or different) hydrogen, alkyl, hydroxyalkyl, aryl, phenyl, preferably methyl,
R₃ is a -CH₂-CO-OR' group
R₄ and R₅ is hydrogen;
R and R' are derived from alcohols (the same or different) of more than four (4) carbon atoms of synthetic or natural origin and n'' has the integer of 1.

6. A composition according to claim 1 wherein the compound is: 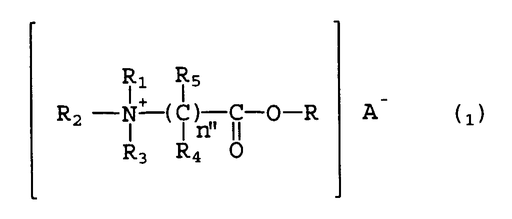 wherein
R₁ and R₂ is -(CH₂)n'-O-CO-R' with n'≧1, preferably 2 or 3 and R' is a C₁-C₂₀ (un)saturated alkyl chain, preferably C₇-C₂₀;
R₃ is hydrogen, alkyl, hydroxy alkyl, aryl, phenyl, preferably methyl,
R₄ and R₅ is a hydrogen;
R is derived from an alcohol of more than four (4) carbon atoms of synthetic or natural origin and n'' has the integer of 1.

7. A composition according to claim 1 wherein the compound is: 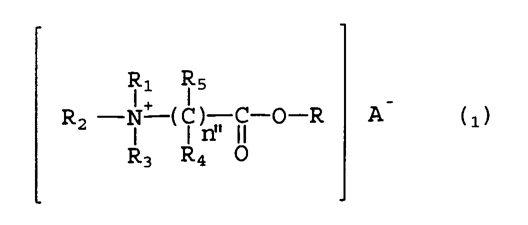
wherein R₁ is hydrogen, alkyl, hydroxyalkyl, aryl, phenyl or -(CH₂)_{n'}-O-CO-R' with n'≧ 1, preferably 2 or 3 and R' is a C₁-C₂₀ (un)saturated alkyl chain, preferably C₇-C₂₀; and R₂ and R₃ are hydrogen, alkyl, hydroxyalkyl, aryl, phenyl, preferably methyl.

8. A compound with the general formula (2) 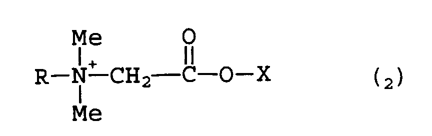
wherein R is alkyl with at least 2 C atoms, preferably butyl, octyl, dodecyl, benzyl, aryl, phenyl, pyridine derivative,
-(CH₂)_{n'}-O-CO-R' with n' is preferably 2 or 3 and R' is C₁-C₂₀ atoms;
Me is a methyl group and
X is an alkyl part of an odoriferous alcohol, such as geranyl, linalyl, tetrahydrolinalyl, 1,2-dihydromyrcenyl.

9. A compound with the general formula (3) 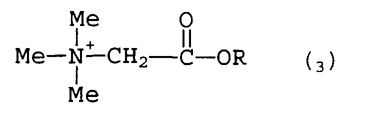
wherein Me is a methyl group and R is defined as an odoriferous alcohol selected from the group of 2-phenoxyethanol, phenylethylalcohol, geraniol, citronellol, 3-methyl-5-phenyl-1-pentanol, 2,4-dimethyl-3-cyclohexene-1-methanol, linalool, tetrahydrolinalool, 1,2-dihydromyrcenol, hydroxycitronellal, farnesol, menthol, eugenol, vanilin, cis-3-hexenol or mixtures thereof.

10. A compound of formula selected from: 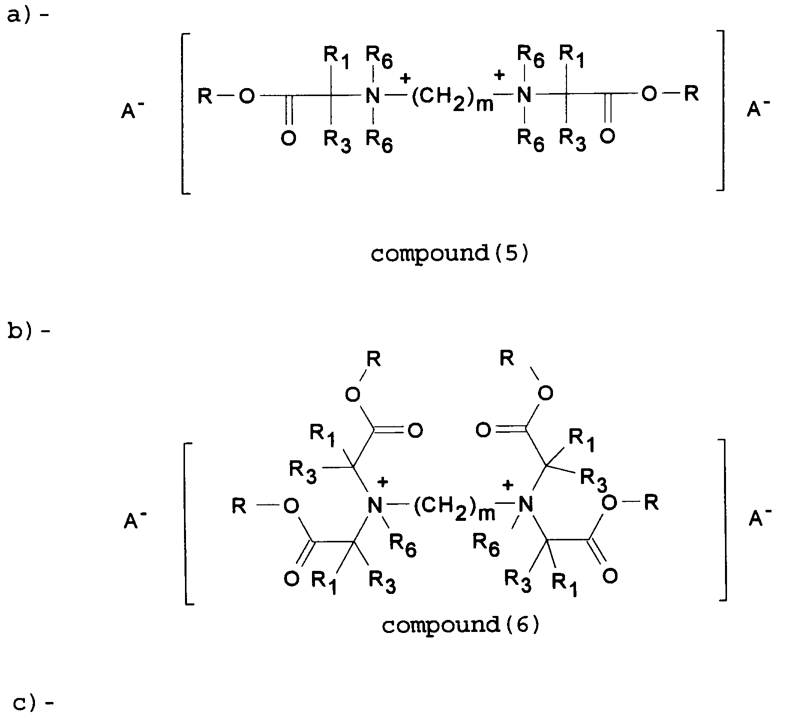 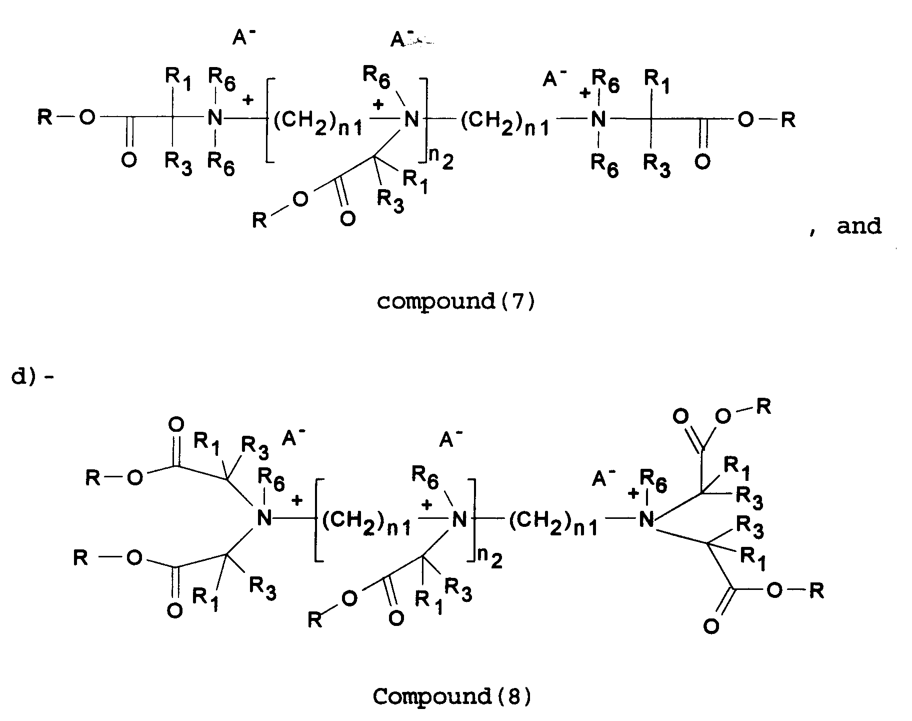 wherein
each R, independently, is derived from an alcohol of more than four (4) carbon atoms of synthetic or natural origin;
each R₁, R₃, independently is hydrogen, alkyl, hydroxyalkyl, aryl, phenyl, -(CH₂)_{n'}-O-CO-R' with n'≧1,
preferably 2 or 3, and R' is a C₁-C₂₀ (un)saturated alkyl chain, preferably C₇-C₂₀, or a -(CH₂)ₙ-CO-OR'' group wherein R'' is derived from an alcohol of synthetic or natural origin and n is 1, 2 or 3, preferably 1;
each R₆, independently is hydrogen, alkyl, hydroxyalkyl, aryl or phenyl,
each A is a compatible anion and
each n, independently, is an integer lying in the range of 0 to 2;
each n'', independently, is an integer lying in the range of 1 to 3, preferably 1, and
each m is an integer of value equal or greater than 1,
each n1 is an integer lying in the range of 1 to 4, and
n2 is an integer lying in the range of 1 to 6.

11. A compound according to Claim 10, wherein
each R is derived from an alcohol of more than four (4) carbon atoms of synthetic or natural origin;
each R₁, R₃ is hydrogen;
each R6 is methyl,
m is an integer of value 1,2,3,4 or 6.

12. A compound according to Claim 10, wherein
each R is derived from an alcohol of more than four (4) carbon atoms of synthetic or natural origin;
each R₁, R₃ is hydrogen;
each R6 is hydrogen,
m is an integer lying in the range of 2 to 12.

13. A compound according to Claim 10, wherein
each R is derived from an alcohol of more than four (4) carbon atoms of synthetic or natural origin;
each R₁ is methyl and each R₃ is hydrogen
each R6 is methyl,
m is an integer of value 1,2,3,4 or 6.

14. A compound according to Claim 10, wherein
each R is derived from an alcohol of more than four (4) carbon atoms of synthetic or natural origin;
each R₁ is methyl and each R₃ is hydrogen
each R6 is hydrogen,
m is an integer lying in the range of 2 to 12.

15. A compound according to any one of Claims 10-14, wherein said compound is compound of formula (5) below:

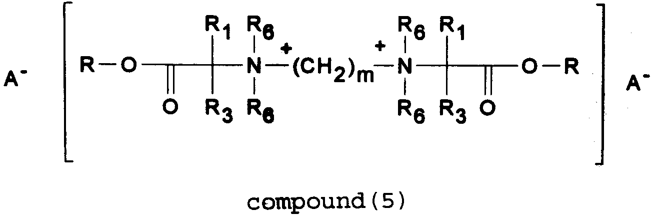

16. A compound according to either one of Claim 12 or 14, wherein
said compound is compound of formula (6) below:

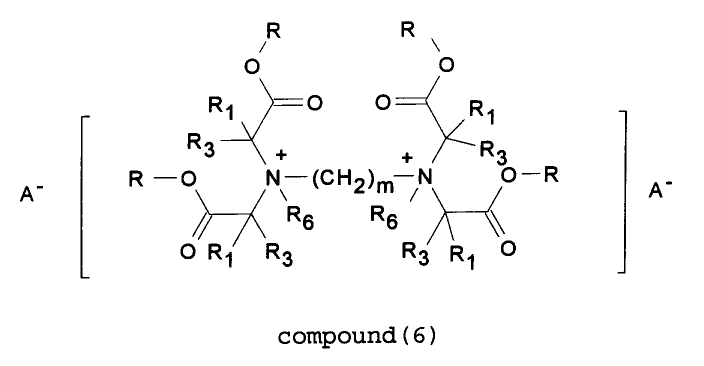

17. A compound according to Claim 10, wherein
each R is derived from an alcohol of more than four (4) carbon atoms of synthetic or natural origin;
each R₁, R₃ is hydrogen;
each R6 is methyl,
each n1 is an integer of value 2 or 3, and
n2 is an integer lying in the range of 1 to 4.

18. A compound according to Claim 10, wherein
each R is derived from an alcohol of more than four (4) carbon atoms of synthetic or natural origin;
each R₁, R₃ is hydrogen;
each R6 is hydrogen,
each n1 is an integer of value 2 or 3, and
n2 is an integer lying in the range of 1 to 4.

19. A compound according to Claim 10, wherein
each R is derived from an alcohol of more than four (4) carbon atoms of synthetic or natural origin;
each R₁ is methyl and each R₃ is hydrogen
each R6 is hydrogen,
each n1 is an integer of value 2 or 3, and
n2 is an integer lying in the range of 1 to 4.

20. A compound according to Claim 10, wherein
each R is derived from an alcohol of more than four (4) carbon atoms of synthetic or natural origin;
each R₁ is methyl and each R₃ is hydrogen
each R6 is methyl,
each n1 is an integer of value 2 or 3, and
n2 is an integer lying in the range of 1 to 4.

21. A compound according to any one of Claims 17-20, wherein said compound is compound of formula (7) below:

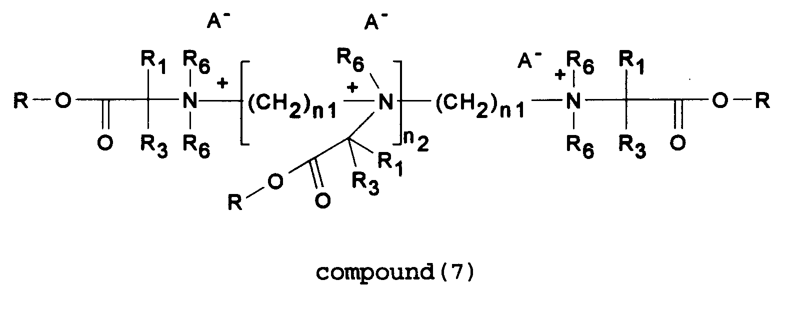

22. A compound according to any one of Claims 17-20, wherein said compound is compound of formula (8) below:

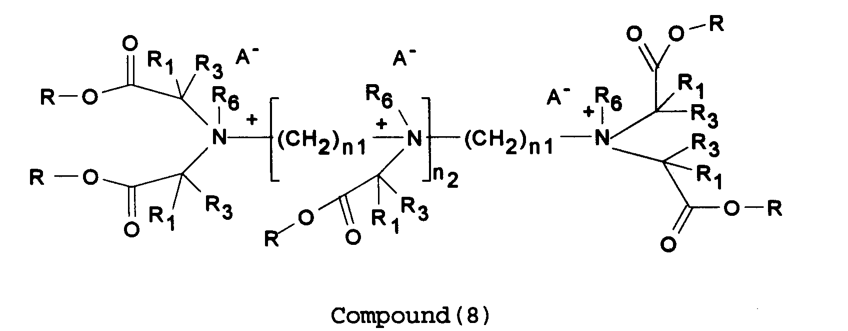

23. A compound according to any one of Claims 10-22, wherein said R group is derived from an odoriferous alcohol selected from 2-phenoxyethanol, phenylethylalcohol, geraniol, citronellol, 3-methyl-5-phenyl-1-pentanol, 2,4-dimethyl-3-cyclohexene-1-methanol, linalool, tetrahydrolinalool, 1,2-dihydromyrcenol, hydroxycitronellal, farnesol, menthol, eugenol, vanilin, cis-3-hexenol and mixtures thereof.

24. A composition according to any of the preceding claims 1-7 wherein the R group of said betaine ester compound is defined as an odoriferous alcohol selected from the group of 2-phenoxyethanol, phenylethylalcohol, geraniol, citronellol, 3-methyl-5-phenyl-1-pentanol, 2,4-dimethyl-3-cyclohexene-1-methanol, linalool, tetrahydrolinalool, 1,2-dihydromyrcenol, hydroxycitronellal, farnesol, menthol, eugenol, vanilin, cis-3-hexenol or mixtures thereof.

25. A composition according to any of the preceding claims 1-7 wherein the R-group of said betaine ester compound is defined as a biocide/bactericide alcohol such as m-chloroxylenol, 2,4-dichlorophenol, triclosan or 2,4-dichlorobenzylalcohol.

26. A composition according to claim 1 which additionally contains conventional fabric conditioner or softener matrix components and additives.

27. A composition according to claim 1 which additionally contains conventional detergent matrix components and additives.

28. A composition according to claim 1 which additionally contains conventional hard surface cleaner matrix components and additives.

29. Use of a composition as defined in claim 26 in a fabric softener matrix as a perfume-, biocide-, fungicide-, antiperspirant-, insecticide-, insect repellent-, acaricide-, or as an iron-aid-delivery system.

30. Use of a composition as defined in claim 27 or 28 in a detergent or hard surface cleaner matrix as a perfume-, biocide-, fungicide-, antiperspirant-, insecticide-, insect repellent-, acaricide-, or as an iron-aid-delivery system.

31. Process for the preparation of chloroacetate esters of alcohols by reacting chloroacetic anhydride with the alcohol, in the presence of a catalyst.

32. Process for the preparation of chloroacetate esters of sterically hindered alcohols, especially tertiary alcohols, by reacting chloroacetic anhydride with the sterically hindered alcohol, especially a tertiary alcohol, in the presence of a catalyst.

33. Process for the preparation of the chloroacetate ester according to claim 31 wherein the catalyst is pyridine (or its derivatives) or a tertiary amine and the molar ratio of chloroacetic anhydride to alcohol ranges from 0.95 to 1.5, preferably from 0.95 to 1.10, and wherein the molar ratio of catalyst to alcohol ranges from 0.95 to 1.5, preferably from 0.95 to 1.10.

34. Process for the preparation of the chloroacetate ester according to claim 32 wherein the catalyst is pyridine (or its derivatives) or a tertiary amine and the molar ratio of chloroacetic anhydride to sterically hindered alcohol ranges from 0.95 to 1.5, preferably from 0.95 to 1.10, and wherein the molar ratio of catalyst to sterically hindered alcohol ranges from 0.95 to 1.5, preferably from 0.95 to 1.10.

35. Process for the preparation of bromoacetate esters of alcohols by reacting bromoacetyl bromide with the alcohol, in the presence of a catalyst.

36. Process for the preparation of bromoacetate esters of sterically hindered alcohols, especially tertiary alcohols, by reacting bromoacetyl bromide with a sterically hindered alcohol, especially a tertiary alcohol, in the presence of a catalyst.

37. Process for the preparation of the bromoacetate ester according to claim 35 wherein the catalyst is pyridine (or its derivatives) or a tertiary amine and the molar ratio of bromoacetyl bromide to alcohol ranges from 0.95 to 1.5, preferably from 0.95 to 1.10, and wherein the molar ratio of catalyst to alcohol ranges from 0.95 to 1.5, preferably from 0.95 to 1.10.

38. Process for the preparation of the bromoacetate ester according to claim 36 wherein the catalyst is pyridine (or its derivatives) or a tertiary amine and the molar ratio of bromoacetyl bromide to tertiary alcohol ranges from 0.95 to 1.5, preferably from 0.95 to 1.10, and wherein the molar ratio of catalyst to tertiary alcohol ranges from 0.95 to 1.5, preferably from 0.95 to 1.10.

39. Process for the preparation of betaine esters as defined in claim 1 by reacting halogenoacetate ester with a tertiary amine at room temperature in a polar solvent wherein the molar ratio of halogeno acetate ester to the tertiary amine ranges from 1:1 to 10:1, preferably from 1:1 to 2:1.

40. Process for preparing an alkaline stable composition comprising betaine esters by addition of anionic surfactant to a composition as defined in claim 1.

41. Process for preparing an alkaline stable composition comprising betaine esters according to claim 36 by further addition of a hydrophobic counter-ion, preferably an anionic surfactant, to the composition.
